# EUROPEAN PATENT APPLICATION

(11) **EP 4 527 383 A1**
(43) Date of publication of application: **26.03.2025**
(21) Application number: 23806963.7
(22) Date of filing: 17.05.2023
(51) Int. Cl.: A61K 31/395, A61P 35/00

(54) **DRUG FOR TREATING TRIPLE NEGATIVE BREAST CANCER**

(30) Priority: 17.05.2022 CN 202210536761
(71) Applicant: Hinova Pharmaceuticals Inc., Chengdu, Sichuan 610041 (CN)
(72) Inventor: LI, Jing, Chengdu, Sichuan 610041 (CN); DU, Wu, Chengdu, Sichuan 610041 (CN); LIU, Guoqing, Chengdu, Sichuan 610041 (CN); HUO, Yongxu, Chengdu, Sichuan 610041 (CN); CHEN, Kefan, Chengdu, Sichuan 610041 (CN); LI, Xinghai, Chengdu, Sichuan 610041 (CN)
(74) Representative: Altmann Stößel Dick Patentanwälte PartG mbB
(86) International application number: PCT/CN2023/094688
(87) International publication number: WO 2023/222012

(57) **Abstract**

Provided is a drug for treating triple negative breast cancer. Provided is the use of a CRBN E3 ligase ligand-based protein degradation agent targeting androgen receptor in the preparation of a drug for preventing and/or treating triple negative breast cancer (including androgen receptor positive triple negative breast cancer). Provided is use of a CRBN E3 ligase ligand-based protein degradation agent of an androgen receptor in combination with a targeting drug (comprising a PI3Kα inhibitor and a PARP inhibitor), an immunotherapy drug or a chemotherapeutic drug in the preparation of a drug for preventing and/or treating triple negative breast cancer.

## Description

### Field of the invention

The present invention belongs to the medical field, and in particular, the present invention relates to medicaments for the treatment of triple negative breast cancer (TNBC).

### Background of the invention

Breast cancer has become the most common female malignant tumor in the world, its incidence rate and mortality rate are the first among female tumors, and thus it seriously threatens and endangers women's physical and mental health. Different types of breast cancer have significantly different biological characteristics and clinical manifestations. At present, immunohistochemical methods are mainly used in clinical practice. According to the detection results of estrogen receptor (ER), progesterone receptor (PR) and human epidermal growth factor receptor 2 (HER-2), breast cancer is divided into four molecular subtypes: luminal A (ER-positive or PR-positive, HER-2-negative, low Ki67 expression), luminal B (ER-positive or PR-positive, HER-2-positive), HER-2 overexpression (ER- and PR-negative, HER-2 positive, high expression of most Ki67) and basal-like (ER-, PR-, and HER-2 negative). Triple negative breast cancer (TNBC), as a subtype of breast cancer with the highest degree of malignancy, is characterized by negative ER, PR and HER-2, together with high recurrence rate, high early metastasis rate, strong invasion and poor prognosis.

At present, chemotherapy is one of the main ways to treat breast cancer. Cyclophosphamide, doxorubicin and 5-fluorouracil (5-FU) have been recognized as first-line chemotherapy drugs for the treatment of advanced breast cancer. With the emergence of paclitaxel and other medicaments, more and more clinicians believe that paclitaxel and cisplatin can also be used as first-line chemotherapy options. However, the therapeutic efficacy of these chemotherapy drugs still needs to be further improved.

Androgen receptor (AR) belongs to the nuclear receptor family and is a type of ligand-dependent transcription factor. The abnormal regulation of AR signaling pathway plays an important role in the occurrence and development of prostate cancer. It has been shown that castration-resistant prostate cancer (CRPC) still depends on the action of AR. Proteolytic targeting chimeras (PROTACs) have attracted widespread attention as small molecules that can induce degradation of target proteins. PROTACs, as bifunctional molecules, include a small molecule compound that can bind to the protein of interest (POI), a linker introduced at its suitable position, and a small molecule compound which can bind to E3 ubiquitin ligases. The obtained small molecule probe can simultaneously bind to the target protein and E3 ubiquitin ligases, thereby promoting the ubiquitination of the target protein, by which the protein can be recognized and degraded by the proteasome. Using the PROTACs strategy, proteolysis-targeting chimeras capable of recognizing/binding androgen receptors (AR PROTACs) were prepared, which can regulate the levels of androgen receptors by the intracellular ubiquitin-proteasome system (UPS), and induce the degradation of androgen receptors, and thus achieve the effect of treating related diseases regulated by androgen receptors such as prostate cancer.

However, it has not been reported that AR PROTACs is used to treat TNBC.

### Content of the invention

An object of the present invention is to provide the use of a CRBN E3 ligase ligand-based protein degradation agent targeting androgen receptor in the manufacture of medicaments for the prevention and/or treatment of TNBC.

Another object of the present invention is to provide the use of a CRBN E3 ligase ligand-based protein degradation agent targeting androgen receptor, in combination with targeted drugs (including PI3Kα inhibitors and PARP inhibitors), immunotherapeutic drugs or chemotherapeutic drugs, in the manufacture of medicaments for the prevention and/or treatment of TNBC.

The present invention specifically provides the use of a CRBN E3 ligase ligand-based protein degradation agent targeting androgen receptor in the manufacture of medicaments for the prevention and/or treatment of TNBC.

Further, the protein degradation agent targeting androgen receptor is a PROTAC bifunctional chimeric molecule represented by formula (I), or an optical isomer thereof, a tautomer thereof, or a pharmaceutically acceptable salt thereof: 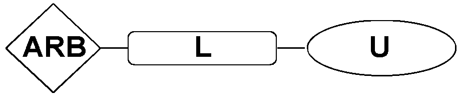 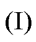 wherein ARB is a ligand for androgen receptor, L is a linker, and U is a ligand for CRBN E3 ligase.

Further, said U is a CRBN E3 ligase ligand with the following structure: 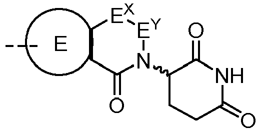
wherein E is selected from the group consisting of unsubstituted and substituted benzene ring, 5-membered heteroaromatic ring, 6-membered heteroaromatic ring, benzoheteroaromatic ring, and benzene-fused unsaturated heterocycle;
E^{X} is selected from the group consisting of absence, halogen, O, S, N, carbonyl, and -(CH₂)-;
E^{Y} is selected from the group consisting of absence, H, and carbonyl.

Further, the structure of said U is selected from the group consisting of:

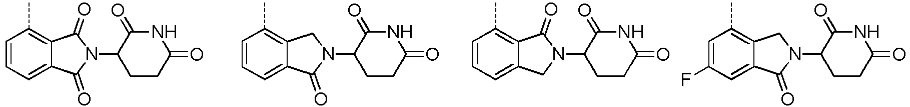

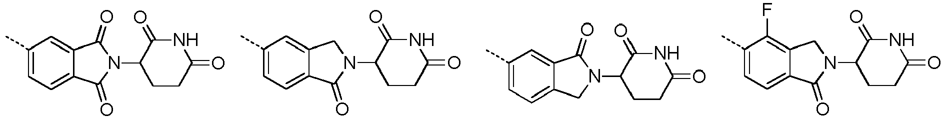

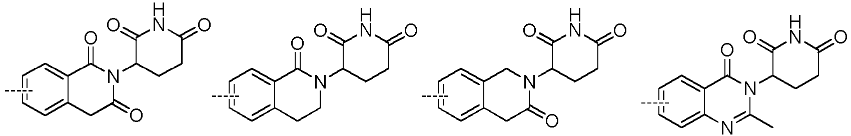

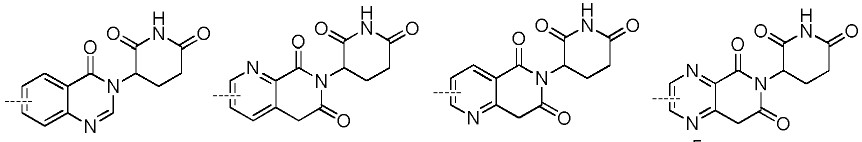

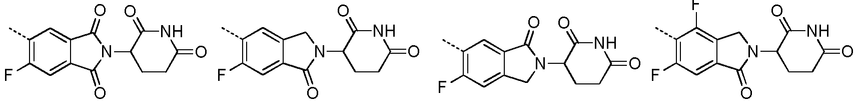

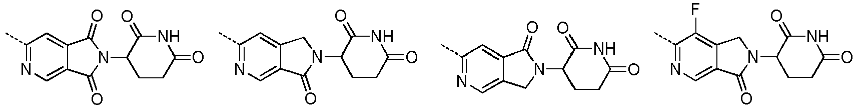

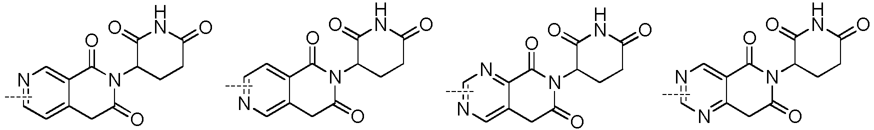

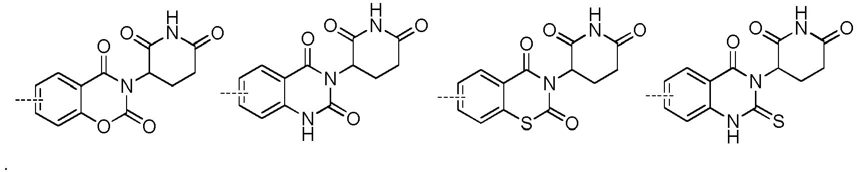

Further, the PROTAC bifunctional chimeric molecule is selected from the group consisting of ARV-110, ARV-766, HP518, AC0176, GT20029, ASN-1780, ARD-2128, and ARD-2585.

Further, the structure of said PROTAC bifunctional chimeric molecule is as represented by formula (II): 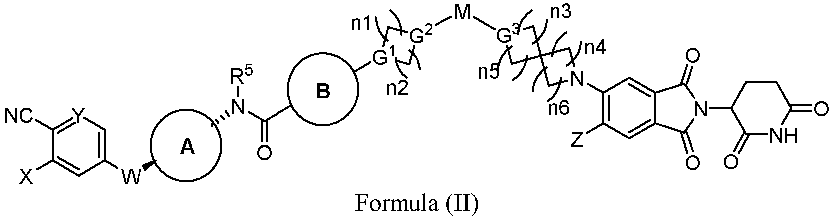
wherein X is selected from the group consisting of F, Cl, Br, Me, and CF₃;
Y is selected from CH or N;
W is selected from the group consisting of O, S, and NMe;
ring A is selected from the group consisting of the following groups substituted with R¹, R², R³ and/or R⁴: cyclopropane, cyclobutane, cyclopentane, cyclohexane, and cycloheptane;
R¹, R², R³, R⁴, and R⁵ are each independently selected from H and Me;
ring B is selected from the group consisting of 5-membered heteroaromatic ring, 6-membered aromatic ring, and 6-membered heteroaromatic ring;
G¹, G², and G³ are each independently selected from the group consisting of CR⁶ and N; wherein R⁶ is selected from the group consisting of H, halogen, and OH;
n1, n2, n3, n4, n5, and n6 are each independently selected from 1 or 2;
M is selected from the group consisting of CR⁷R⁸, wherein R⁷ and R⁸ are each independently selected from H and Me;
Z is selected from the group consisting of H, F, Cl, and Me.

Further, the structure of said PROTAC bifunctional chimeric molecule is selected from the group consisting of:

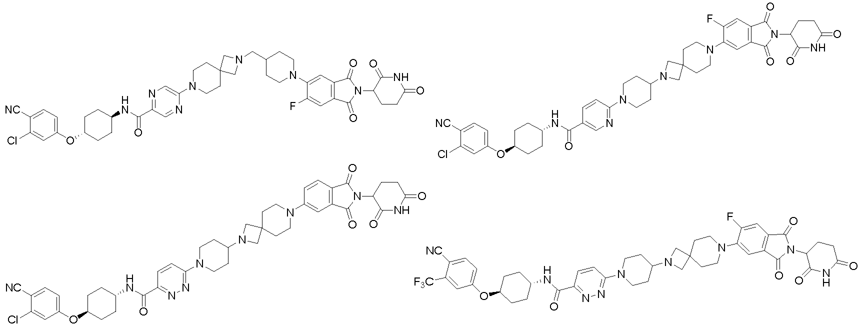

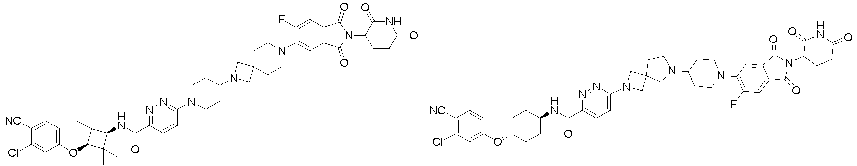

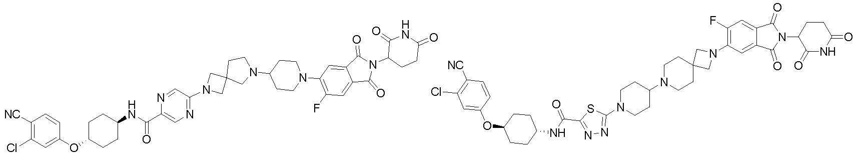

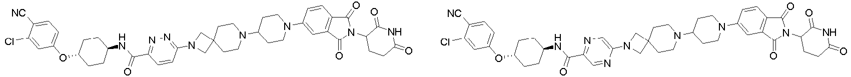

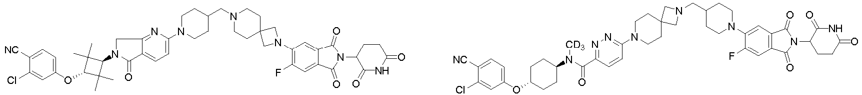

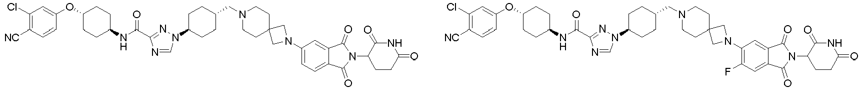

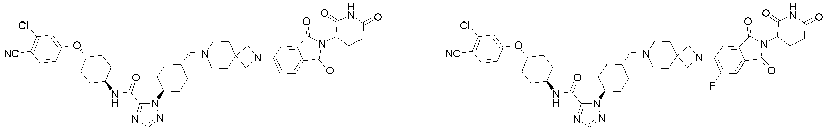

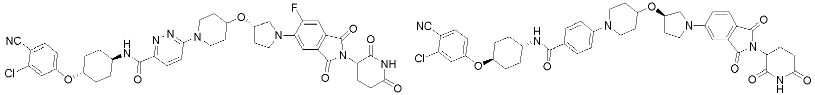

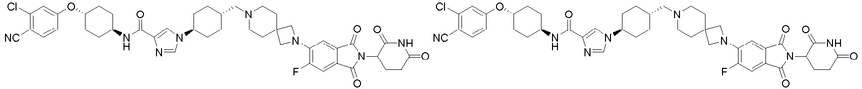

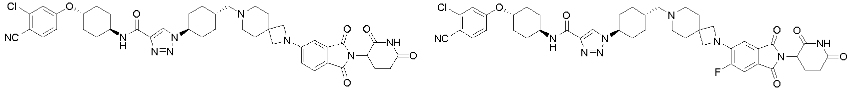

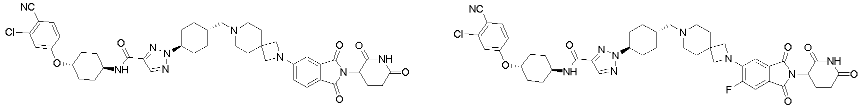

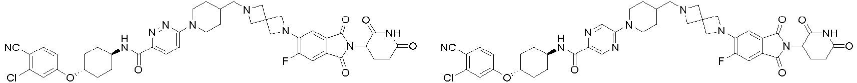

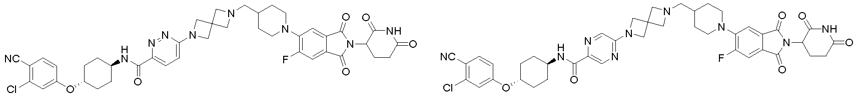

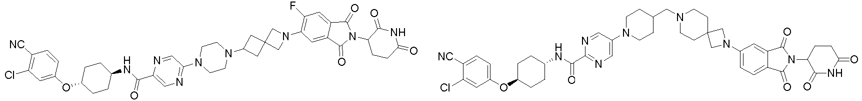

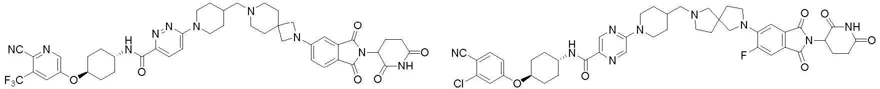

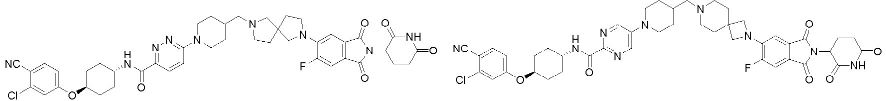

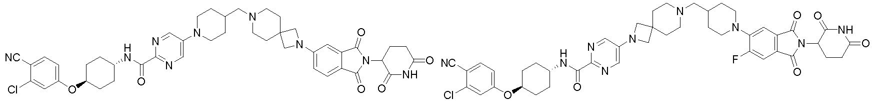

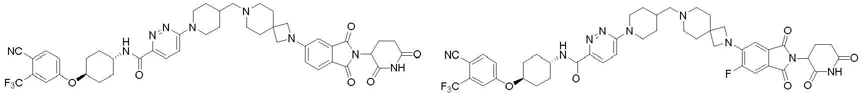

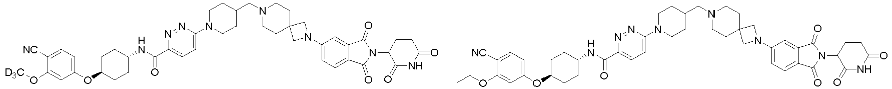

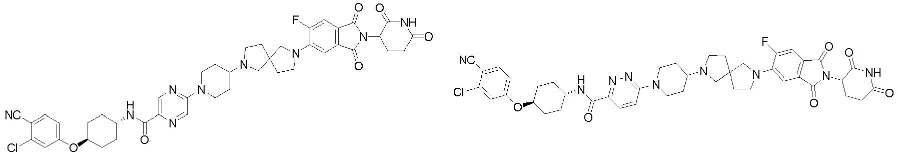

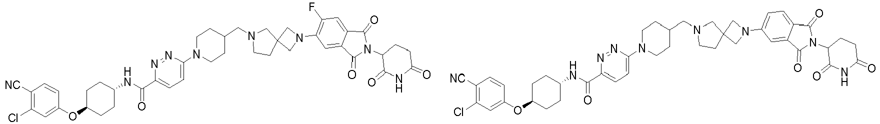

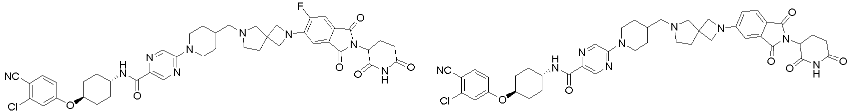

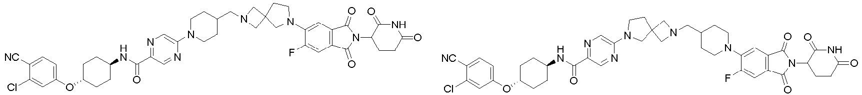

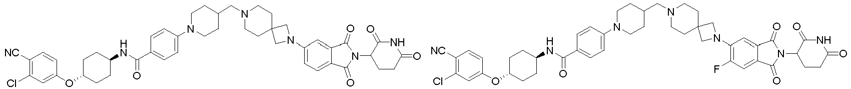

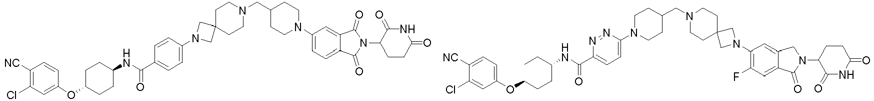

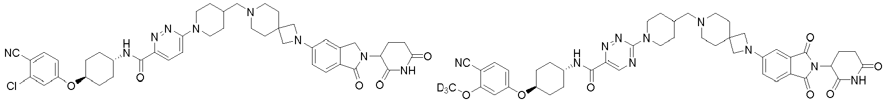

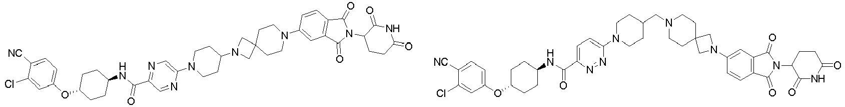

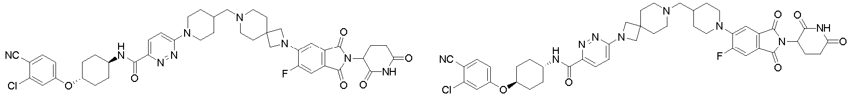

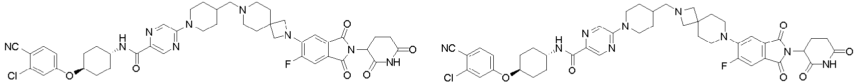

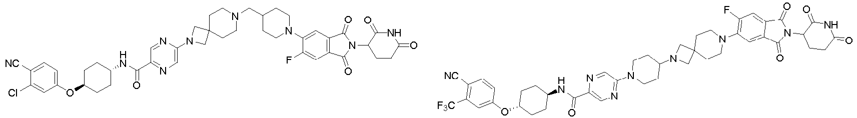

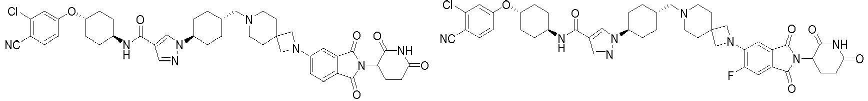

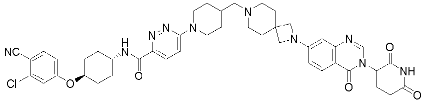

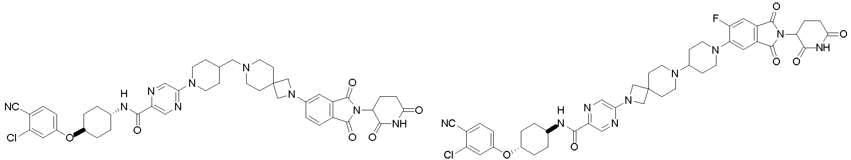

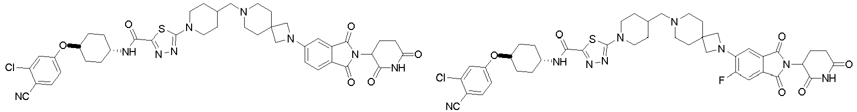

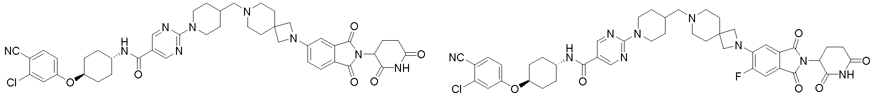

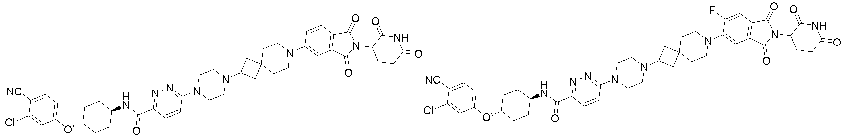

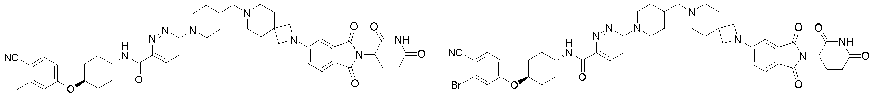

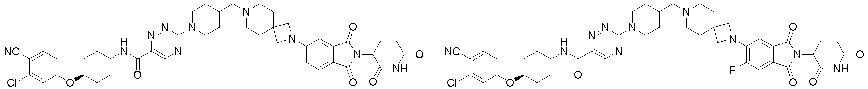

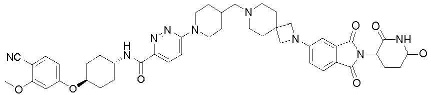

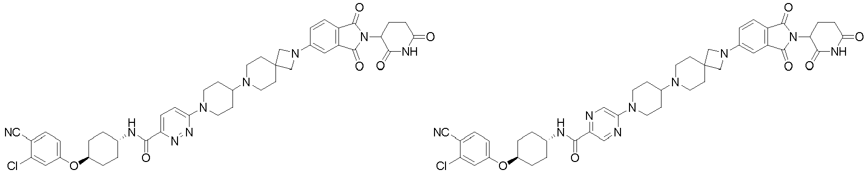

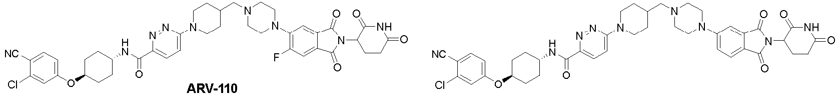

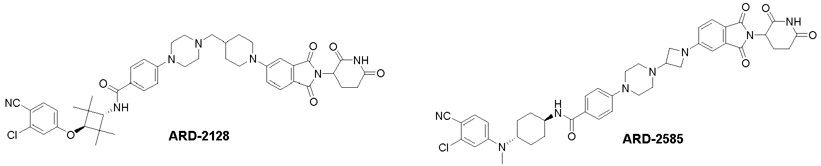

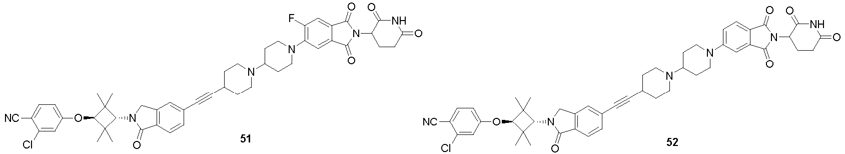

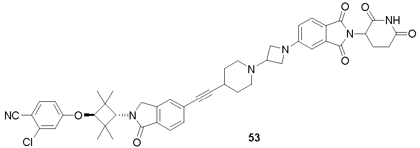

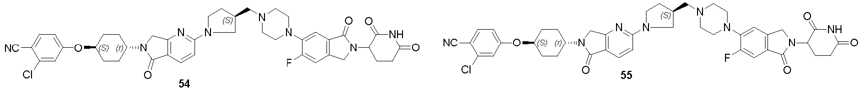

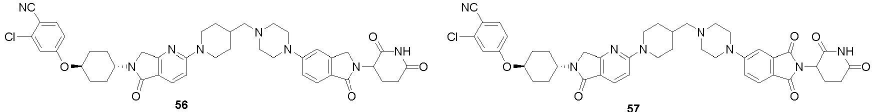

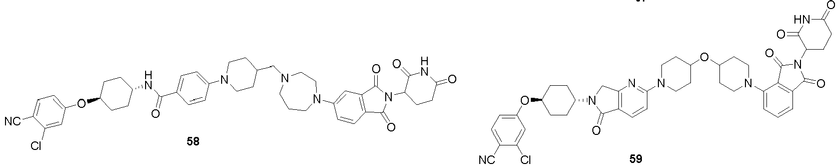

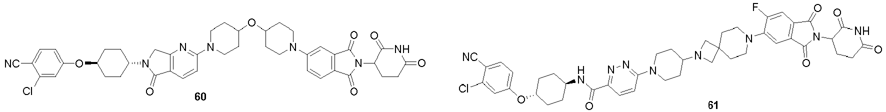

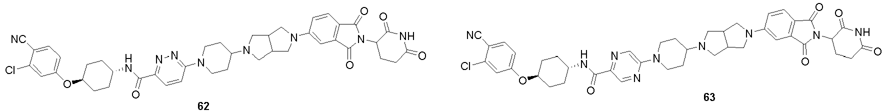

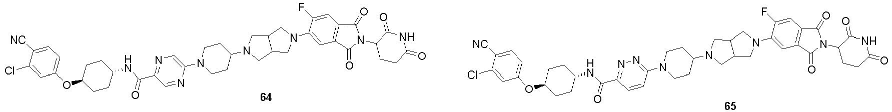

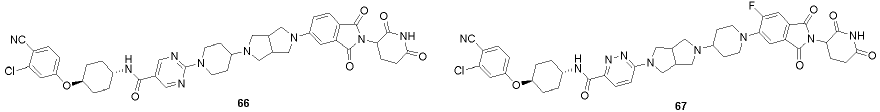

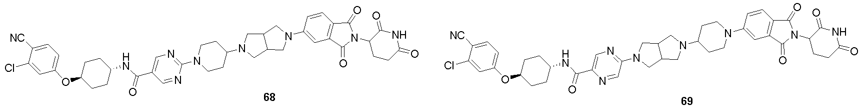

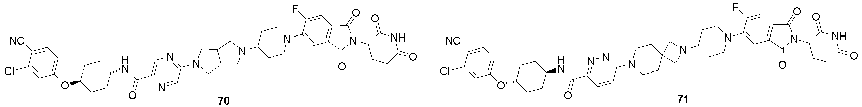

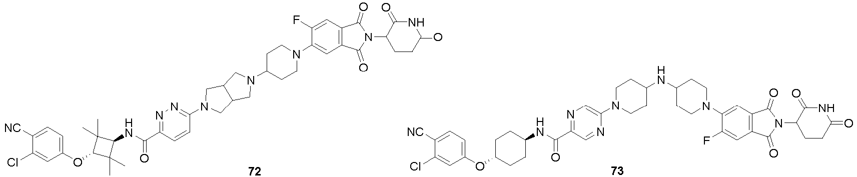

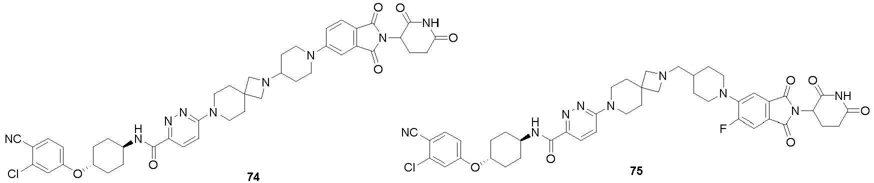

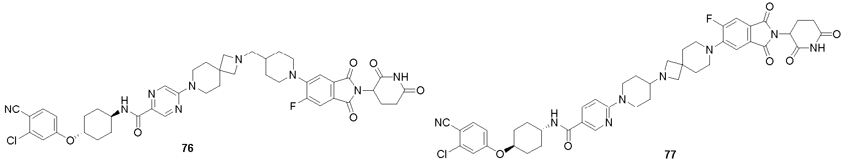

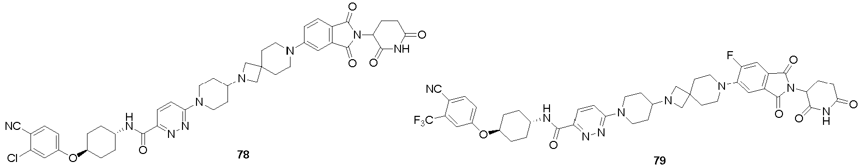

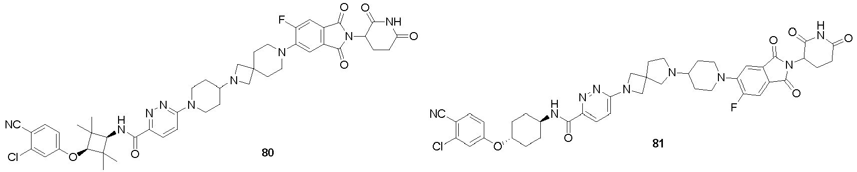

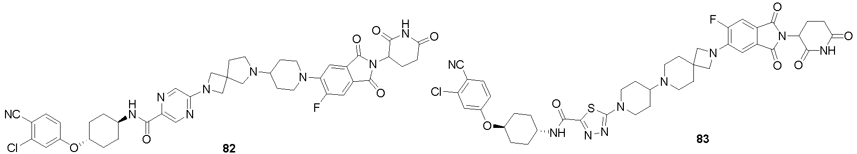

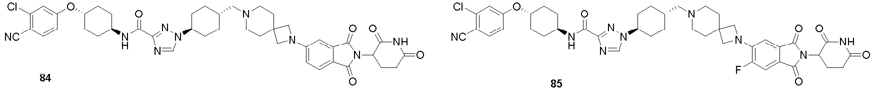

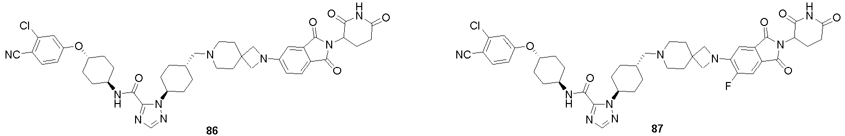

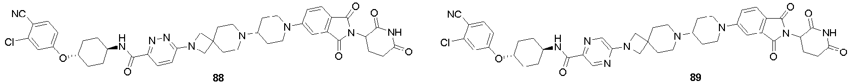

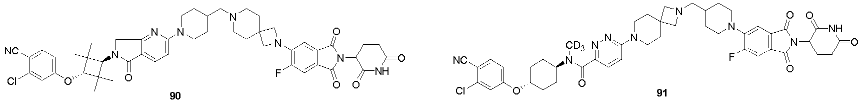

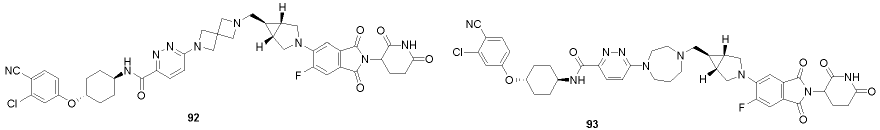

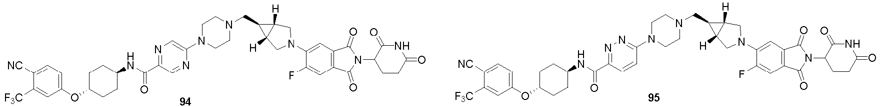

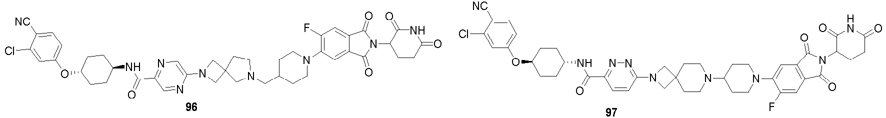

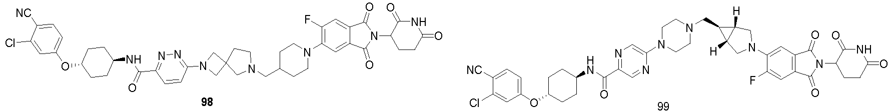

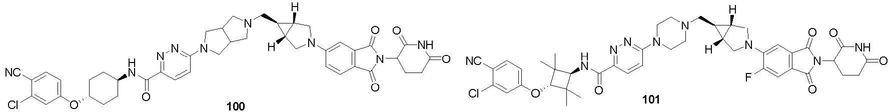

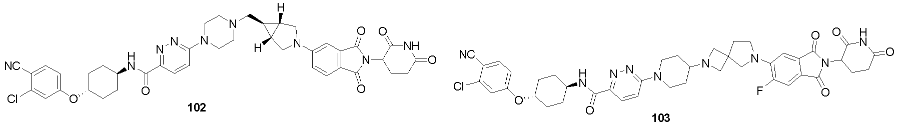

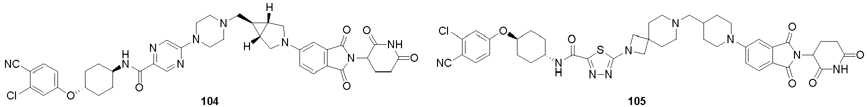

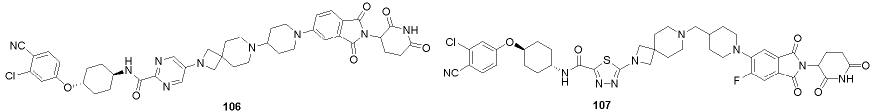

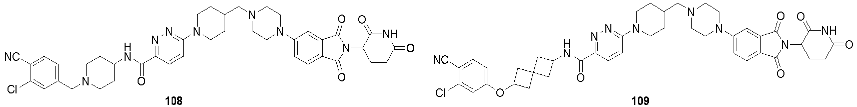

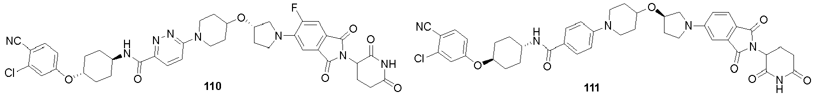

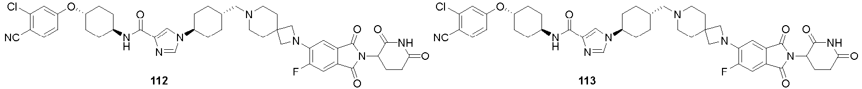

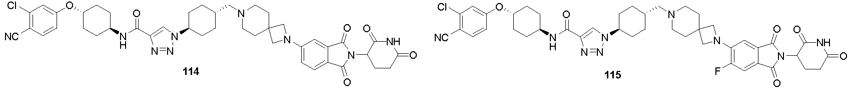

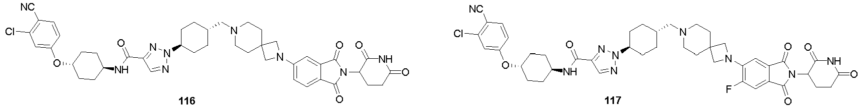

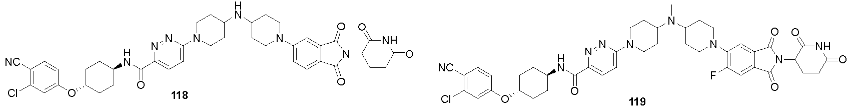

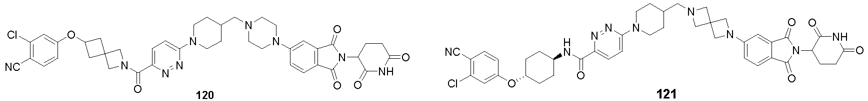

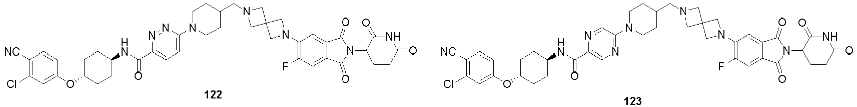

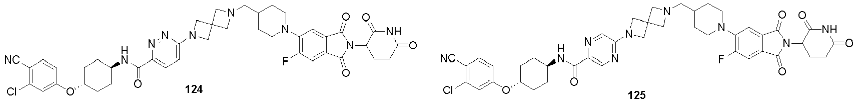

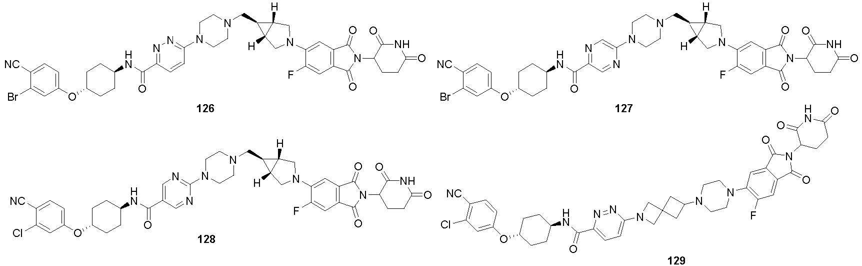

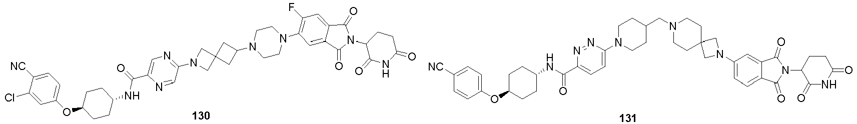

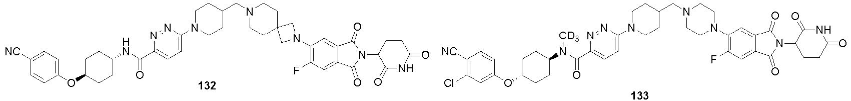

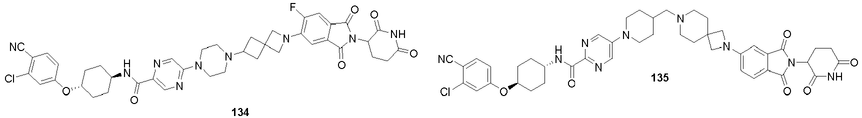

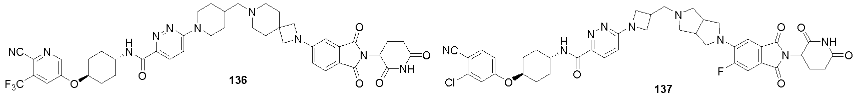

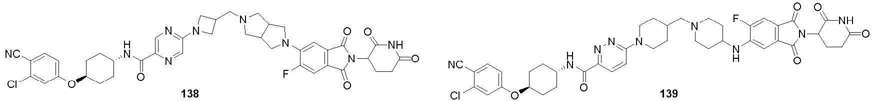

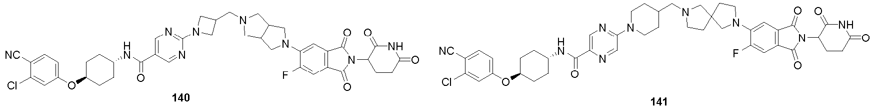

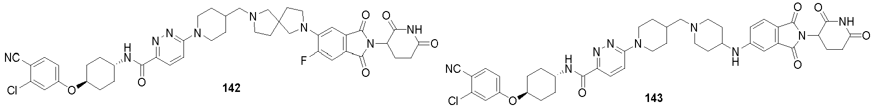

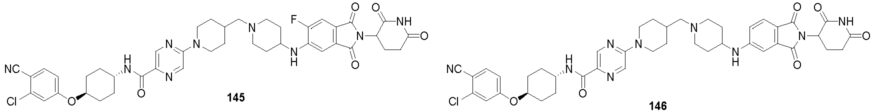

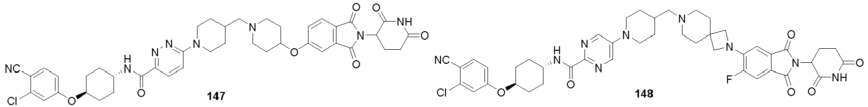

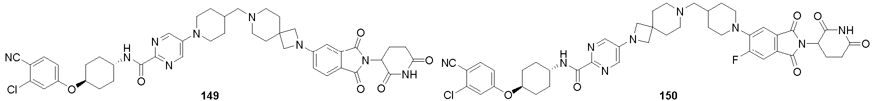

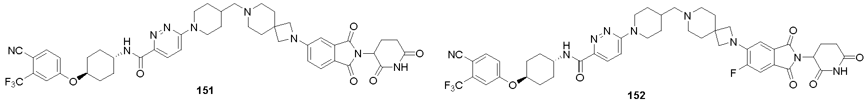

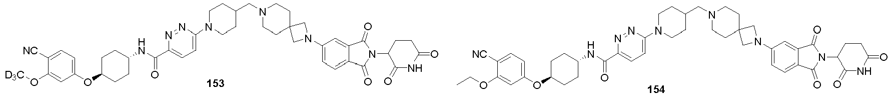

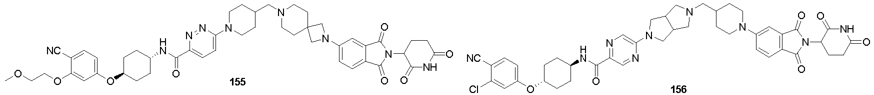

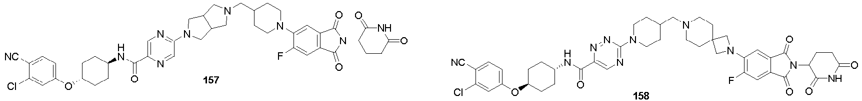

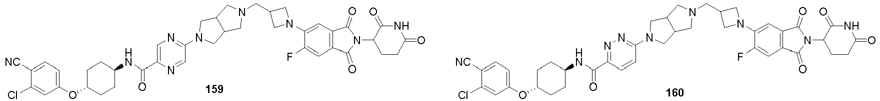

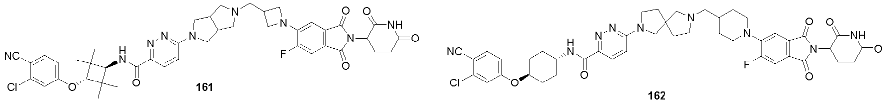

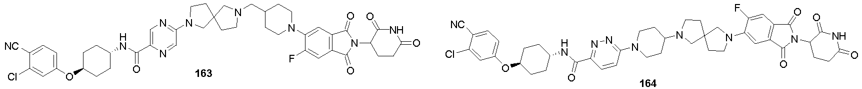

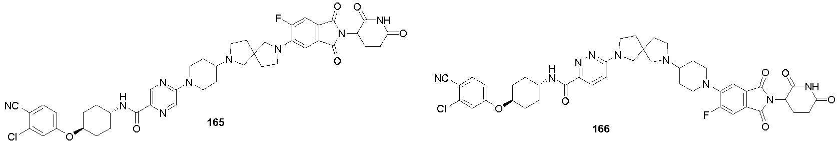

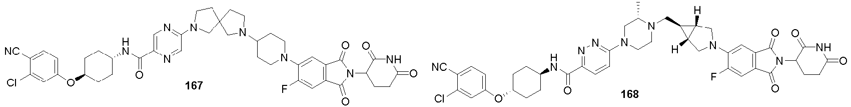

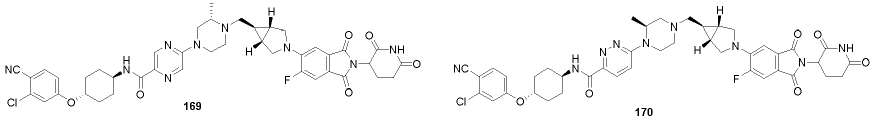

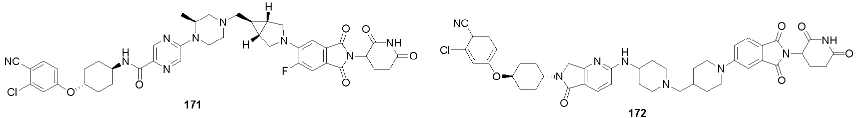

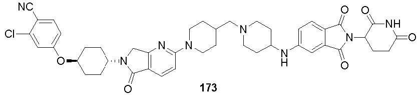

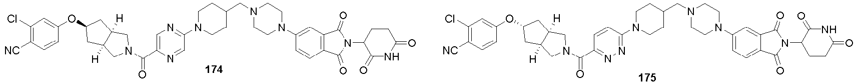

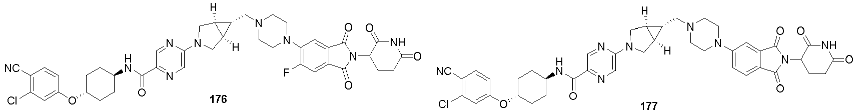

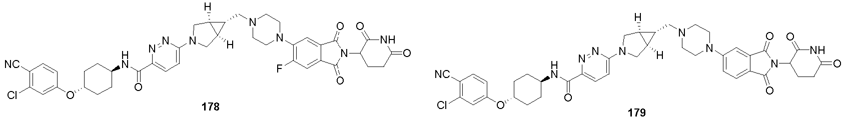

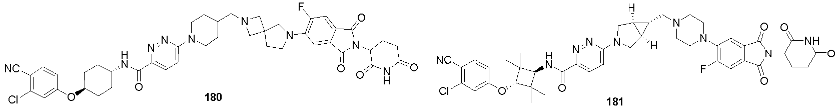

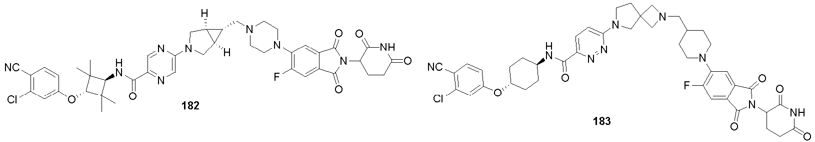

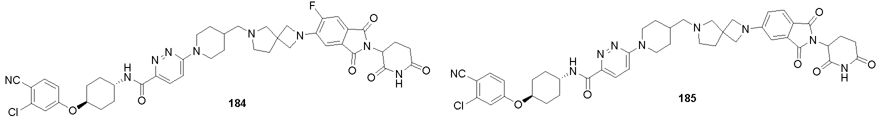

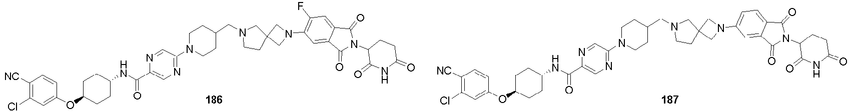

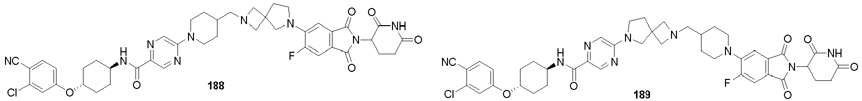

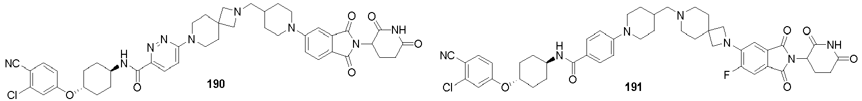

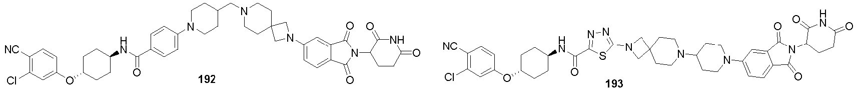

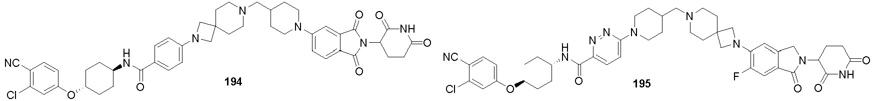

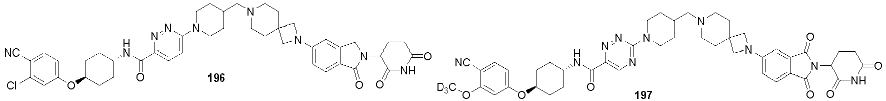

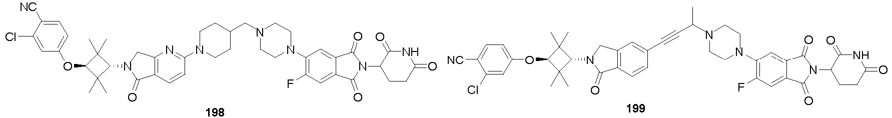

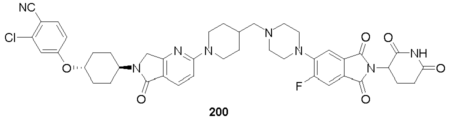

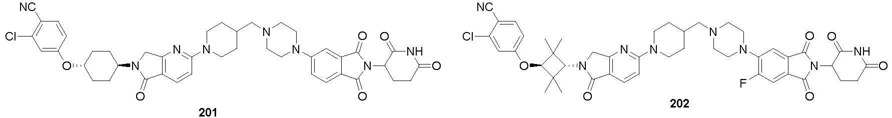

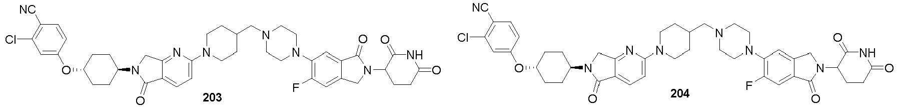

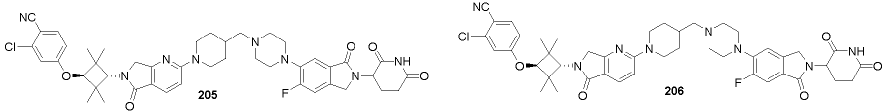

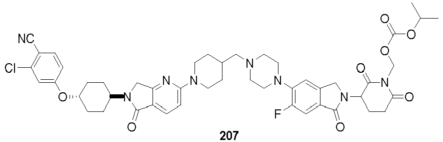

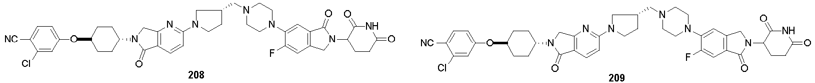

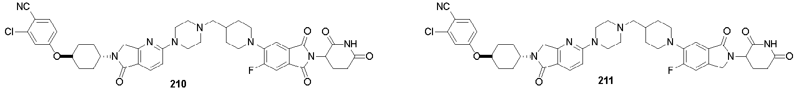

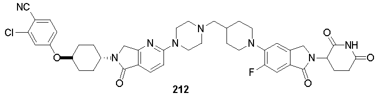

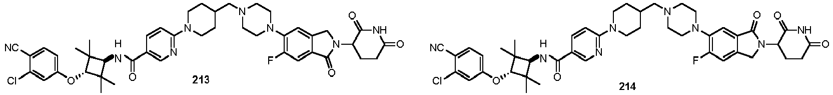

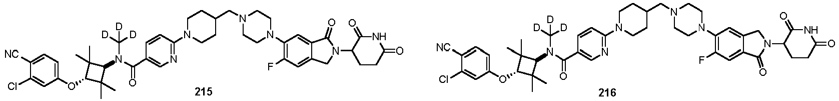

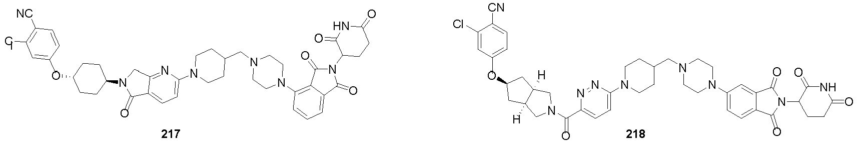

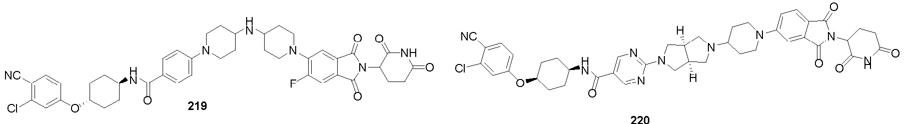

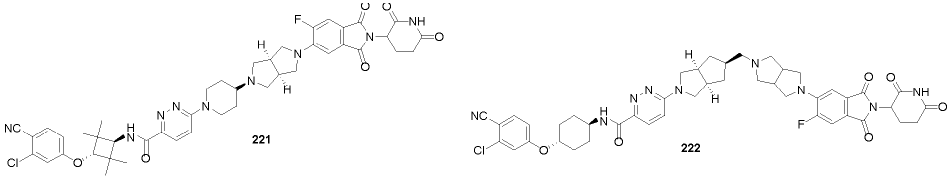

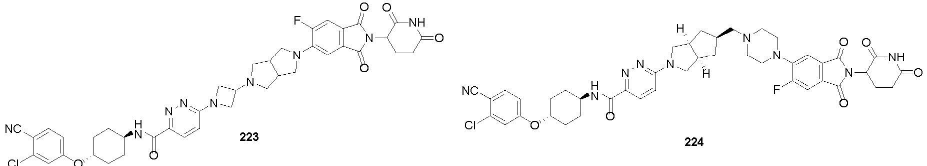

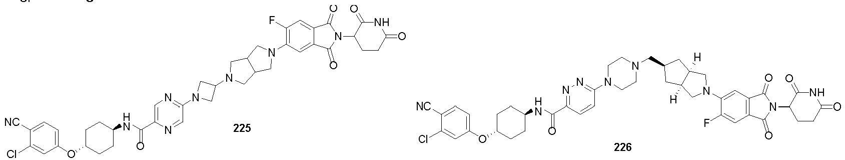

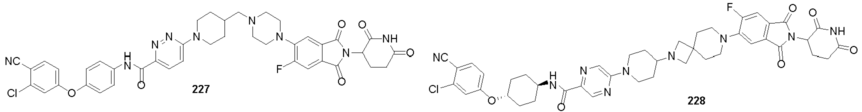

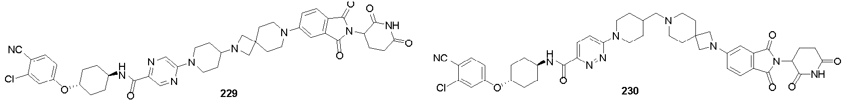

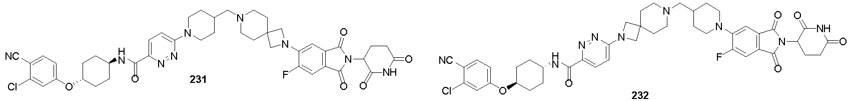

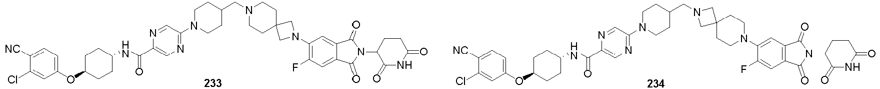

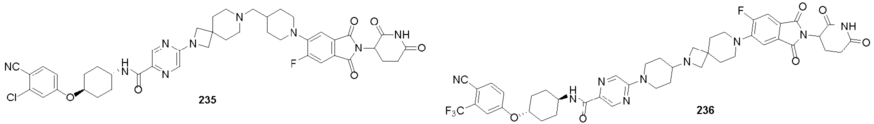

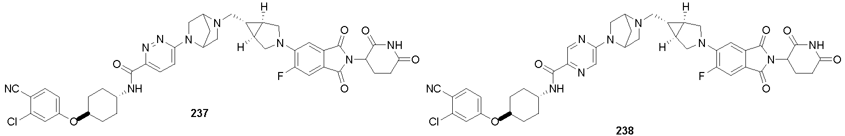

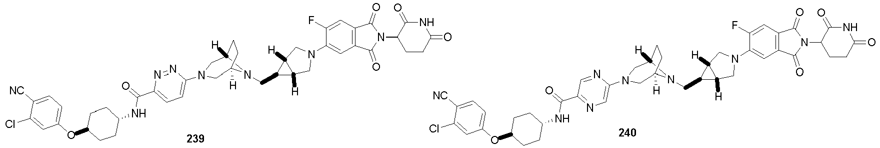

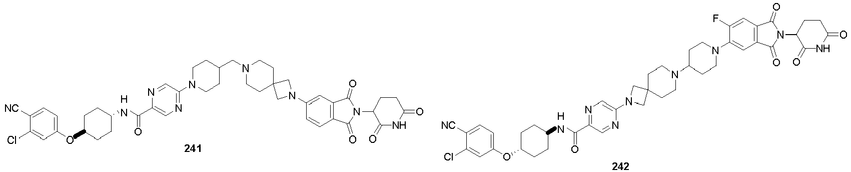

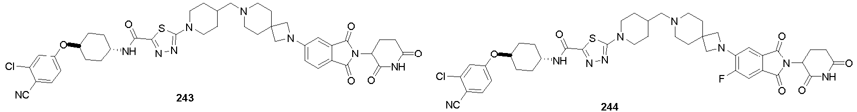

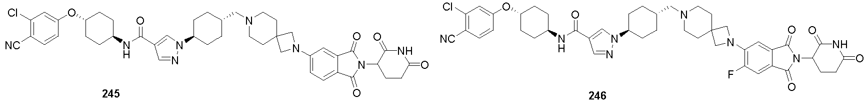

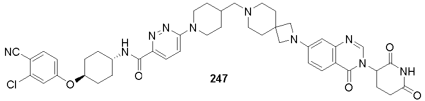

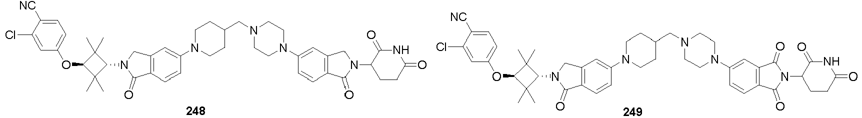

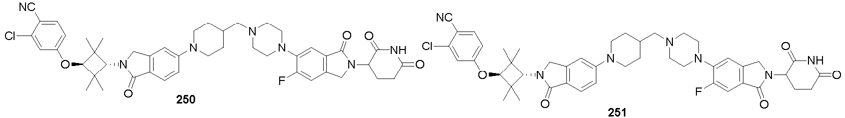

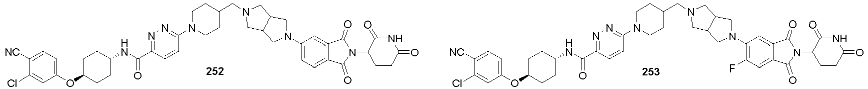

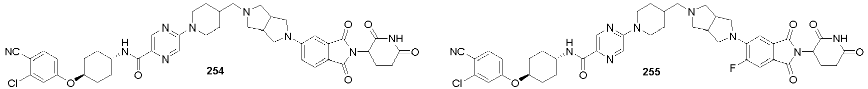

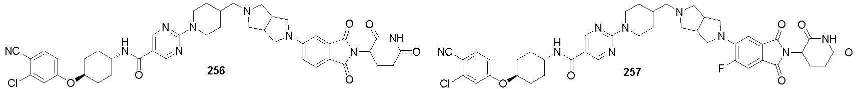

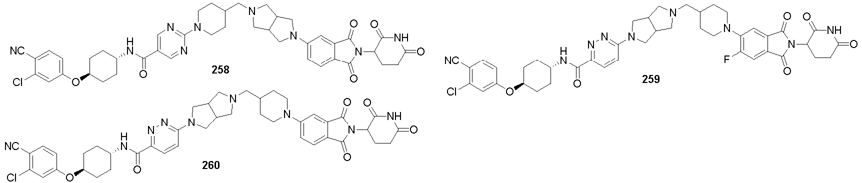

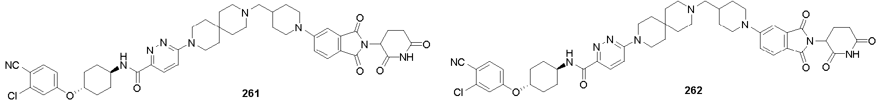

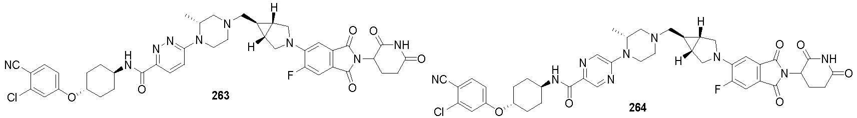

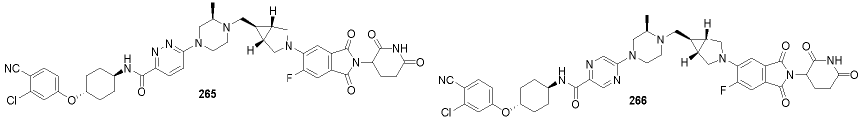

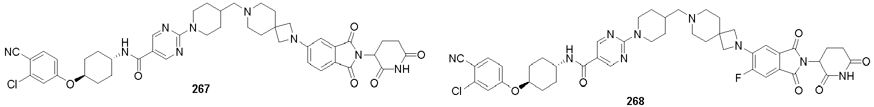

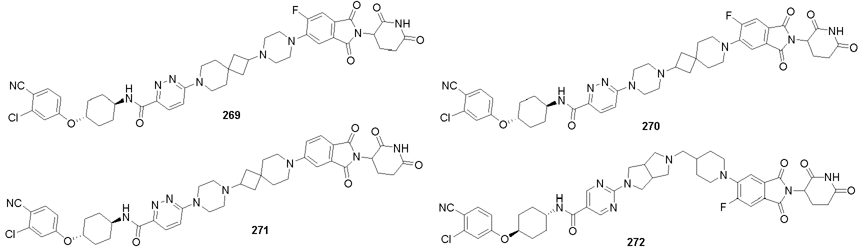

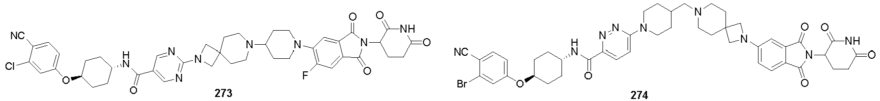

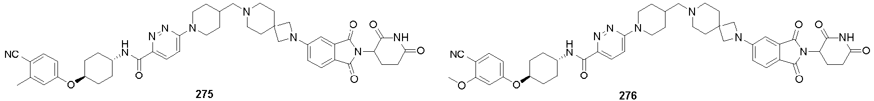

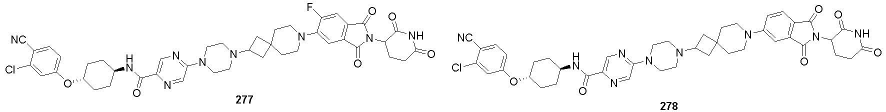

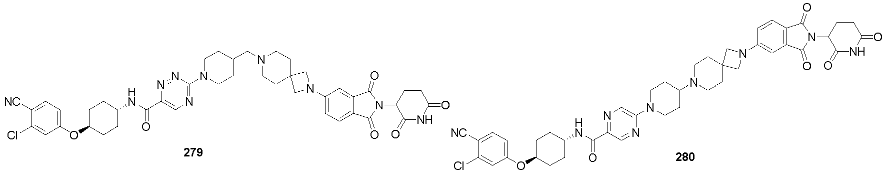

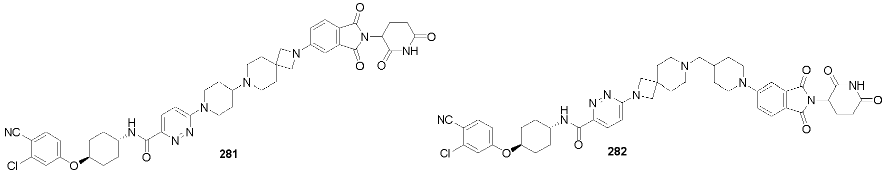

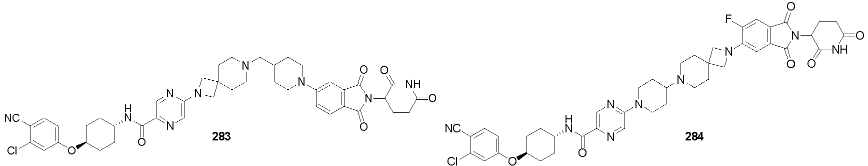

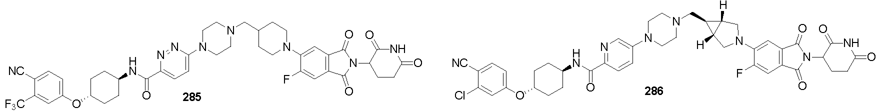

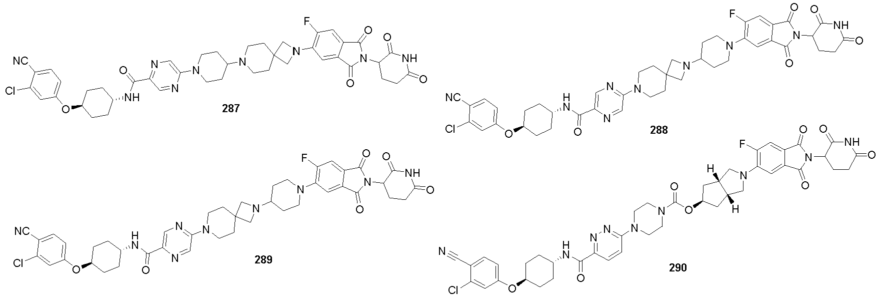

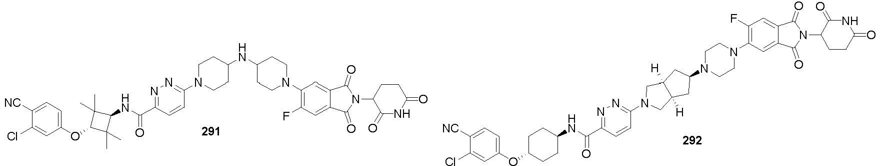

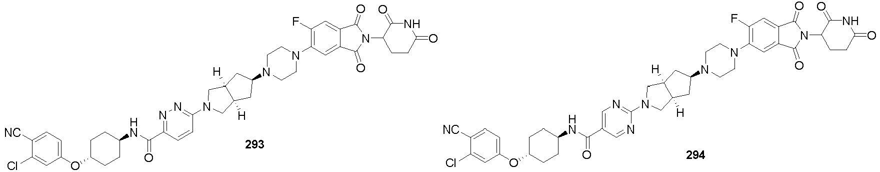

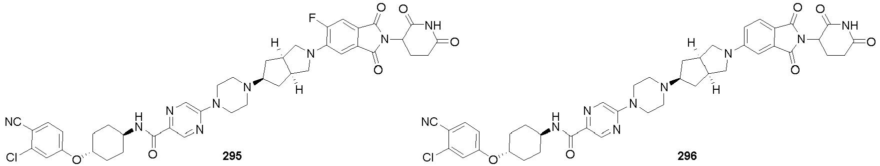

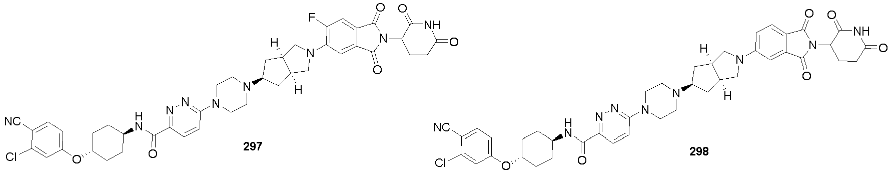

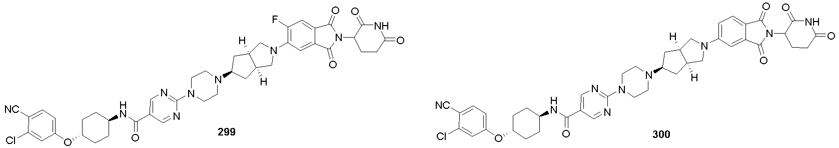

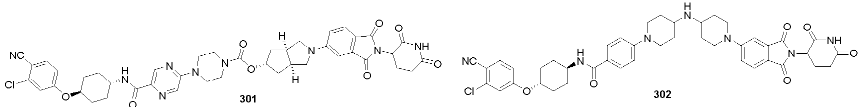

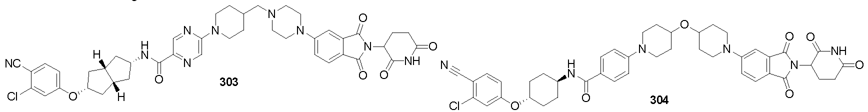

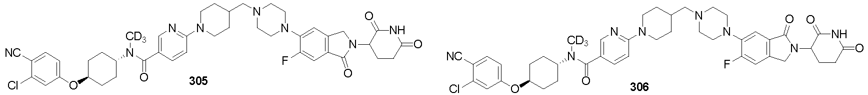

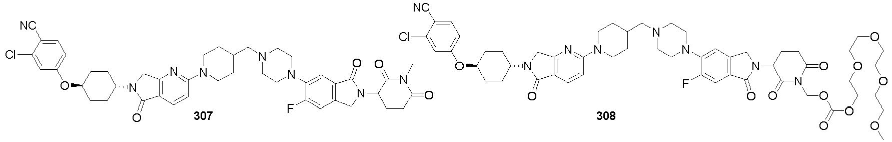

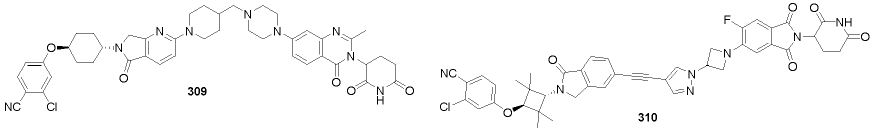

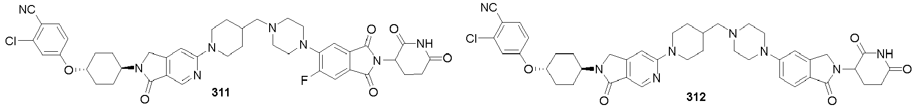

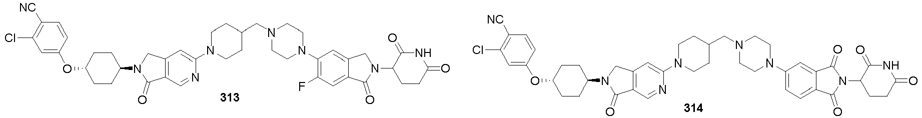

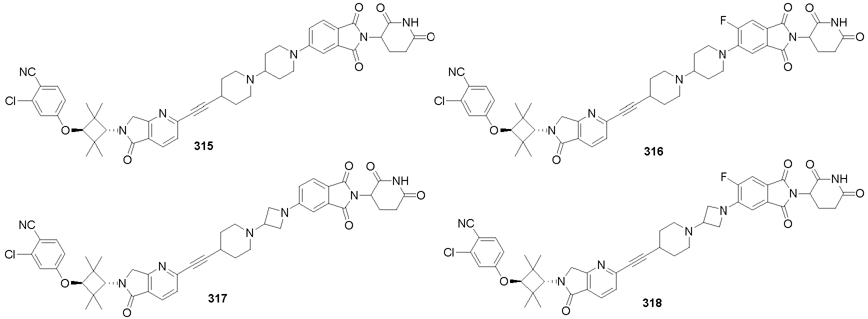

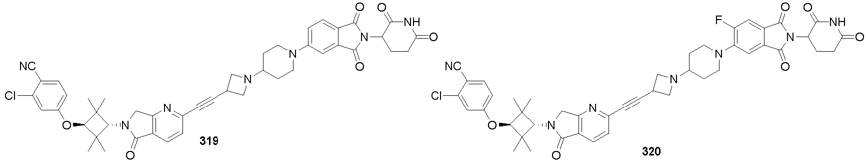

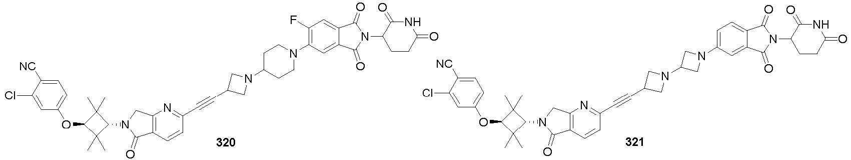

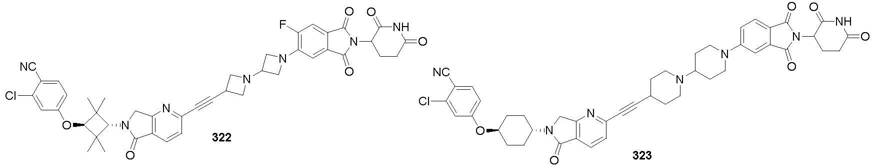

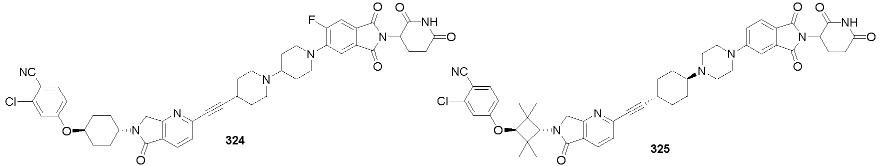

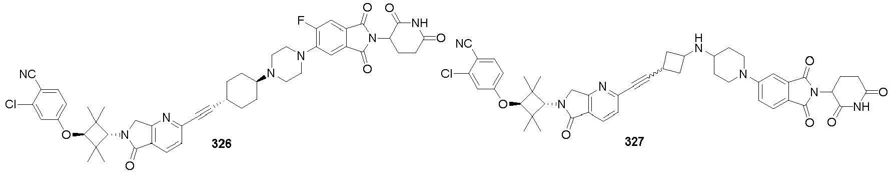

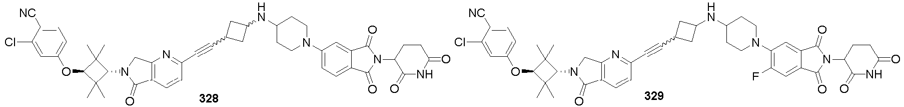

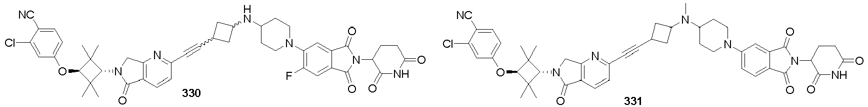

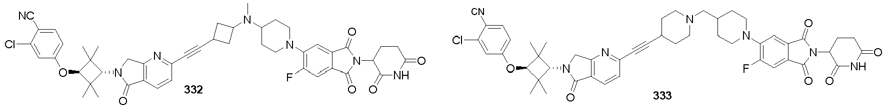

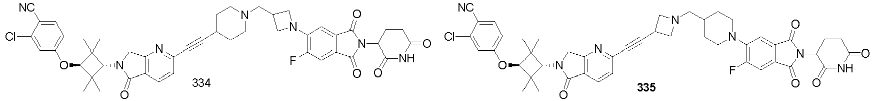

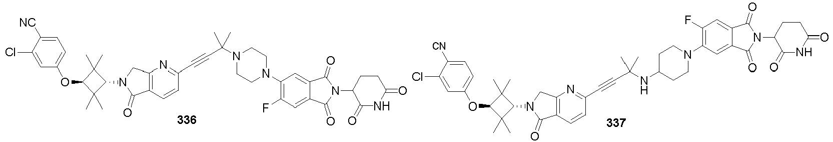

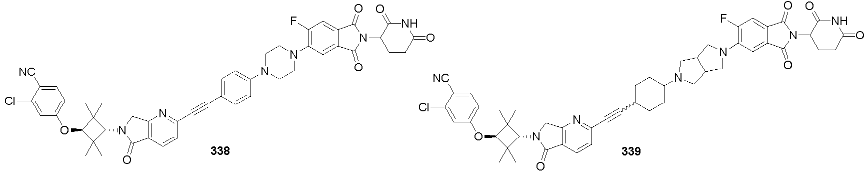

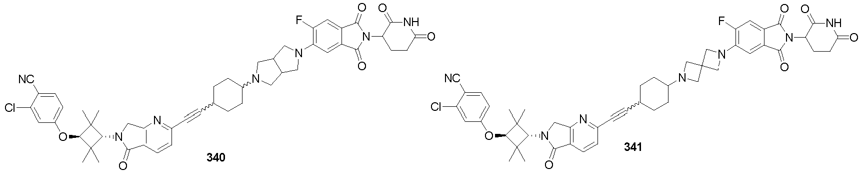

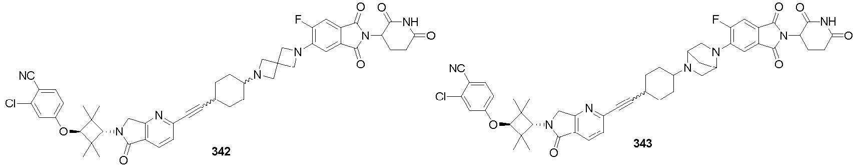

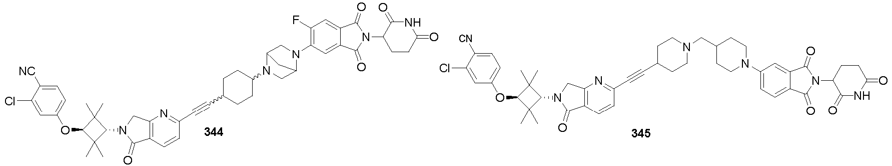

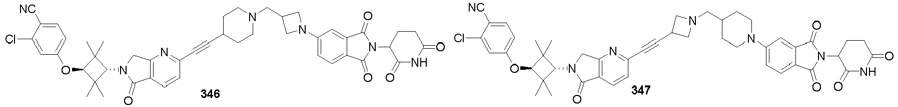

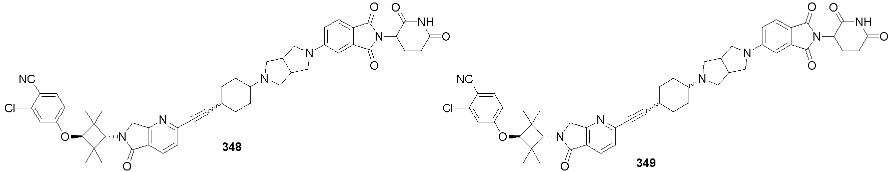

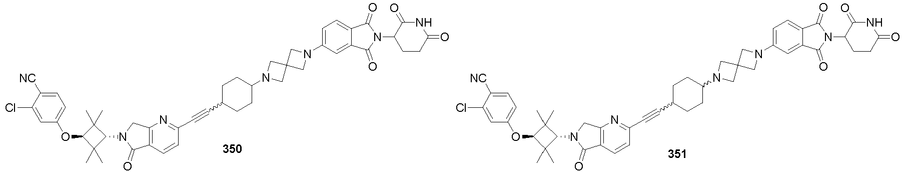

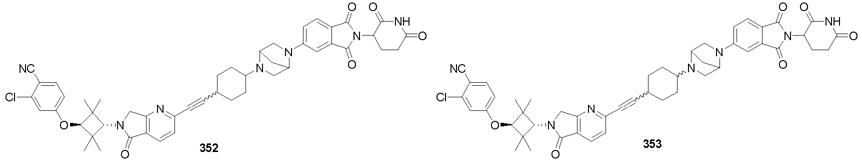

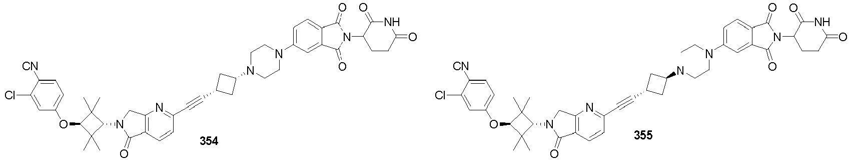

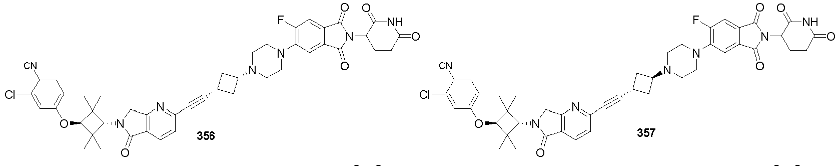

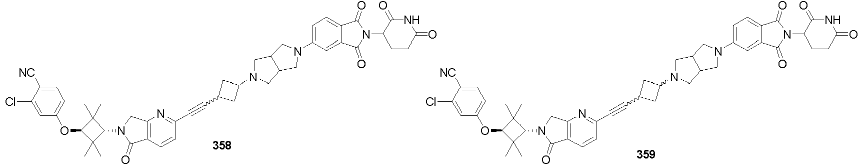

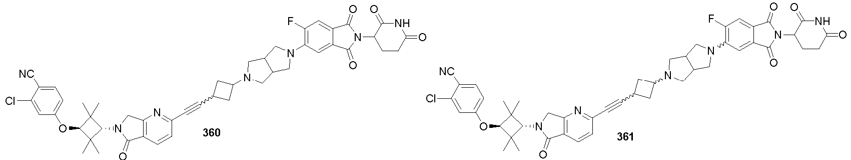

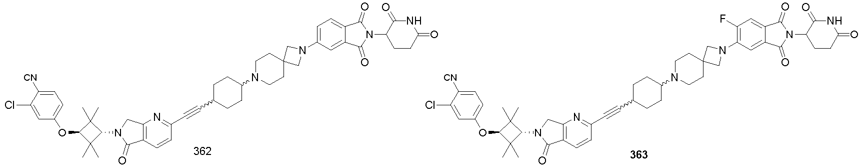

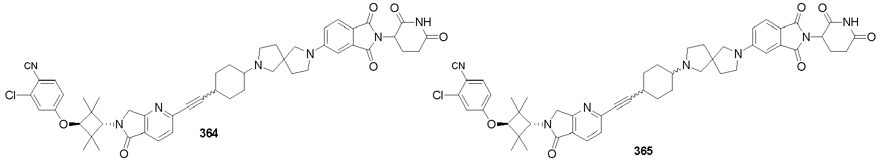

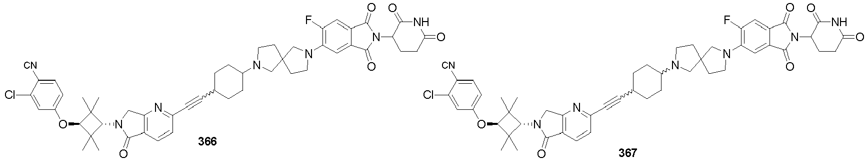

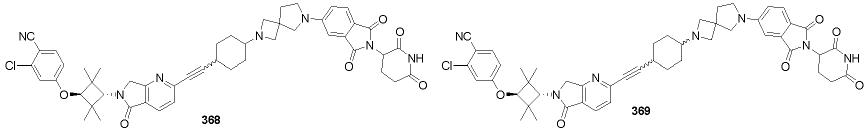

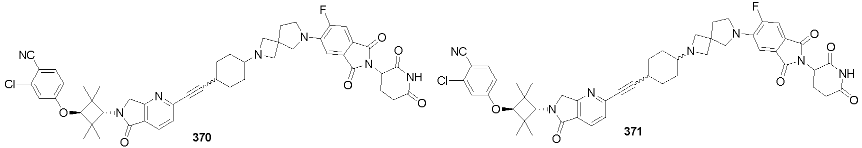

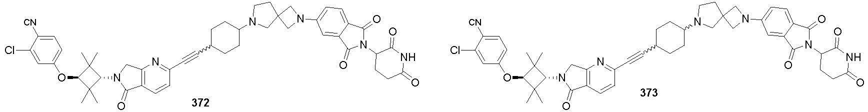

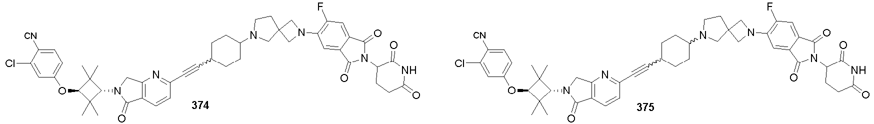

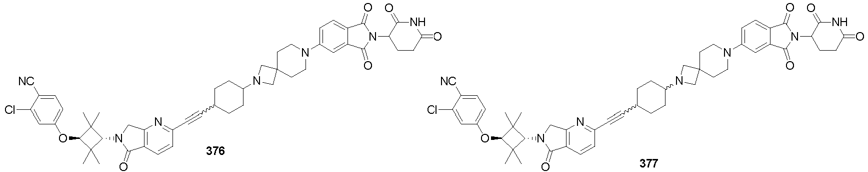

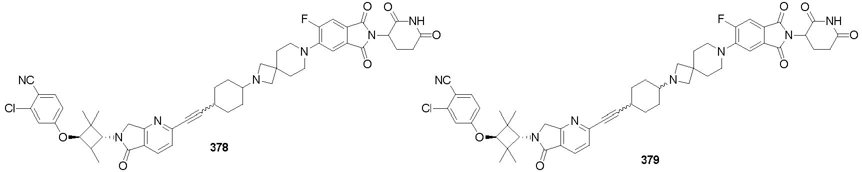

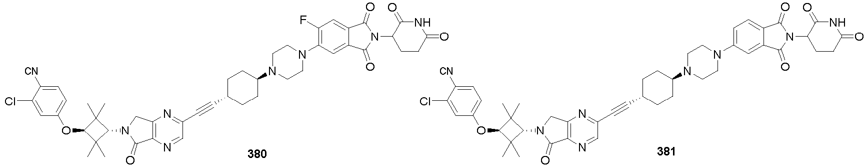

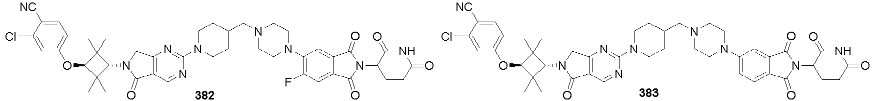

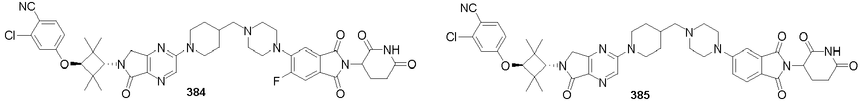

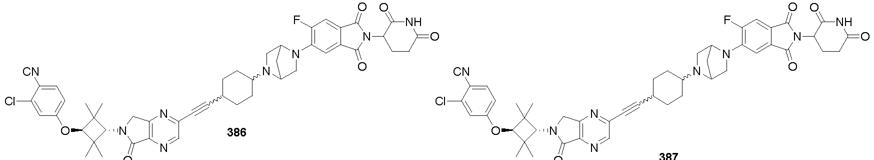

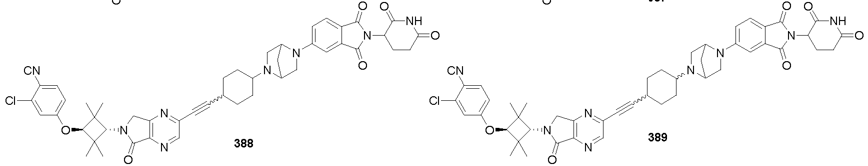

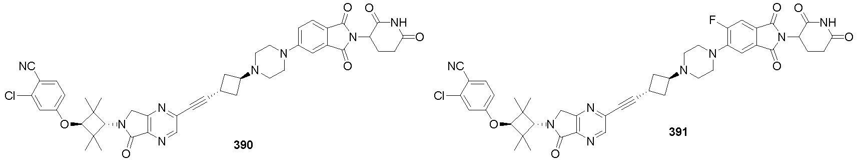

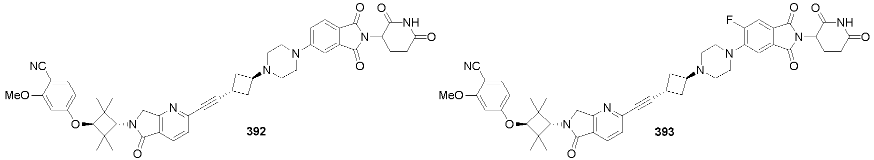

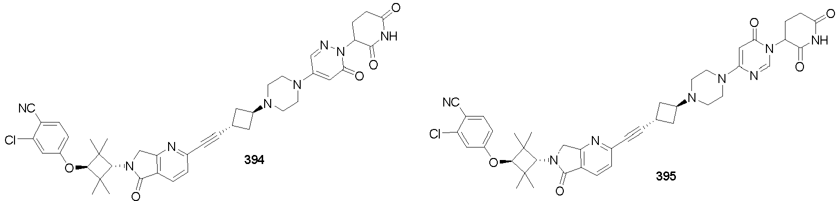

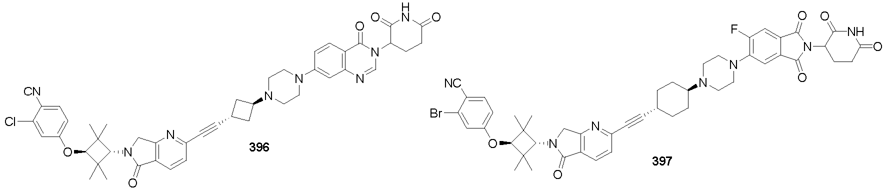

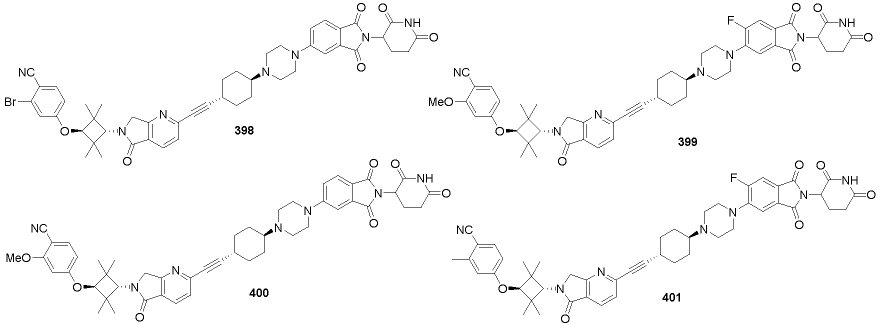

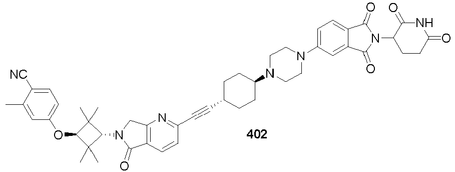

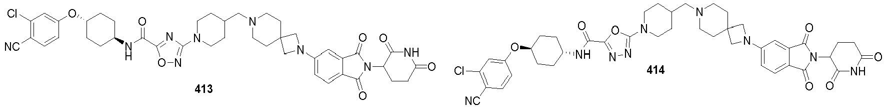

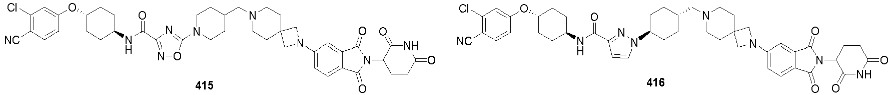

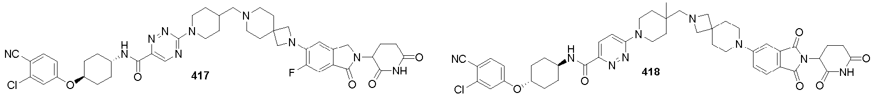

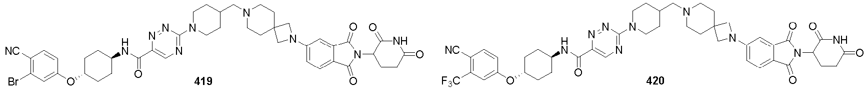

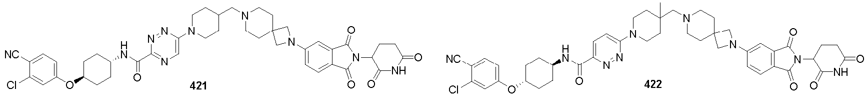

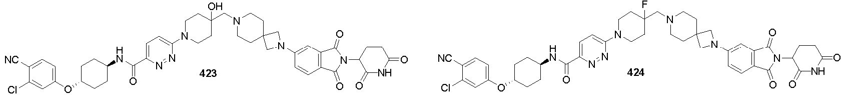

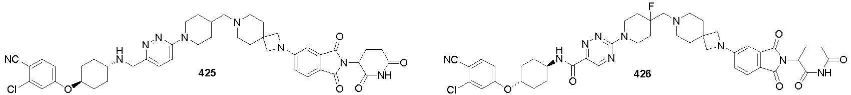

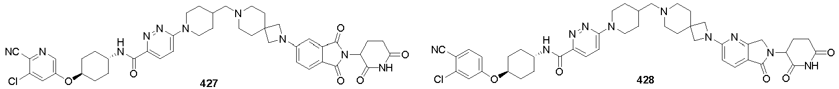

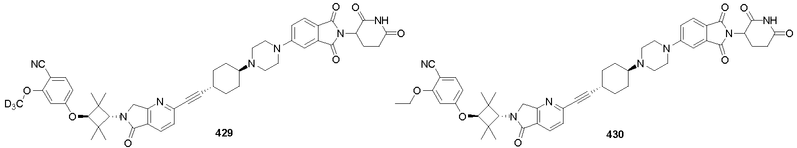

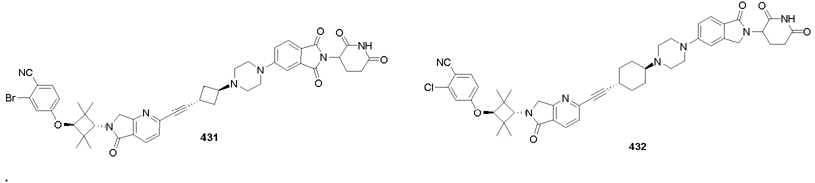

Further, said triple negative breast cancer (TNBC) is androgen receptor (AR)-positive TNBC.

The present invention also provides the use of the protein degradation agent targeting androgen receptor, in combination with targeted drugs, in the manufacture of medicaments for preventing and/or treating TNBC.

Further, the targeted drug is a PI3K inhibitor.

Further, the PI3K inhibitor is a PI3Kα inhibitor.

Further, the PI3K inhibitor is selected from the group consisting of following compounds, or optical isomers thereof, or tautomers thereof, or salts thereof, or prodrugs thereof, or hydrates thereof, or solvates thereof: Alpelisib, GDC-0077, TAK-117, AZD-8186, IPI-549, Idelalisib, Buparlisib, Pilaralisib, Copanlisib, PX-866, Paxalisib, Duvelisib, Umbralisib, Taselisib, Perifosine, Buparlisib, Dactolisib, CUDC-907, and Voxtalisib.

Further, the targeted drug is a PARP inhibitor.

Further, the PARP inhibitor is selected from the group consisting of Olaparib, Rupapani, Talazoparib, Niraparib, Pamiparib, or Fluzopanib.

Further, the targeted drug is Alpelisib, and the molar ratio of the protein degradation agent targeting AR to Alpelisib is 1:27-3:1;
alternatively, the targeted drug is Duvelisib, and the molar ratio of the protein degradation agent targeting AR to Duvelisib is 1:30-1:10;
alternatively, the targeted drug is Idelalisib, and the molar ratio of the protein degradation agent targeting AR to Idelalisib is 1:270-1:90.

The present invention also provides the use of the protein degradation agent targeting AR, in combination with immunotherapeutic drugs, in the manufacture of medicaments for preventing and/or treating TNBC.

The present invention also provides the use of the protein degradation agent targeting AR, in combination with chemotherapeutic drugs, in the manufacture of medicaments for preventing and/or treating TNBC.

Further, the chemotherapeutic drug is Paclitaxel or 5-Fluorouracil.

Further, the chemotherapeutic drug is Paclitaxel, and the molar ratio of the protein degradation agent targeting AR to Paclitaxel is 20:1-5000:1;
alternatively, the chemotherapeutic drug is 5-Fluorouracil, and the molar ratio of the protein degradation agent targeting AR to 5-Fluorouracil is 1.85:1-5.54:1.

Further, the TNBC is an AR-positive TNBC.

Further, the AR degradation agent is that mentioned above.

In the present invention, it is first discovered that the protein degrading agent targeting AR can effectively prevent and/or treat TNBC (including AR-positive TNBC), and has good prospects in the manufacture of medicaments for preventing and/or treating TNBC. In the present invention, it is first disclosed that the protein degradation agent targeting AR, in combination with targeted drugs (including PI3Kα inhibitors and PARP inhibitors), immunotherapeutic drugs or chemotherapeutic drugs, can effectively prevent and/or treat TNBC, and has good prospects in the manufacture of medicaments for preventing and/or treating TNBC.

For the definition of the terms used in the present invention: unless indicated otherwise, the initial definition provided for the group or the term herein is applicable to those in the whole specification; for terms not specifically defined herein, according to the disclosure content and the context, the term will have their common meaning as understood by one of ordinary skill in the art to which this invention pertains.

In the present invention, "aromatic ring" denotes all-carbon monocyclic group or fused polycyclic group with conjugated π electron system, such as phenyl and naphthyl. Said aromatic ring can be fused to other cyclic moieties (including saturated and unsaturated rings), but cannot contain heteroatoms such as nitrogen, oxygen, or sulfur. At the same time, the point linking to the parent must be on the carbon in the ring having the conjugated π electron system.

"Heteroaromatic ring" refers to a heteroaromatic group containing one or more heteroatoms. The heteroatoms, as used herein, include oxygen, sulfur, and nitrogen. For example, furanyl, thienyl, pyridinyl, pyrazolyl, pyrrolyl, N-alkylpyrrolyl, pyrimidyl, pyrazinyl, imidazolyl, tetrazolyl, etc. The heteroaryl ring can be fused to aryl, heterocyclic group, or cycloalkyl ring, wherein the ring linked to the parent structure is a heteroaryl ring.

In the present invention, the protein degradation agent targeting AR refers to the AR degradation agent.

"Pharmaceutically acceptable" means certain carriers, vehicles, diluents, excipients, and/or formed salts are usually chemically or physically compatible with other ingredients constituting certain pharmaceutical dosage forms, as well as physiologically compatible with the recipient.

"Salt" means acid and/or basic salt which is formed by reaction of compound or its stereoisomer with inorganic and/or organic acid and/or base, and also includes zwitterionic salts (inner salts), and further includes quaternary ammonium salts, such as alkylammonium salt. These salts can be directly obtained during the final isolation and purification of a compound. The salts can also be obtained by mixing the compound or its stereoisomers with a certain amount of acid or base appropriately (for example, in equivalent). These salts may form a precipitate in the solution, and be collected by filtration, or recovered after evaporation of the solvent, or obtained by freeze-drying after reaction in an aqueous medium. The salt in the present invention may be compounds' hydrochloride, sulfate, citrate, benzenesulfonate, hydrobromide, hydrofluoride, phosphate, acetate, propionate, succinate, oxalate, malate, succinate, fumarate, maleate, tartrate or trifluoroacetate.

"Solvate" means a solvate formed by the compound of the present invention and a solvent, wherein the solvent includes (but is not limited to) water, ethanol, methanol, isopropanol, propanediol, tetrahydrofuran, and dichloromethane.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Description of Figures

Figure 1. The inhibitory effects of compound **279** in combination with Alpelisib on the proliferation of MDA-MB-453 cells.
Figure 2. The inhibitory effects of compound **279** in combination with Alpelisib on the proliferation of MDA-MB-453 cells.
Figure 3. The inhibitory effects of compound **281** in combination with Alpelisib on the proliferation of MDA-MB-453 cells.
Figure 4. The inhibitory effects of compound **281** in combination with Alpelisib on the proliferation of MDA-MB-453 cells.
Figure 5. The inhibitory effects of compound **282** in combination with Alpelisib on the proliferation of MDA-MB-453 cells.
Figure 6. The inhibitory effects of compound **282** in combination with Alpelisib on the proliferation of MDA-MB-453 cells.
Figure 7. The inhibitory effects of ARV-110 in combination with Alpelisib on the proliferation of MDA-MB-453 cells.
Figure 8. The inhibitory effects of ARV-110 in combination with Alpelisib on the proliferation of MDA-MB-453 cells.
Figure 9. The inhibitory effects of ARV-766 in combination with Alpelisib on the proliferation of MDA-MB-453 cells.
Figure 10. The inhibitory effects of ARV-766 in combination with Alpelisib on the proliferation of MDA-MB-453 cells.
Figure 11. The inhibitory effects of compound **279** in combination with Duvelisib on the proliferation of MDA-MB-453 cells.
Figure 12. The inhibitory effects of compound **279** in combination with Duvelisib on the proliferation of MDA-MB-453 cells.
Figure 13. The inhibitory effects of compound **281** in combination with Duvelisib on the proliferation of MDA-MB-453 cells.
Figure 14. The inhibitory effects of compound **281** in combination with Duvelisib on the proliferation of MDA-MB-453 cells.
Figure 15. The inhibitory effects of compound **282** in combination with Duvelisib on the proliferation of MDA-MB-453 cells.
Figure 16. The inhibitory effects of compound **282** in combination with Duvelisib on the proliferation of MDA-MB-453 cells.
Figure 17. The inhibitory effects of ARV-110 in combination with Duvelisib on the proliferation of MDA-MB-453 cells.
Figure 18. The inhibitory effects of ARV-110 in combination with Duvelisib on the proliferation of MDA-MB-453 cells.
Figure 19. The inhibitory effects of compound **279** in combination with Idelalisib on the proliferation of MDA-MB-453 cells.
Figure 20. The inhibitory effects of compound **279** in combination with Idelalisib on the proliferation of MDA-MB-453 cells.
Figure 21. The inhibitory effects of compound **281** in combination with Idelalisib on the proliferation of MDA-MB-453 cells.
Figure 22. The inhibitory effects of compound **281** in combination with Idelalisib on the proliferation of MDA-MB-453 cells.
Figure 23. The inhibitory effects of compound **282** in combination with Idelalisib on the proliferation of MDA-MB-453 cells.
Figure 24. The inhibitory effects of compound **282** in combination with Idelalisib on the proliferation of MDA-MB-453 cells.
Figure 25. The inhibitory effects of ARV-110 in combination with Idelalisib on the proliferation of MDA-MB-453 cells.
Figure 26. The inhibitory effects of ARV-110 in combination with Idelalisib on the proliferation of MDA-MB-453 cells.
Figure 27. The inhibitory effects of ARV-766 in combination with Idelalisib on the proliferation of MDA-MB-453 cells.
Figure 28. The inhibitory effects of ARV-766 in combination with Idelalisib on the proliferation of MDA-MB-453 cells.
Figure 29. The inhibitory effects of compound **279** in combination with Paclitaxel on the proliferation of MDA-MB-453 cells.
Figure 30. The inhibitory effects of compound **279** in combination with Paclitaxel on the proliferation of MDA-MB-453 cells.
Figure 31. The inhibitory effects of compound **281** in combination with Paclitaxel on the proliferation of MDA-MB-453 cells.
Figure 32. The inhibitory effects of compound **282** in combination with Paclitaxel on the proliferation of MDA-MB-453 cells.
Figure 33. The inhibitory effects of ARV-110 in combination with Paclitaxel on the proliferation of MDA-MB-453 cells.

### Examples

The starting materials and equipment used in the present invention are all known products and can be obtained by purchasing those commercially available.

### Example 1: Effect of AR protein degrading agent on TNBC (in vitro experiment)

### 1. Experimental methods

Triple negative breast cancer MDA-MB-453 cells in logarithmic growth stage were inoculated into a 96-well plate at a concentration of 4×10³ cells/well, and then incubated in an incubator at 37 °C, 5% CO₂, and saturated humidity for 24 h or 48 h. To the negative control well, was added RPMI 1640 cell medium containing 10% fetal bovine serum in the same volume as the drug. After cultured for 24 h or 48 h, to the 96-well plate, were added AR PROTACs compounds **162, 177, 230, 259, 279, 281, 282, 325, 357, 400, ARV-110, ARV-766** and AR inhibitor **HC-1119,** respectively. The concentrations of compounds **177, 230, 259, 279, 281, 282, 325, 357, 400, ARV-110, and ARV-766** are 0 µM, 0.002 µM, 0.005 µM, 0.014 µM, 0.041 µM, 0.123 µM, 0.370 µM, 1.111µM, 3.333 µM or 10 µM; while the concentrations of HC-1119 are 0 µM, 1.23 µM, 3.70 µM, 11.11 µM, 33.33 µM or 100 µM.

After addition of the drugs and co-incubation for 7-10 days, 10 µL of CCK-8 was added to each well, and then the plate was further cultured for 2 h. The absorbance value (OD) of each well was measured using a microplate reader at a wavelength of 450 nm. The survival rate was calculated using Prism software, the relative survival rate = (OD value of drug well/OD value of control well) × 100%. Alternatively, 100 µL of medium was removed from each well, and then 50 µL of CTG was added. The plate was shaken in the dark for 2 min, allowed to stand for 10 min, and then measured using a microplate reader in Luminescence mode. Prism software was used to calculate the survival rate, the relative survival rate = (the RLU value of drug well / the RLU value of control well) × 100%.

### 2. Experimental results

### (1) IC₅₀ values of compounds 177, 230, 259, 279, 281, 282, 325, 357, 400, ARV-110, and ARV-766 were obtained against MDA-MB-453 cells.

IC₅₀ values of compounds **177, 230, 259, 279, 281, 282, 325, 357, 400, ARV-110,** and **ARV-766** against MDA-MB-453 cells are shown in Table 1, and the judgment criteria were as follows: when IC₅₀ value ≤ 2 µM, it was expressed as A, while when IC₅₀ value > 2 µM, it was expressed as B. As shown in Table 1, compounds **177, 230, 259, 279, 281, 282, 325, 357, 400, ARV-110,** and **ARV-766** could inhibit the proliferation of MDA-MB-453 cells to varying degrees, and could be used to effectively treat TNBC.

**Table 1**

| IC₅₀ values against MDA-MB-453 cells | |
|---|---|
| Compound | IC₅₀ |
| Compound **162** | B |
| Compound **177** | B |
| Compound **230** | B |
| Compound **259** | B |
| Compound **279** | A |
| Compound **281** | A |
| Compound **282** | A |
| Compound **325** | B |
| Compound **357** | A |
| Compound **400** | A |
| **ARV-110** | A |
| **ARV-766** | A |
| **HC-1119** | B |

### (2) IC₅₀ value of HC-1119 against MDA-MB-453 cells

The IC₅₀ value of **HC-1119** against MDA-MB-453 cells is shown in Table 1, and the judgment criteria were as follows: when IC₅₀ value ≤ 2 µM, it was expressed as A, while when IC₅₀ value > 2 µM, it was expressed as B. As shown in Table 1, HC-1119 has a weak inhibitory effect on the proliferation of MDA-MB-453 cells, indicating that the effect of AR inhibitor **HC-1119** on TNBC was significantly weaker than that of AR PROTACs compounds **177, 230, 259, 279, 281, 282, 325, 357, 400, ARV-110,** and **ARV-766.**

### Example 2: Effect of AR protein degrading agent in combination with PI3K inhibitor Alpelisib on TNBC (in vitro experiment)

### 1. Experimental methods

Triple negative breast cancer MDA-MB-453 cells in logarithmic growth stage were inoculated into a 96-well plate at a concentration of 4×10³ cells/well, and then incubated in an incubator at 37 °C, 5% CO₂, and saturated humidity for 24 h. To the negative control wells, were added RPMI 1640 cell culture medium containing 10% fetal bovine serum in the same volume as the drug. After cultured for 24 h, to the 96-well plate, were sequentially added AR PROTACs compounds **279, 281, 282, ARV-110, ARV-766** and PI3K inhibitor Alpelisib, respectively.

The concentrations of compounds **279, 281, 282, ARV-110,** and **ARV-766** were: 0 µM, 0.002 µM, 0.005 µM, 0.014 µM, 0.041 µM, 0.123 µM, 0.370 µM, 1.111µM, 3.333 µM or 10 µM; the concentration of Alpelisib was: 0 µM, 0.123 µM, 0.370 µM, 1.111 µM, 3.333 µM or 10 µM.

After addition of the drugs and co-incubation for 7 days, 10 µL of CCK-8 was added to each well, and then the plate was further cultured for 2 h. The absorbance value (OD) of each well was measured using a microplate reader at a wavelength of 450 nm. The survival rate was calculated using Prism software, the relative survival rate = (OD value of drug well/OD value of control well) × 100%. Alternatively, 100 µL of medium was removed from each well, and then 50 µL of CTG was added. The plate was shaken in the dark for 2 min, allowed to stand for 10 min, and then measured using a microplate reader in Luminescence mode. Prism software was used to calculate the survival rate, the relative survival rate = (the RLU value of drug well / the RLU value of control well) × 100%. Then, the experimental results were determined according to the drug Combination Index (CI), with the following criteria: when CI < 1, it was indicated that the drug combination had played a synergistic effect.

### 2. Experimental results

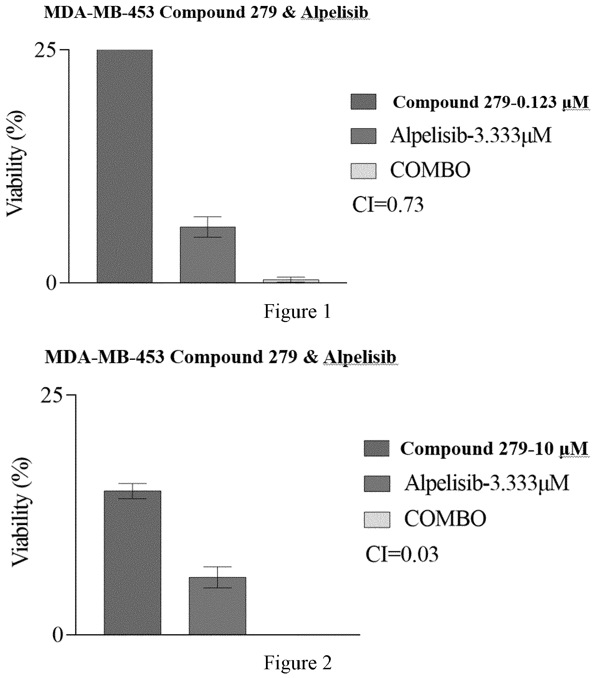

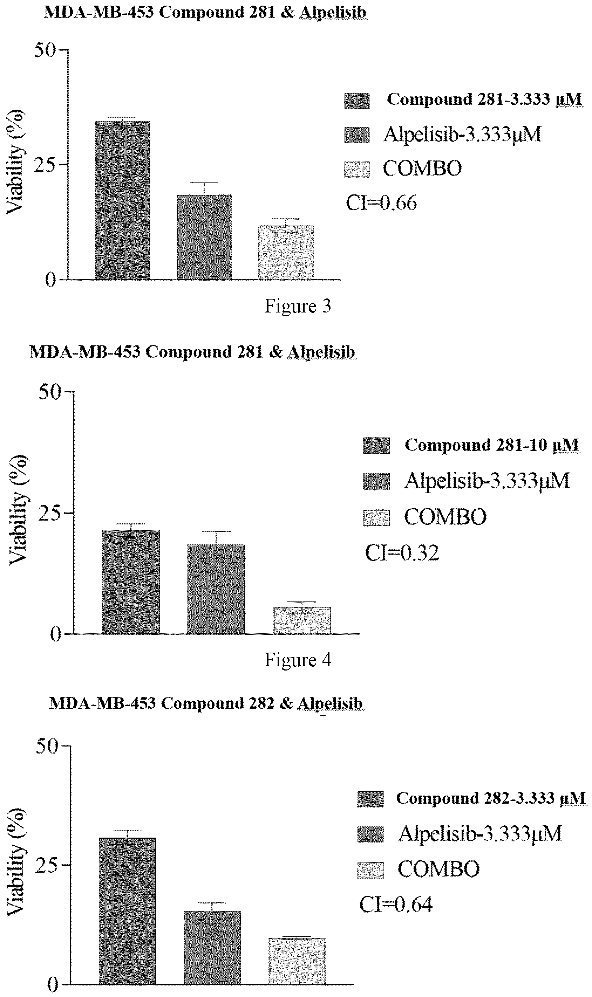

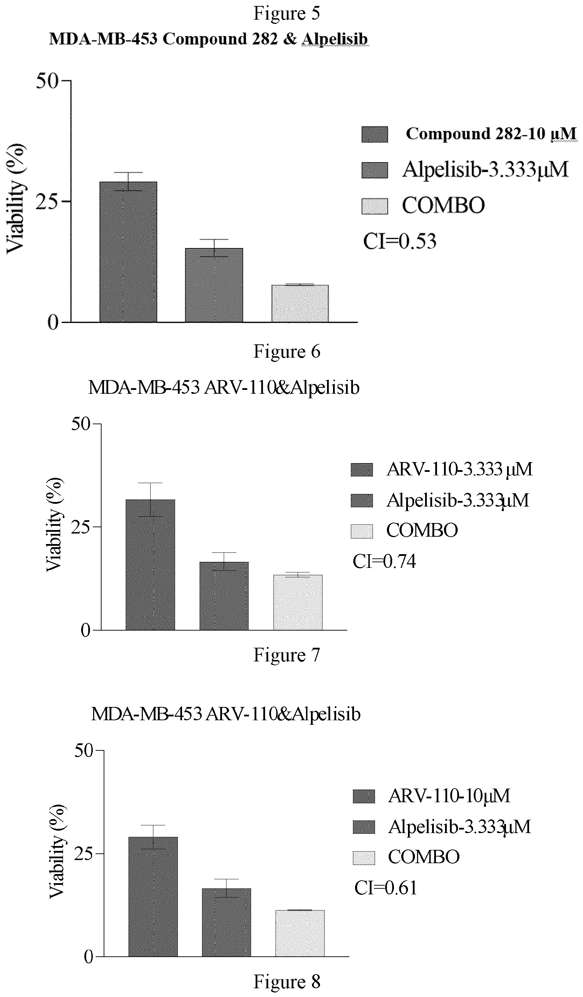

The results for the combination of compounds **279, 281, 282, ARV-110, ARV-766** with Alpelisib are shown in Figures 1-10, respectively, and as shown, when the molar ratio of compounds **279, 281, 282, ARV-110, ARV-766** to Alpelisib was 1:27-9:1, the drug combination of compounds **279, 281, 282, ARV-110, ARV-766** with Alpelisib had synergistic effect on inhibiting the proliferation of MDA-MB-453 cells, and thus could be used for synergistic treatment of TNBC.

### Example 3: Effect of AR protein degrading agent in combination with PI3K inhibitor Duvelisib on TNBC (in vitro experiment)

### 1. Experimental methods

Triple negative breast cancer MDA-MB-453 cells in logarithmic growth stage were inoculated into a 96-well plate at a concentration of 4×10³ cells/well, and then incubated in an incubator at 37 °C, 5% CO₂, and saturated humidity for 24 h. To the negative control wells, were added RPMI 1640 cell culture medium containing 10% fetal bovine serum in the same volume as the drug. After cultured for 24 h, to the 96-well plate, were sequentially added AR PROTACs compounds **279, 281, 282, ARV-110** and PI3K inhibitor Duvelisib, respectively.

The concentrations of compounds **279, 281, 282,** and **ARV- 110** were: 0 µM, 0.002 µM, 0.005 µM, 0.014 µM, 0.041 µM, 0.123 µM, 0.370 µM, 1.111 µM, 3.333 µM or 10 µM; the concentration of Duvelisib was: 0 µM, 1.23 µM, 3.70 µM, 11.11 µM, 33.33 µM or 100 µM.

After adding the drugs and co-incubating for 7-10 days, 10 µL of CCK-8 was added to each well, and then the plate was further cultured for 2 h. The absorbance value (OD) of each well was measured using a microplate reader at a wavelength of 450 nm. The survival rate was calculated using Prism software, the relative survival rate = (OD value of drug well/OD value of control well) × 100%. Alternatively, 100 µL of medium was removed from each well, and then 50 µL of CTG was added. The plate was shaken in the dark for 2 min, allowed to stand for 10 min, and then measured using a microplate reader in Luminescence mode. Prism software was used to calculate the survival rate, the relative survival rate = (the RLU value of drug well / the RLU value of control well) × 100%. Then, the experimental results were determined according to the drug Combination Index (CI), with the following criteria: when CI < 1, it was indicated that the drug combination had played a synergistic effect.

### 2. Experimental results

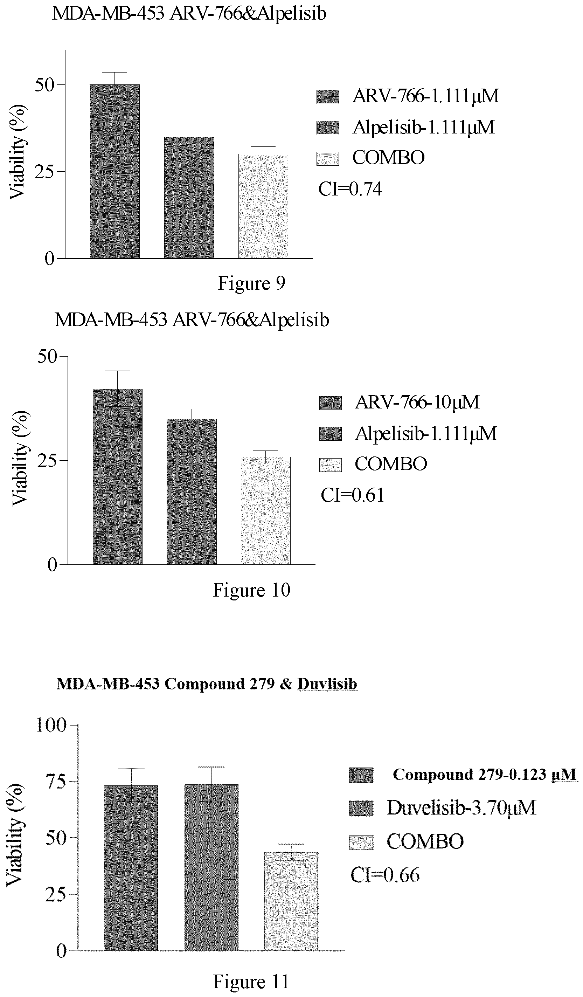

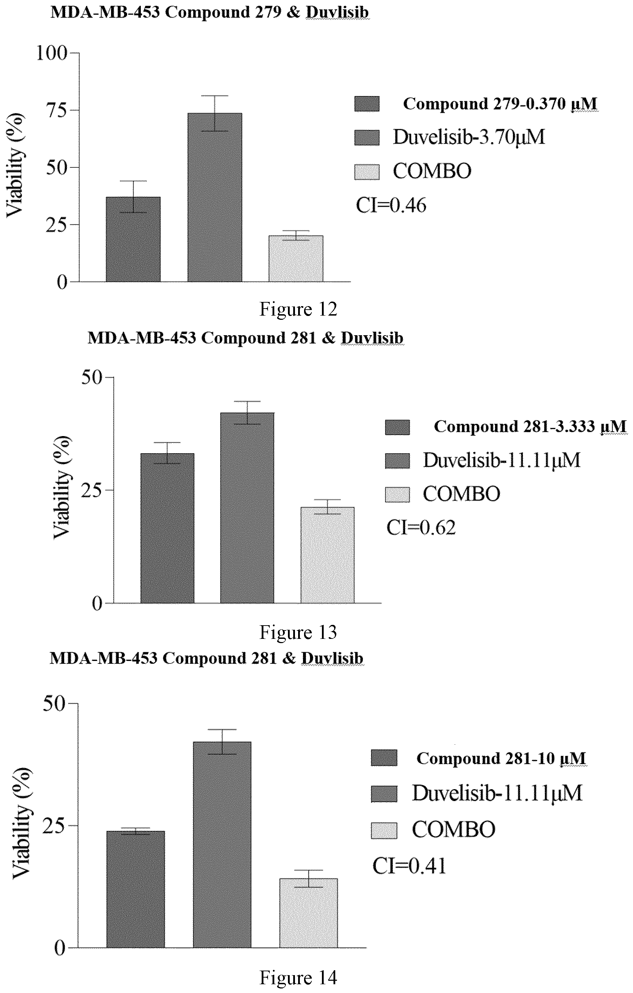

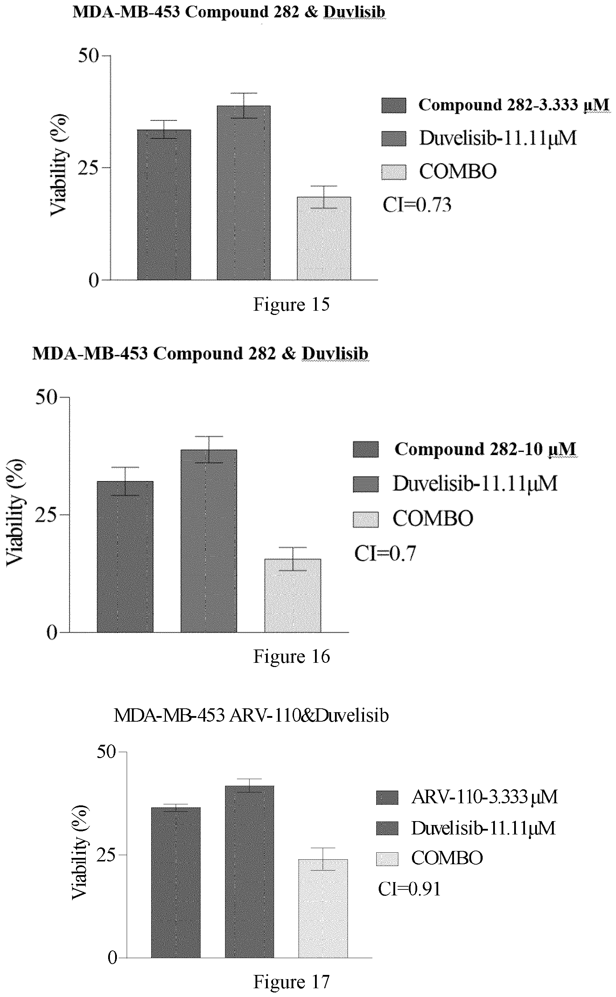

The results for the combination of compounds **279, 281, 282, ARV-110** with Duvelisib are shown in Figures 11-18, respectively, and as shown, when the molar ratio of compounds **279, 281, 282, ARV-110** to Duvelisib was 1:30-1:1.111, the drug combination of compounds **279, 281, 282, ARV-110** with Alpelisib had synergistic effect on inhibiting the proliferation of MDA-MB-453 cells, and thus could be used for synergistic treatment of TNBC.

### Example 4: Effect of AR protein degrading agent in combination with PI3K inhibitor Idelalisib on TNBC (in vitro experiment)

### 1. Experimental methods

Triple negative breast cancer MDA-MB-453 cells in logarithmic growth stage were inoculated into a 96-well plate at a concentration of 4×10³ cells/well, and then incubated in an incubator at 37 °C, 5% CO₂, and saturated humidity for 24 h. To the negative control wells, were added RPMI 1640 cell culture medium containing 10% fetal bovine serum in the same volume as the drug. After cultured for 24 h, to the 96-well plate, were sequentially added AR PROTACs compounds **279, 281, 282, ARV-110, ARV-766** and PI3K inhibitor Idelalisib, respectively.

The concentrations of compounds **279, 281, 282, ARV-110,** and **ARV-766** were: 0 µM, 0.002 µM, 0.005 µM, 0.014 µM, 0.041 µM, 0.123 µM, 0.370 µM, 1.111 µM, 3.333 µM or 10 µM; the concentration of Idelalisib was: 0 µM, 1.23 µM, 3.70 µM, 11.11 µM, 33.33 µM or 100 µM.

After adding the drugs and co-incubating for 7-10 days, 10 µL of CCK-8 was added to each well, and then the plate was further cultured for 2 h. The absorbance value (OD) of each well was measured using a microplate reader at a wavelength of 450 nm. The survival rate was calculated using Prism software, the relative survival rate = (OD value of drug well/OD value of control well) × 100%. Alternatively, 100 µL of medium was removed from each well, and then 50 µL of CTG was added. The plate was shaken in the dark for 2 min, allowed to stand for 10 min, and then measured using a microplate reader in Luminescence mode. Prism software was used to calculate the survival rate, the relative survival rate = (the RLU value of drug well / the RLU value of control well) × 100%. Then, the experimental results were determined according to the drug Combination Index (CI), with the following criteria: when CI < 1, it was indicated that the drug combination had played a synergistic effect.

### 2. Experimental results

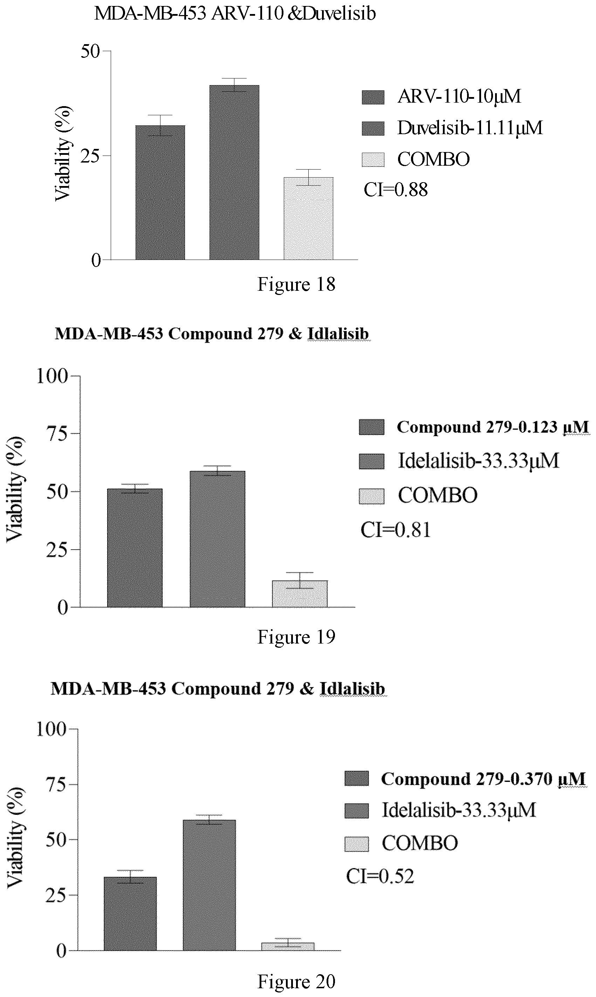

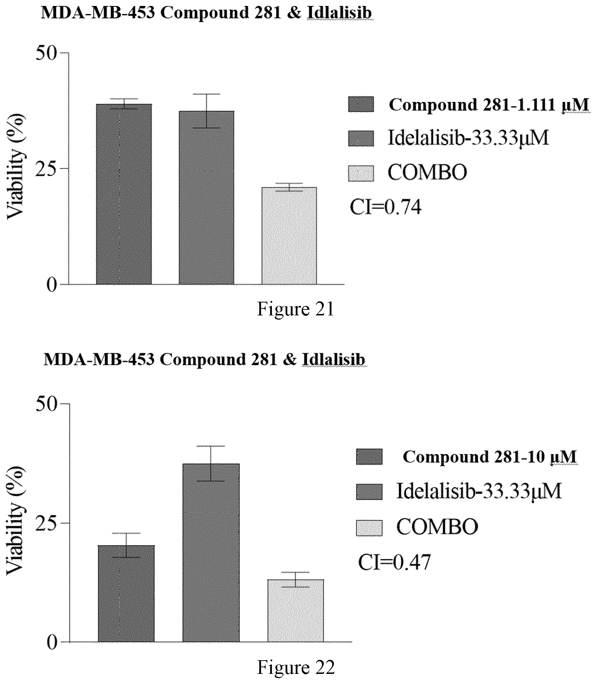

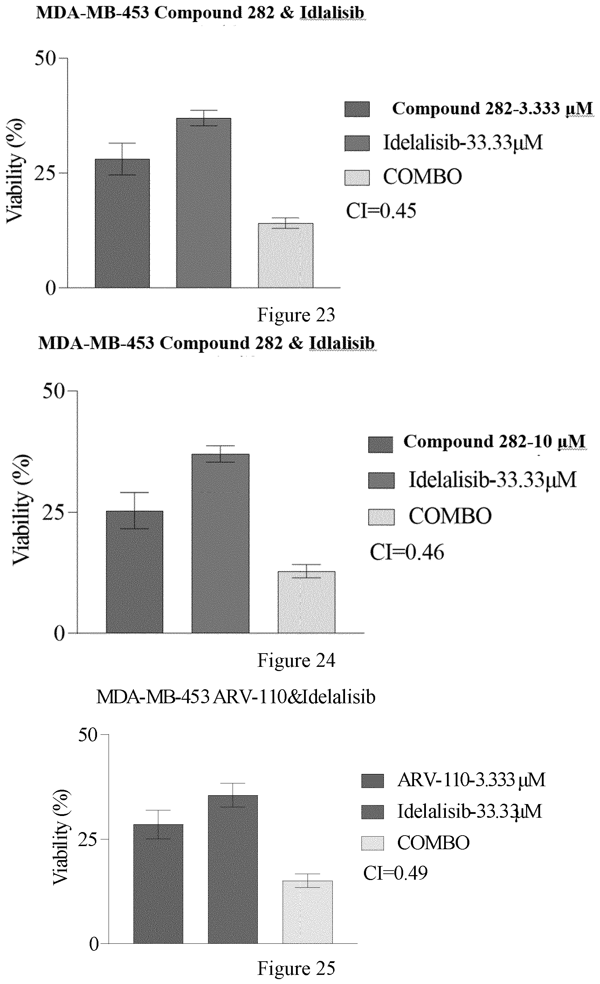

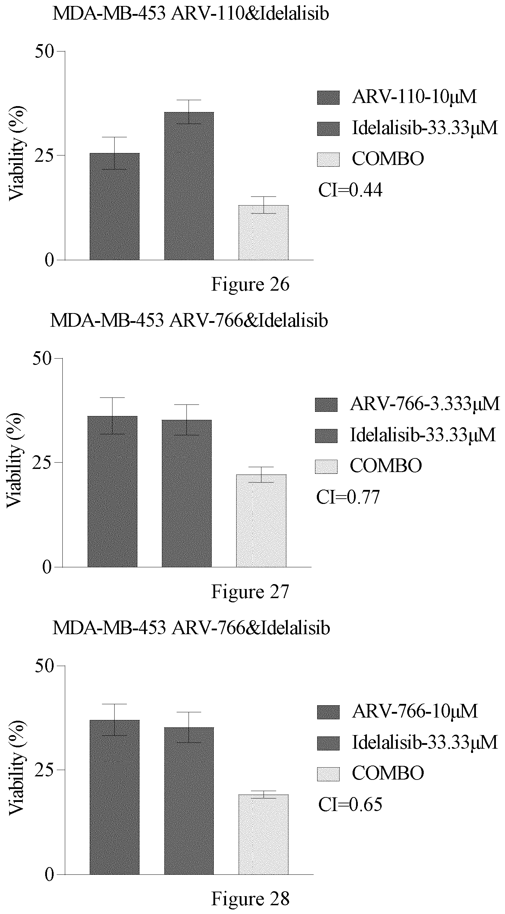

The results for the combination of compounds **279, 281, 282, ARV-110, ARV-766** with Idelalisib are shown in Figures 19-28, respectively, and as shown, when the molar ratio of compounds **279, 281, 282, ARV-110, ARV-766** to Idelalisib was 1:270-1:3.333, the drug combination of compounds **279, 281, 282, ARV-110, ARV-766** with Idelalisib had synergistic effect on inhibiting the proliferation of MDA-MB-453 cells, and thus could be used for synergistic treatment of TNBC.

### Example 5: Effect of AR protein degrading agent in combination with chemotherapeutic drug Paclitaxel on TNBC (in vitro experiment)

### 1. Experimental methods

Triple negative breast cancer MDA-MB-453 cells in logarithmic growth stage were inoculated into a 96-well plate at a concentration of 4×10³ cells/well, and then incubated in an incubator at 37 °C, 5% CO₂, and saturated humidity for 24 h. To the negative control wells, were added RPMI 1640 cell culture medium containing 10% fetal bovine serum in the same volume as the drug. After cultured for 24 h, to the 96-well plate, were sequentially added AR PROTACs compounds **279, 281, 282, ARV-110** and chemotherapeutic drug Paclitaxel, respectively.

The concentrations of compounds **279, 281, 282,** and **ARV- 110** were: 0 µM, 0.002 µM, 0.005 µM, 0.014 µM, 0.041 µM, 0.123 µM, 0.370 µM, 1.111 µM, 3.333 µM or 10 µM; the concentration of Paclitaxel was: 0 nM, 0.025 nM, 0.074 nM, 0.222 nM, 0.667 nM or 2 nM.

After adding the drugs and co-incubating for 7 days, 10 µL of CCK-8 was added to each well, and then the plate was further cultured for 2 h. The absorbance value (OD) of each well was measured using a microplate reader at a wavelength of 450 nm. The survival rate was calculated using Prism software, the relative survival rate = (OD value of drug well/OD value of control well) × 100%. Alternatively, 100 µL of medium was removed from each well, and then 50 µL of CTG was added. The plate was shaken in the dark for 2 min, allowed to stand for 10 min, and then measured using a microplate reader in Luminescence mode. Prism software was used to calculate the survival rate, the relative survival rate = (the RLU value of drug well / the RLU value of control well) × 100%. Then, the experimental results were determined according to the drug Combination Index (CI), with the following criteria: when CI < 1, it was indicated that the drug combination had played a synergistic effect.

### 2. Experimental results

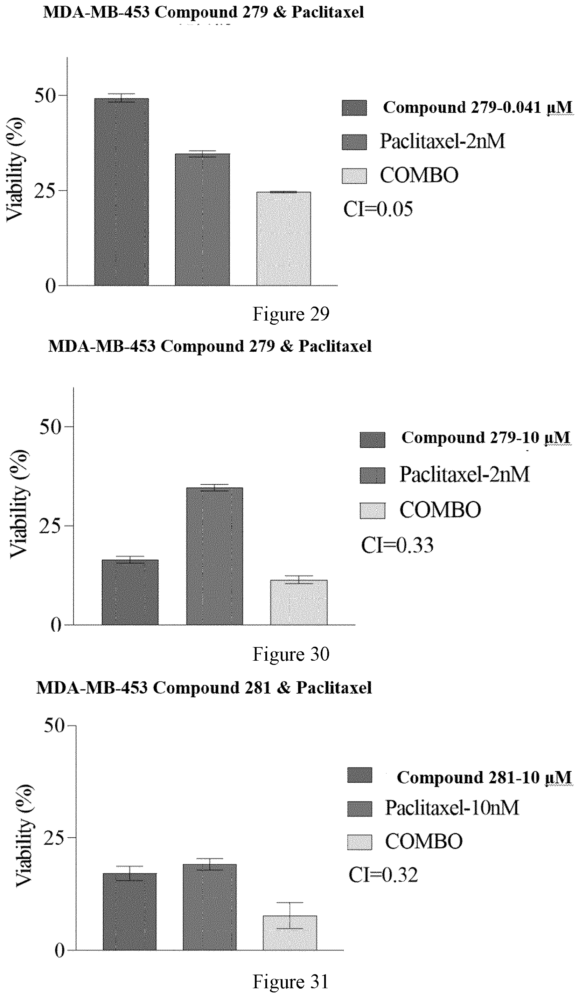

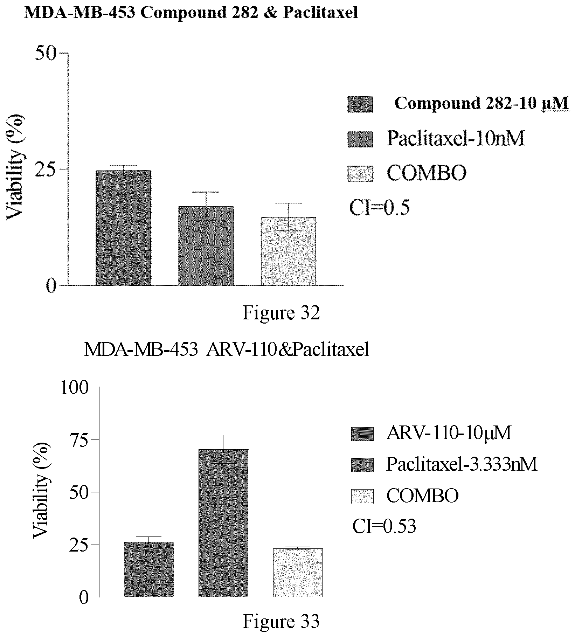

The results for the combination of compounds **279, 281, 282, ARV-110** with Paclitaxel are shown in Figures 19-36, respectively, and as shown, when the molar ratio of compounds **279, 281, 282, ARV-110** to Paclitaxel was 20:1-5000:1, the drug combination of compounds **279, 281, 282, ARV-110** with Paclitaxel had synergistic effect on inhibiting the proliferation of MDA-MB-453 cells, and thus could be used for synergistic treatment of TNBC.

In summary, in the present invention, it was first discovered that the protein degrading agent targeting AR could effectively prevent and/or treat TNBC (including AR-positive TNBC), and has good prospects in the manufacture of medicaments for preventing and/or treating TNBC. In the present invention, it was first disclosed that the protein degradation agent targeting AR, in combination with targeted drugs (including PI3Kα inhibitors and PARP inhibitors), immunotherapeutic drugs or chemotherapeutic drugs, could effectively prevent and/or treat TNBC, and has good prospects in the manufacture of medicaments for preventing and/or treating TNBC.

## Claims

1. A CRBN E3 ligase ligand-based protein degradation agent targeting androgen receptor for use in the manufacture of medicaments for the prevention and/or treatment of triple negative breast cancer.

2. The use according to claim 1, **characterized in that** the protein degradation agent targeting androgen receptor is a PROTAC bifunctional chimeric molecule represented by formula (I), or an optical isomer thereof, a tautomer thereof, or a pharmaceutically acceptable salt thereof: 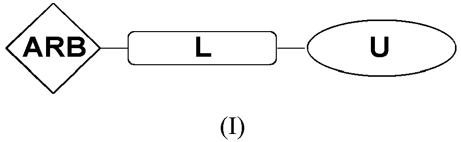 wherein ARB is a ligand for androgen receptor, L is a linker, and U is a ligand for CRBN E3 ligase.

3. The use according to claim 2, **characterized in that** said U is a CRBN E3 ligase ligand with the following structure: 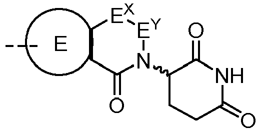
wherein E is selected from the group consisting of unsubstituted and substituted benzene ring, 5-membered heteroaromatic ring, 6-membered heteroaromatic ring, benzoheteroaromatic ring, and benzene-fused unsaturated heterocycle;
E^{X} is selected from the group consisting of absence, halogen, O, S, N, carbonyl, and - (CH₂)-;
E^{Y} is selected from the group consisting of absence, H, and carbonyl.

4. The use according to claim 2 or 3, **characterized in that** the structure of said U is selected from the group consisting of:

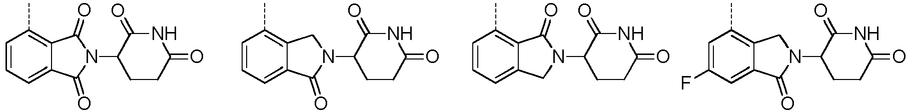

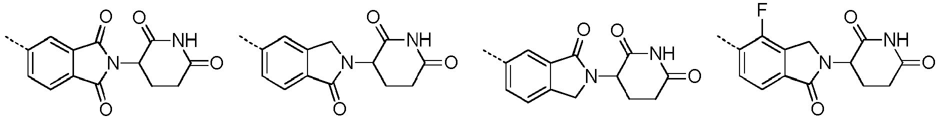

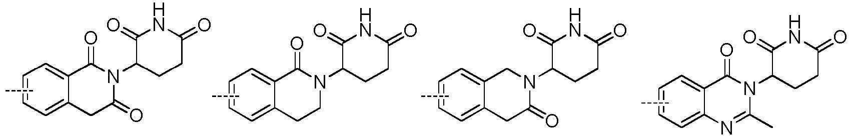

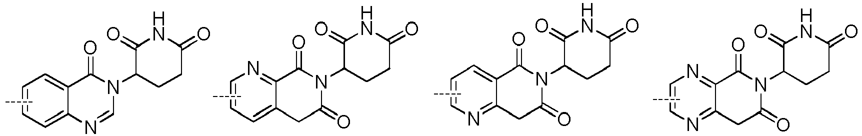

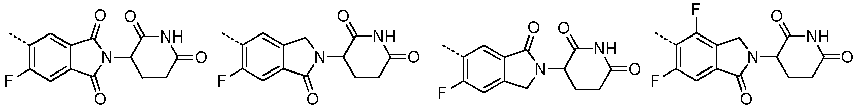

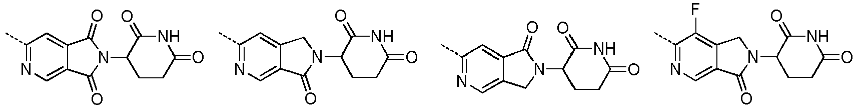

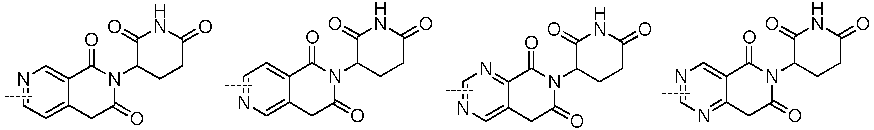

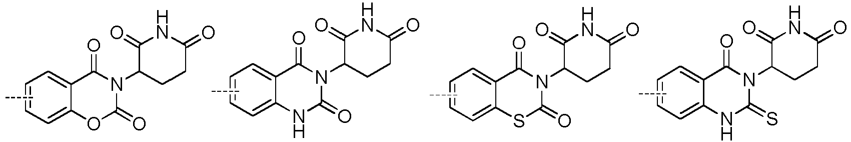

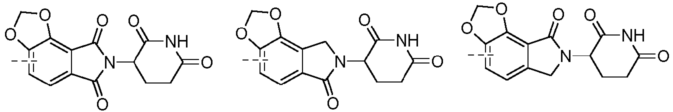

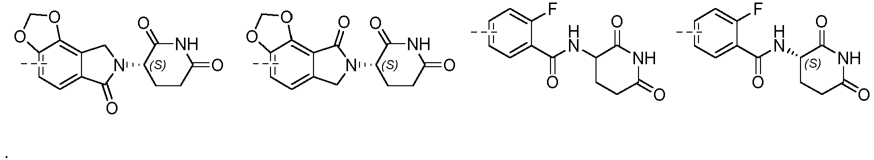

5. The use according to claim 2, **characterized in that** the PROTAC bifunctional chimeric molecule is selected from the group consisting of ARV-110, ARV-766, HP518, AC0176, GT20029, ASN-1780, ARD-2128, and ARD-2585.

6. The use according to claim 2, **characterized in that** the structure of said PROTAC bifunctional chimeric molecule is as represented by formula (II): 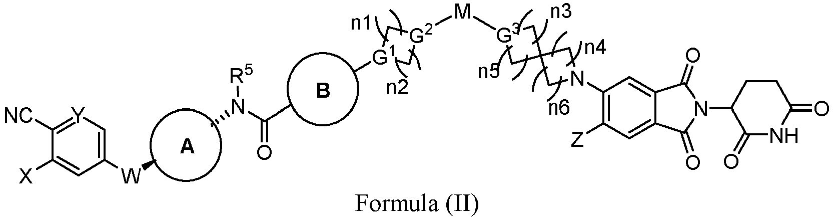
wherein X is selected from the group consisting of F, Cl, Br, Me, and CF₃;
Y is selected from CH or N;
W is selected from the group consisting of O, S, and NMe;
ring A is selected from the group consisting of the following groups substituted with R¹, R², R³ and/or R⁴: cyclopropane, cyclobutane, cyclopentane, cyclohexane, and cycloheptane;
R¹, R², R³, R⁴, and R⁵ are each independently selected from H and Me;
ring B is selected from the group consisting of 5-membered heteroaromatic ring, 6-membered aromatic ring, and 6-membered heteroaromatic ring;
G¹, G², and G³ are each independently selected from the group consisting of CR⁶ and N; wherein R⁶ is selected from the group consisting of H, halogen, and OH;
n1, n2, n3, n4, n5, and n6 are each independently selected from 1 or 2;
M is selected from the group consisting of CR⁷R⁸, wherein R⁷ and R⁸ are each independently selected from H and Me;
Z is selected from the group consisting of H, F, Cl, and Me.

7. The use according to claim 2, **characterized in that** the structure of said PROTAC bifunctional chimeric molecule is selected from the group consisting of:

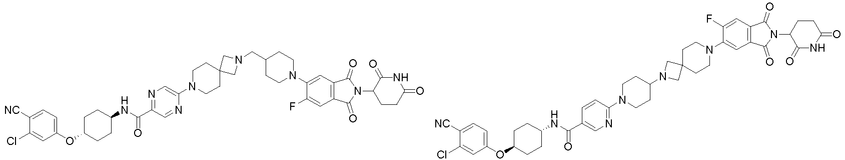

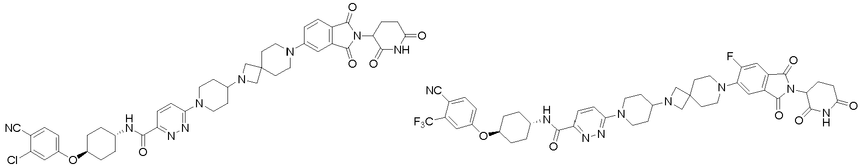

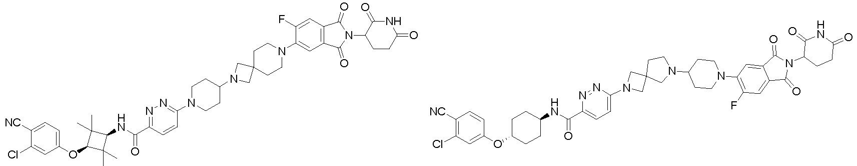

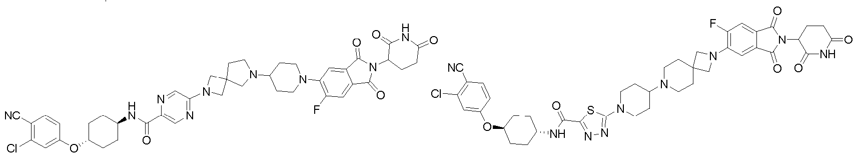

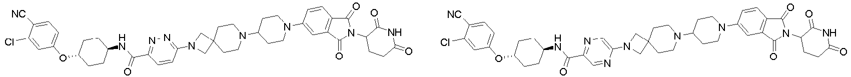

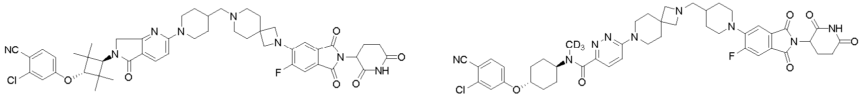

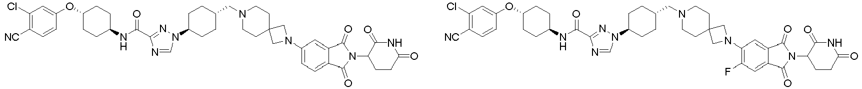

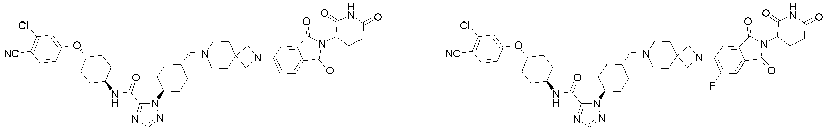

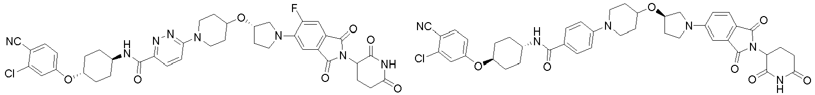

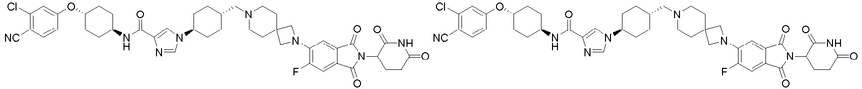

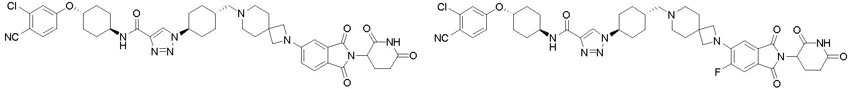

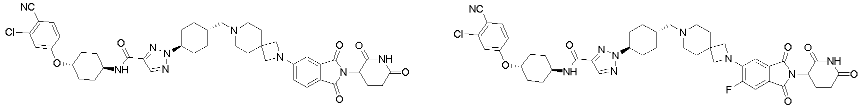

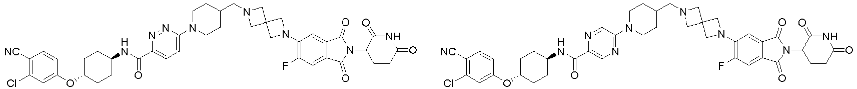

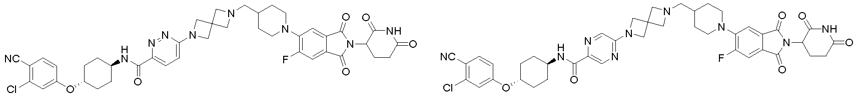

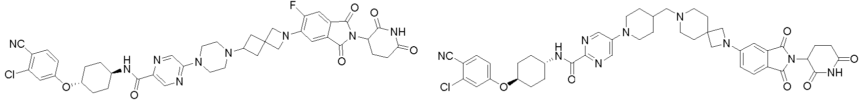

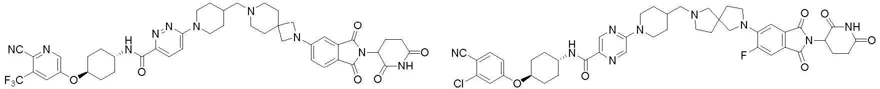

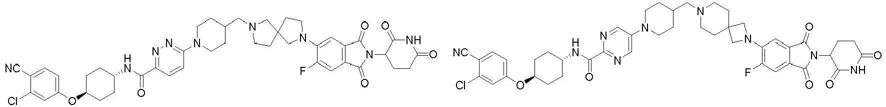

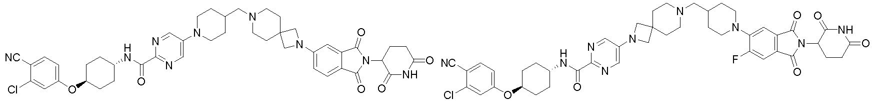

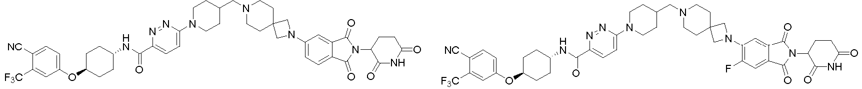

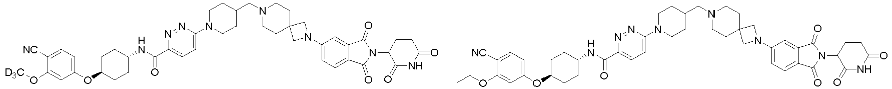

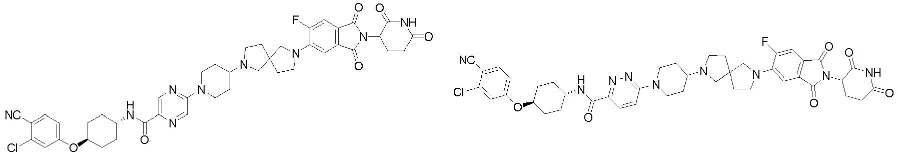

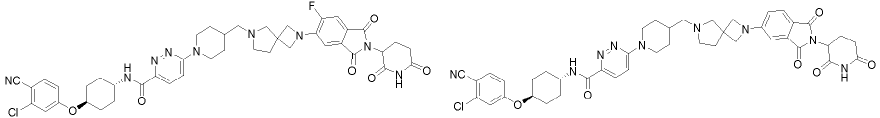

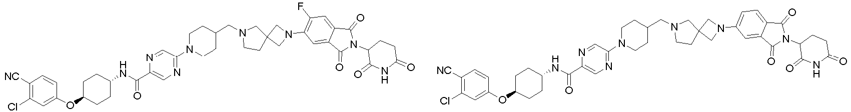

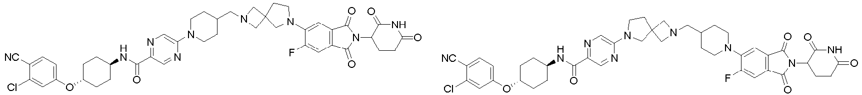

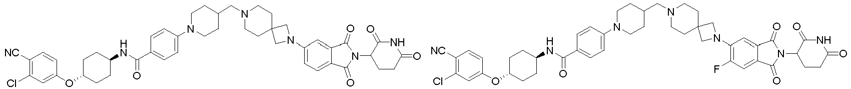

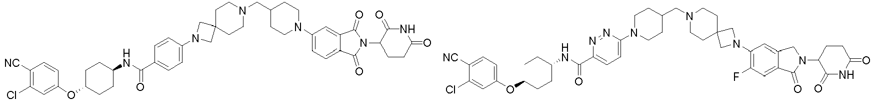

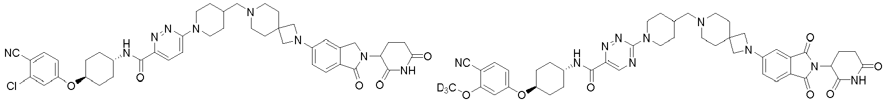

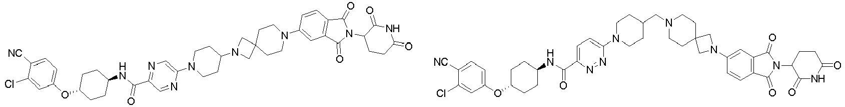

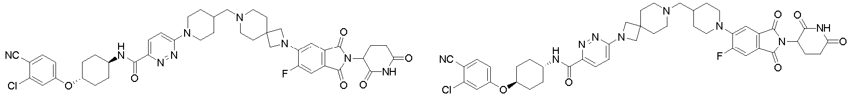

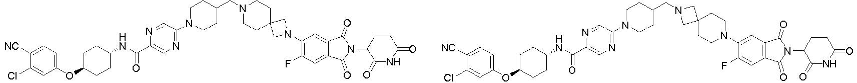

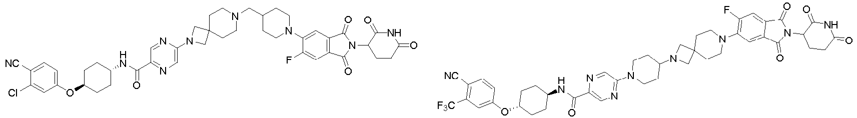

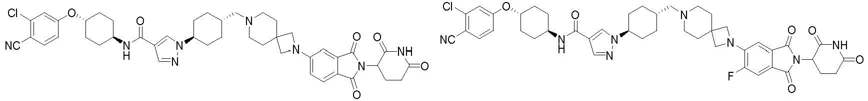

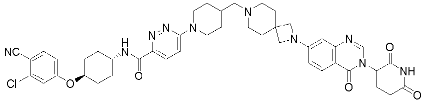

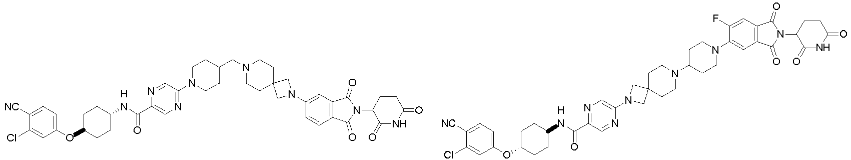

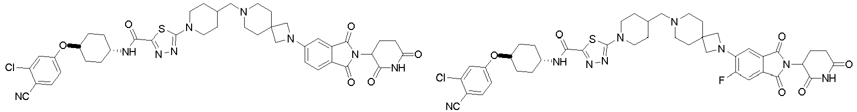

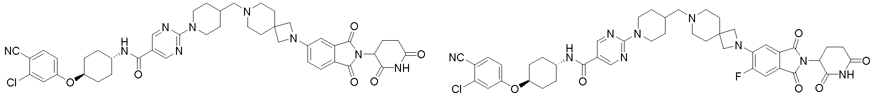

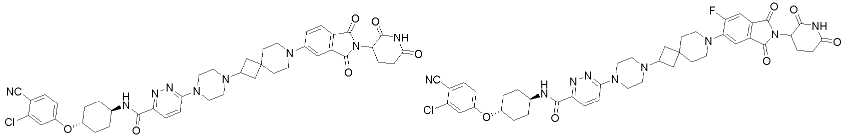

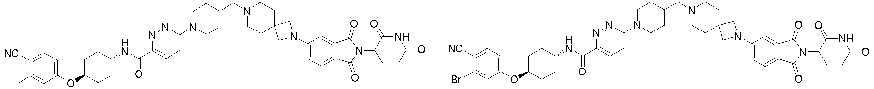

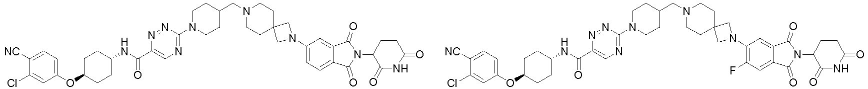

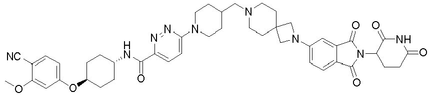

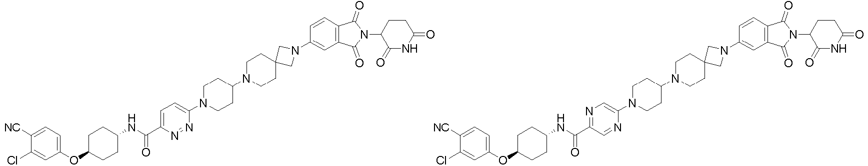

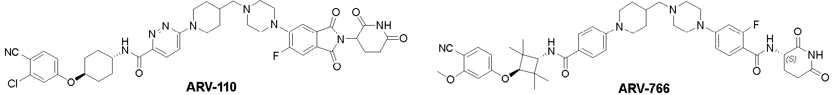

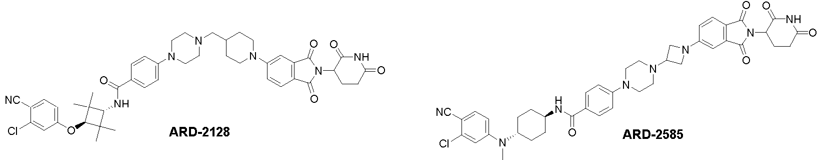

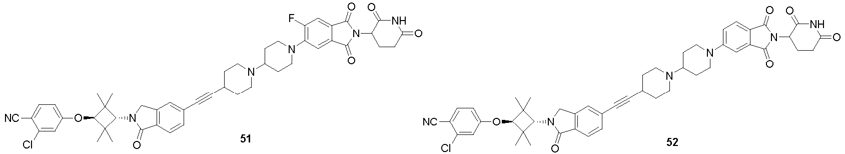

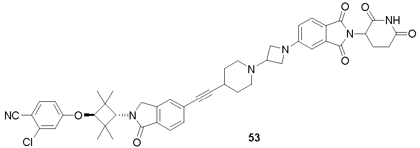

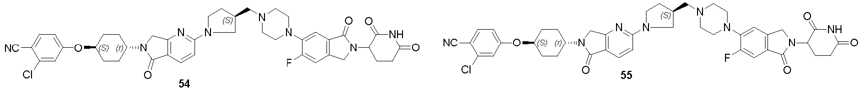

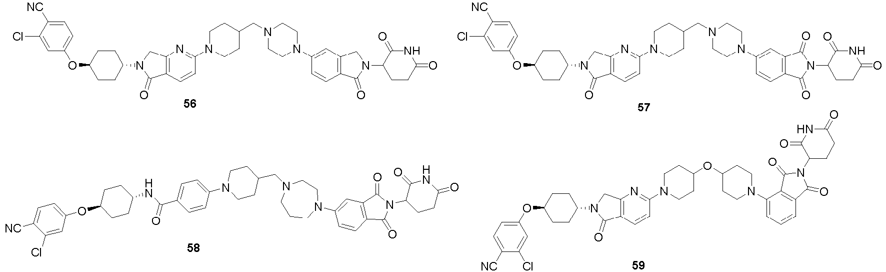

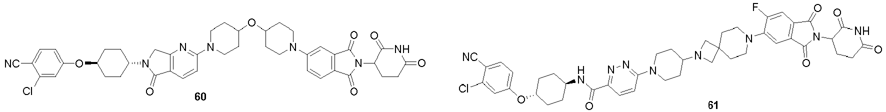

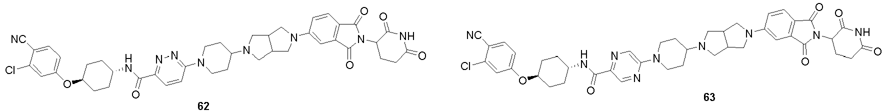

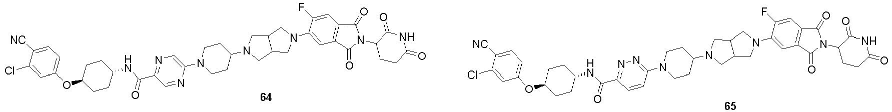

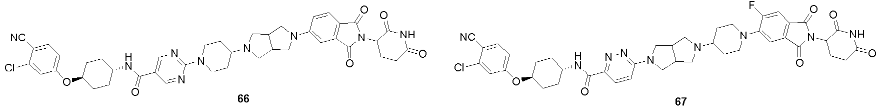

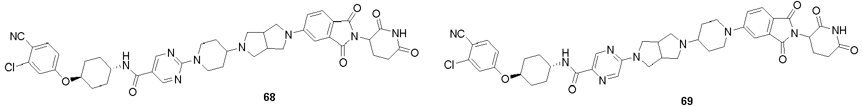

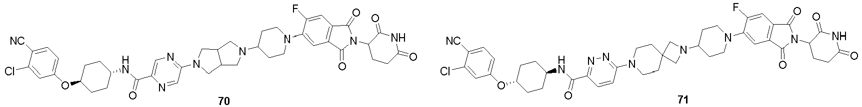

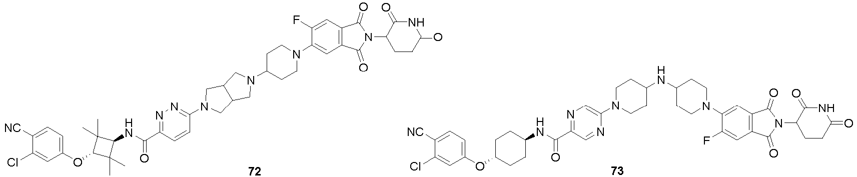

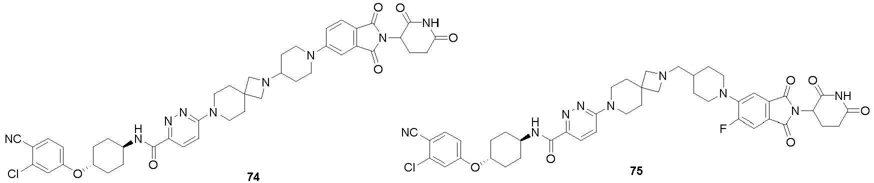

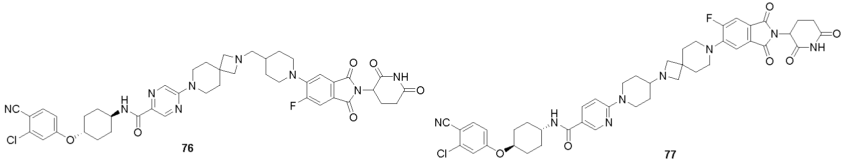

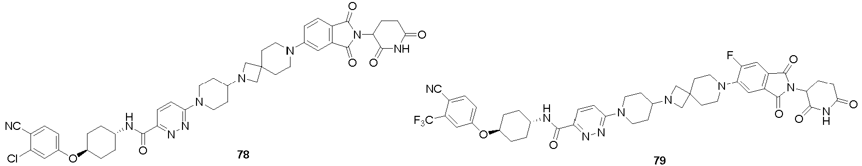

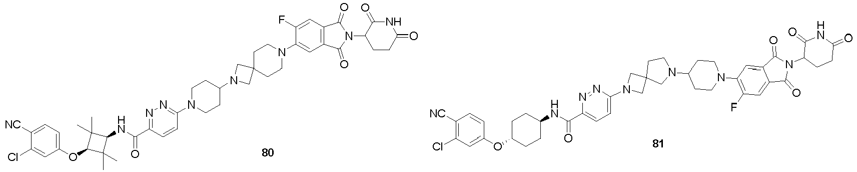

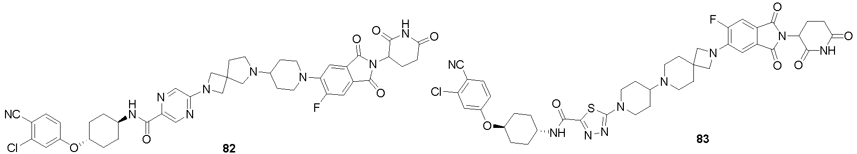

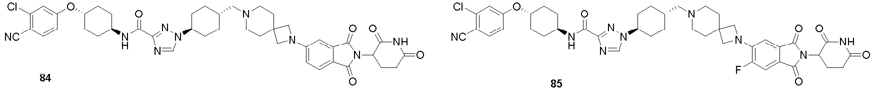

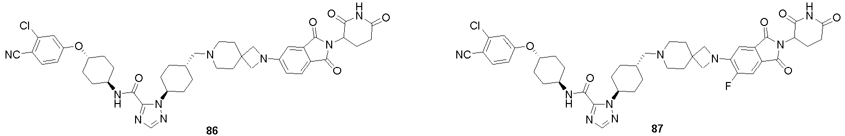

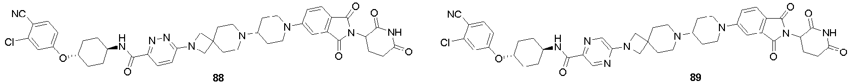

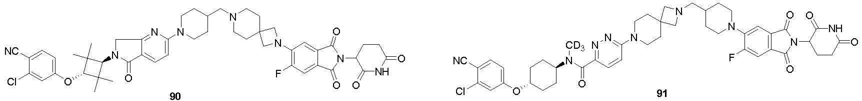

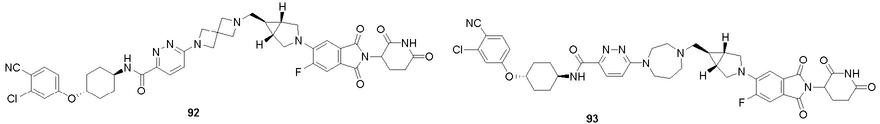

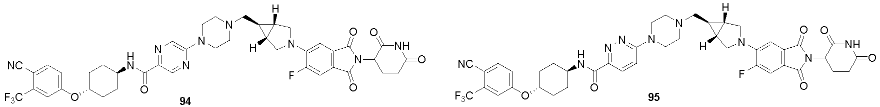

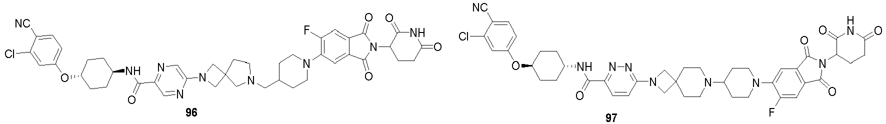

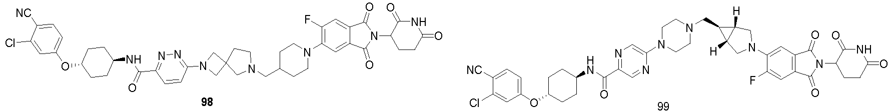

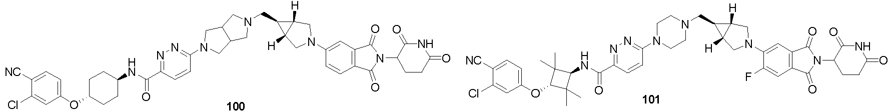

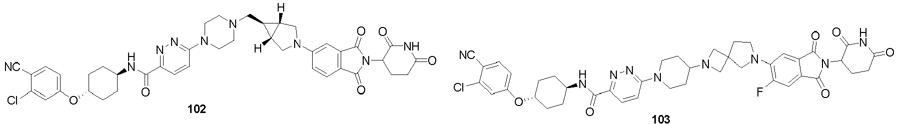

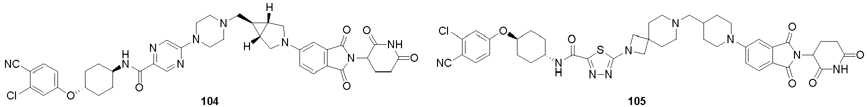

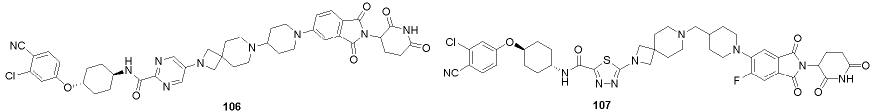

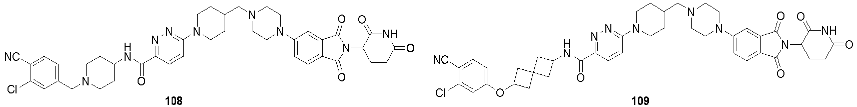

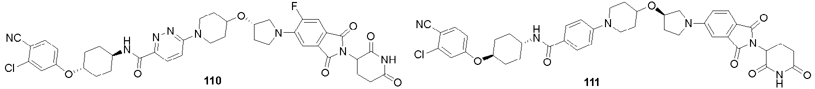

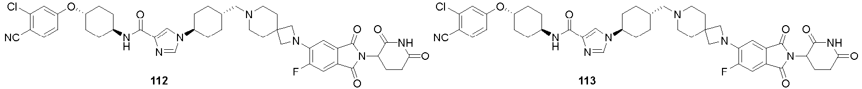

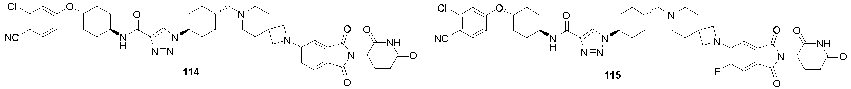

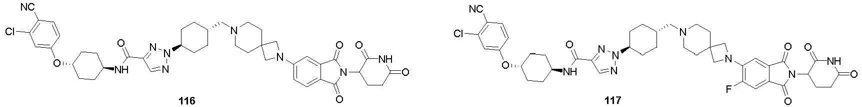

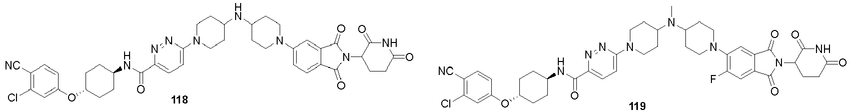

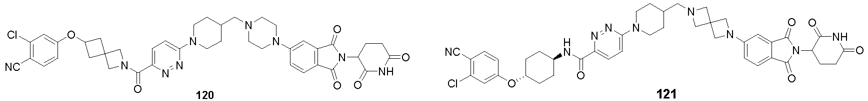

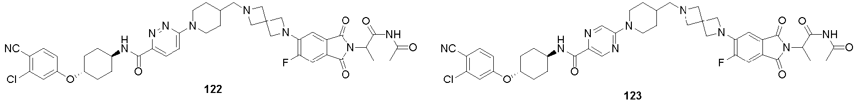

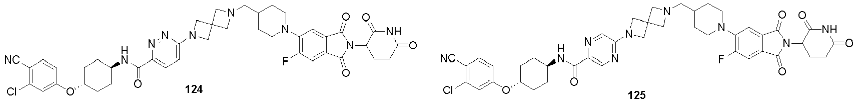

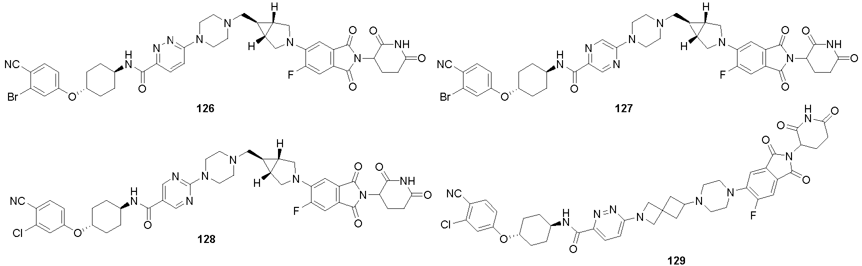

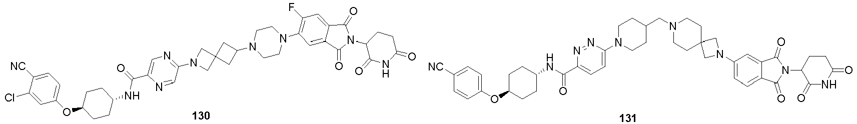

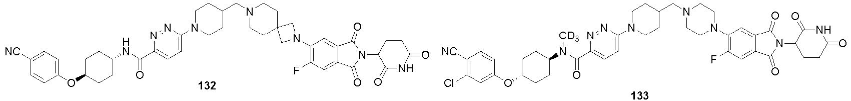

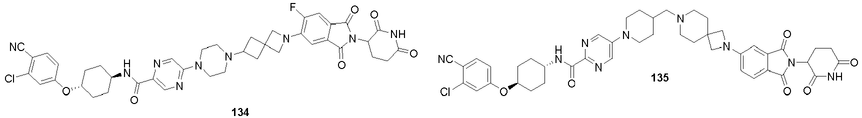

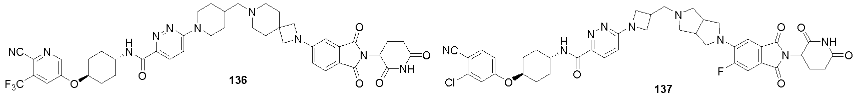

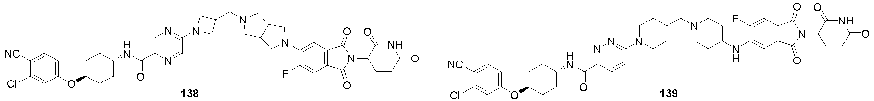

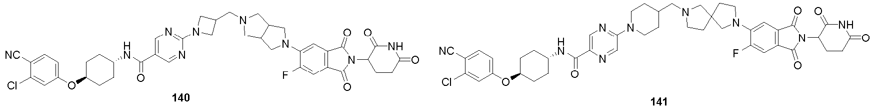

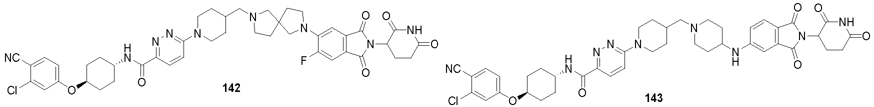

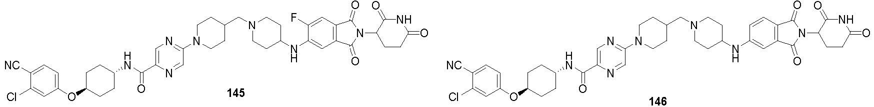

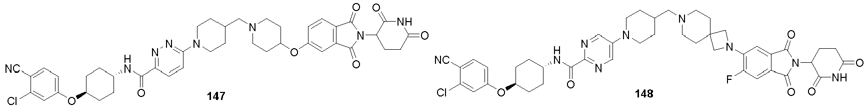

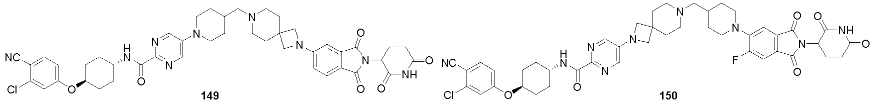

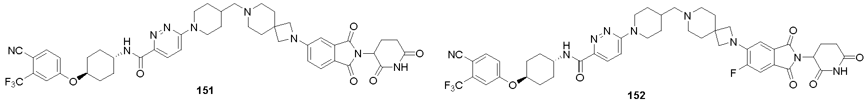

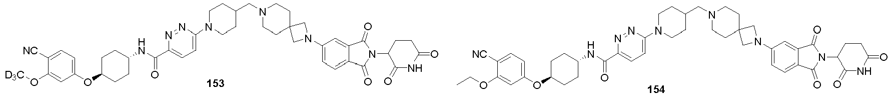

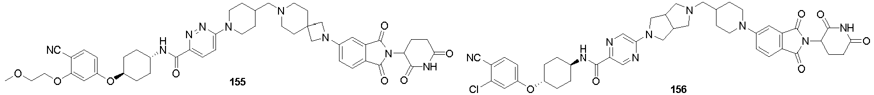

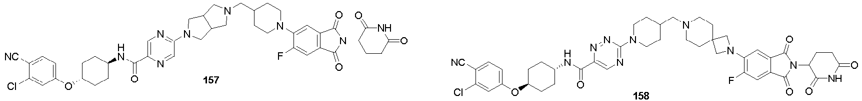

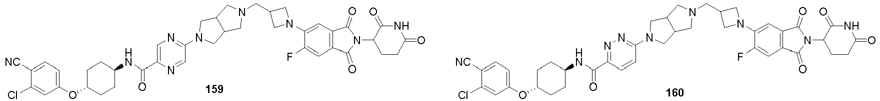

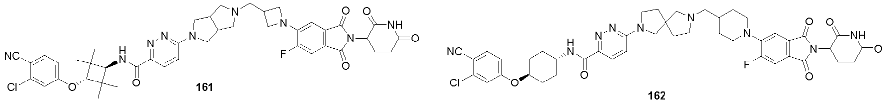

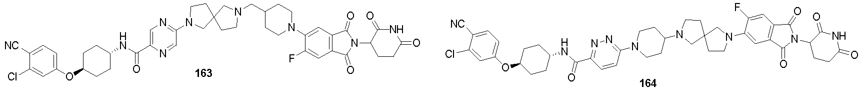

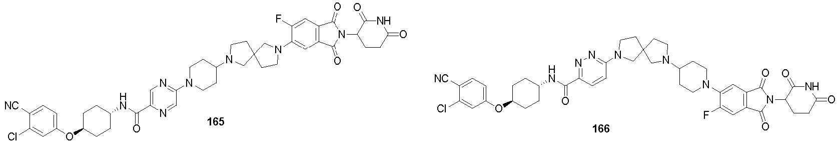

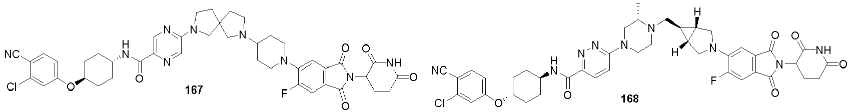

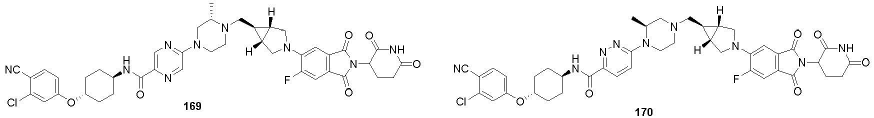

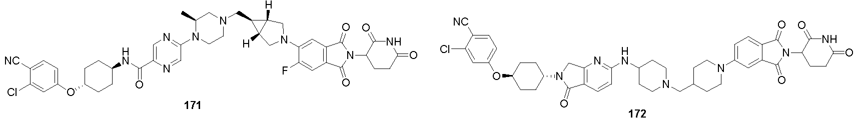

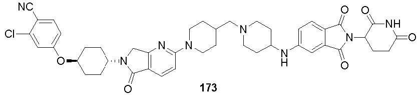

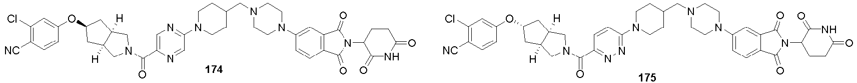

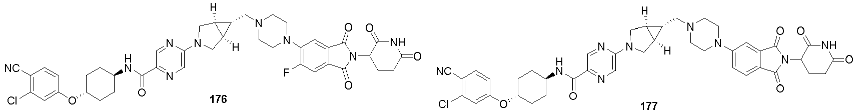

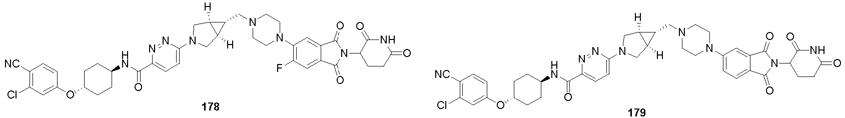

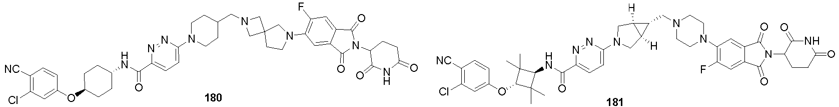

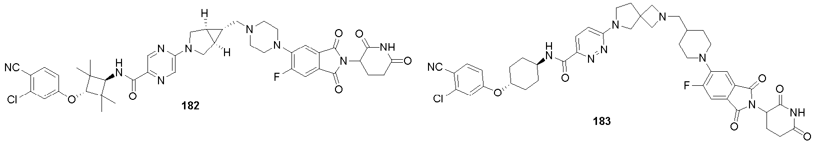

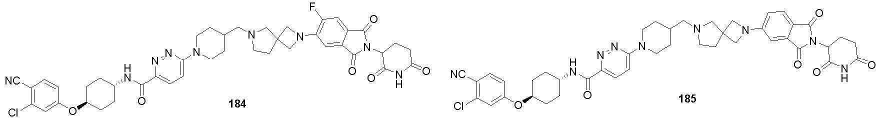

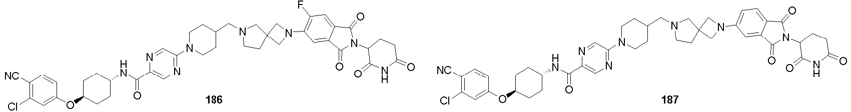

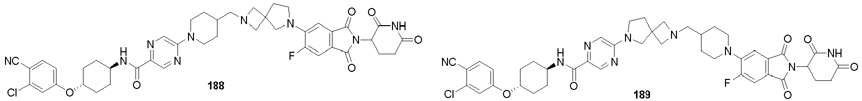

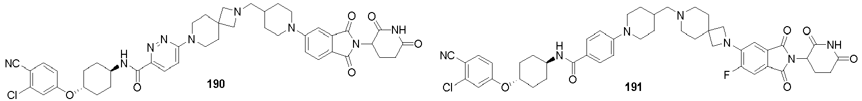

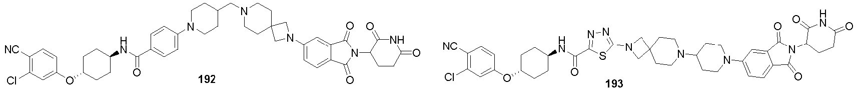

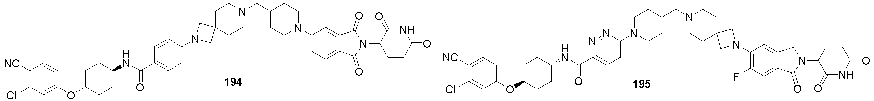

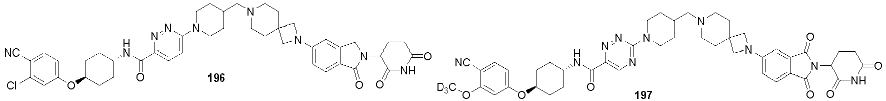

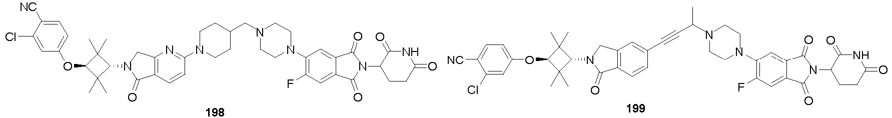

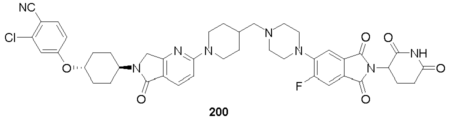

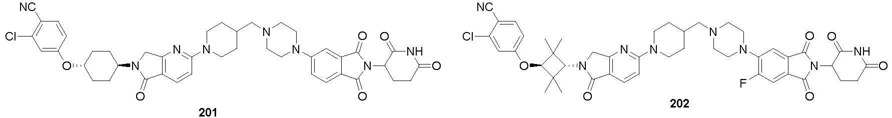

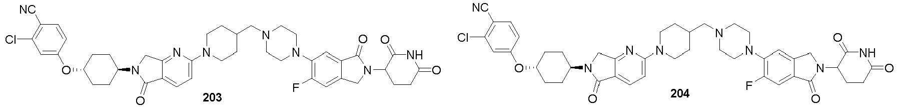

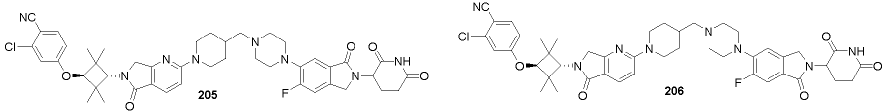

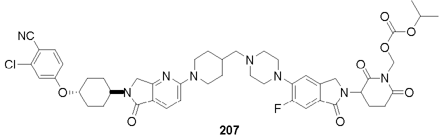

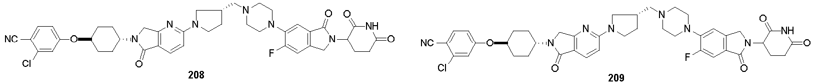

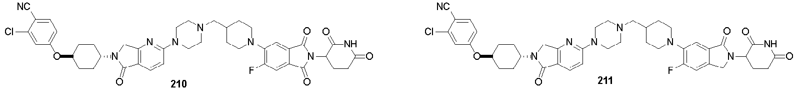

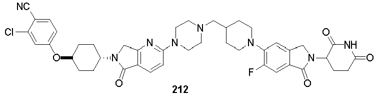

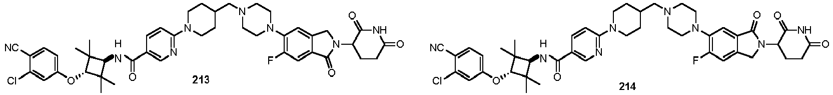

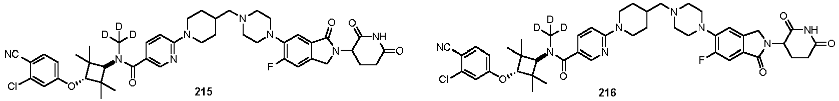

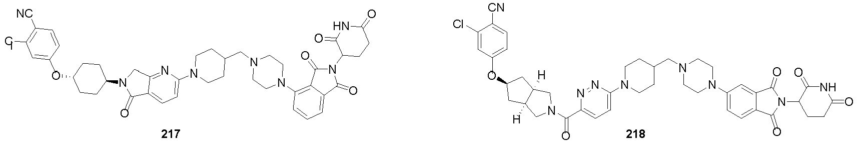

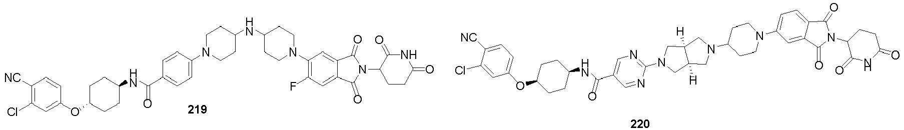

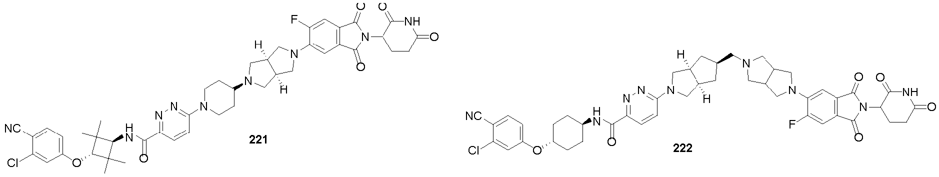

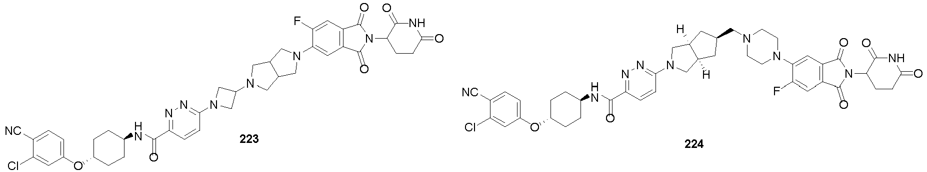

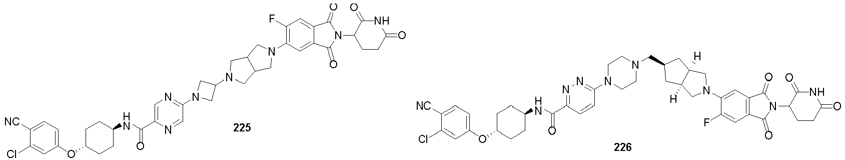

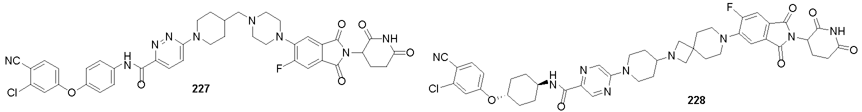

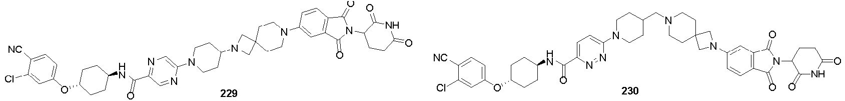

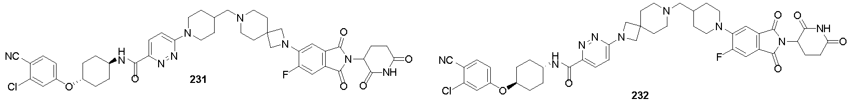

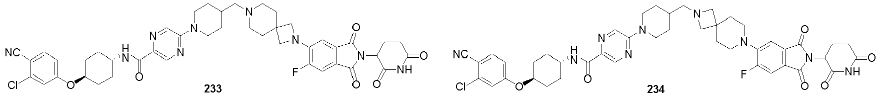

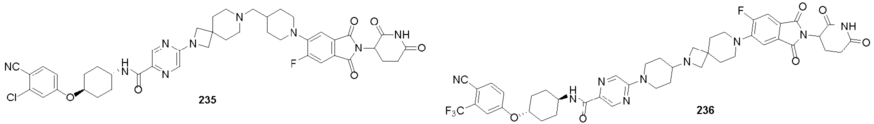

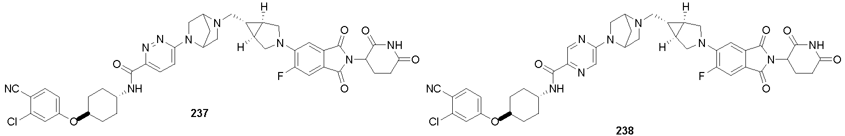

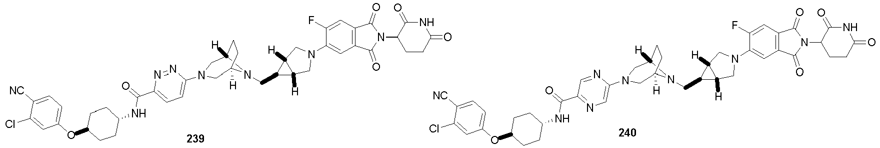

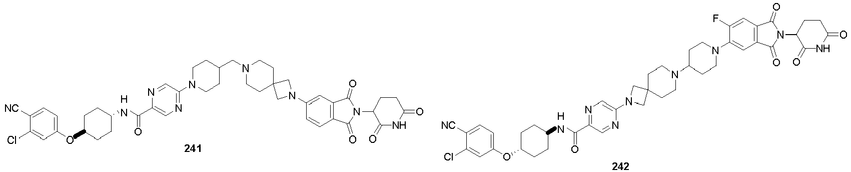

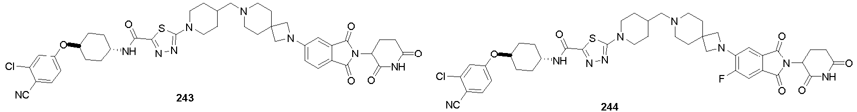

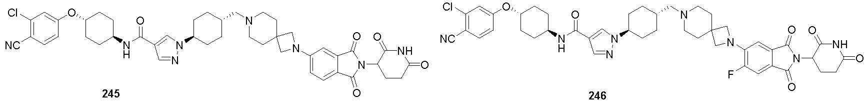

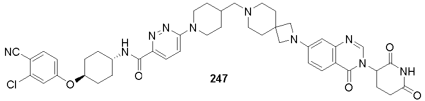

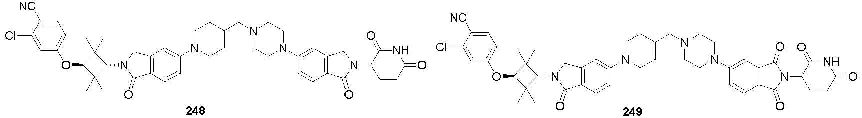

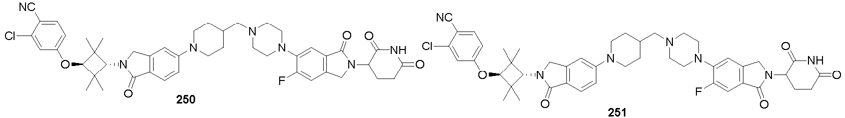

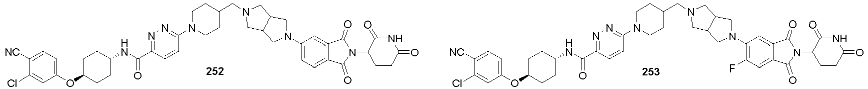

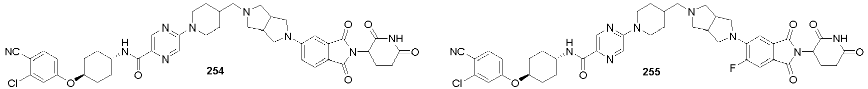

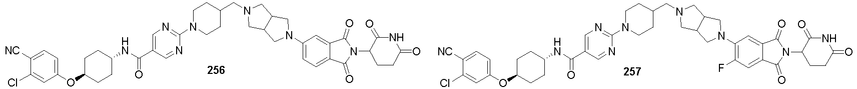

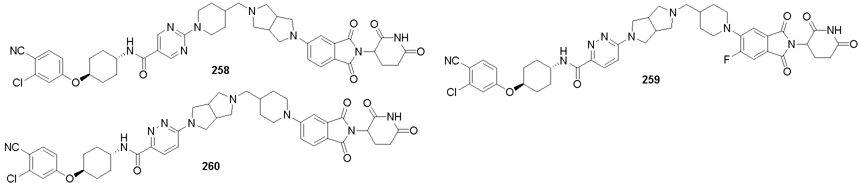

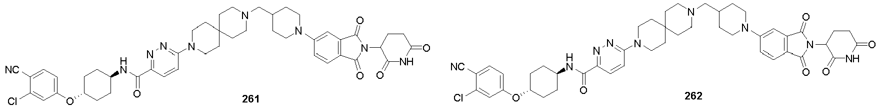

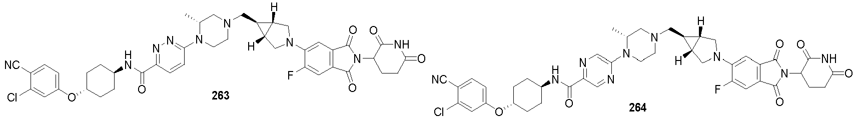

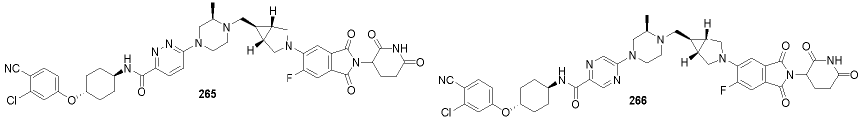

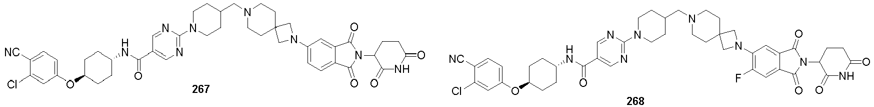

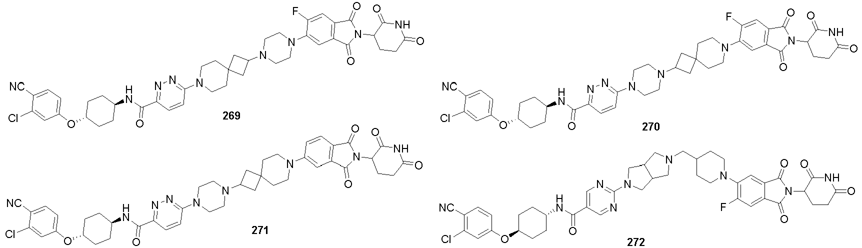

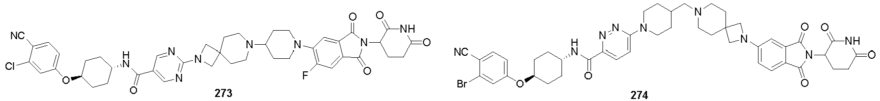

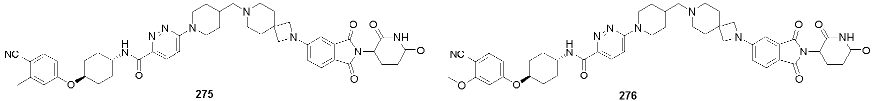

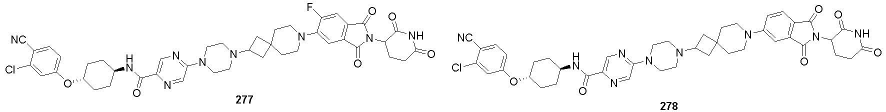

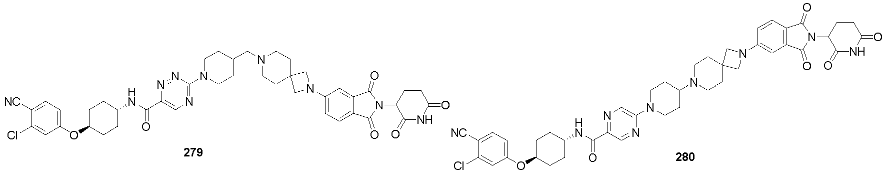

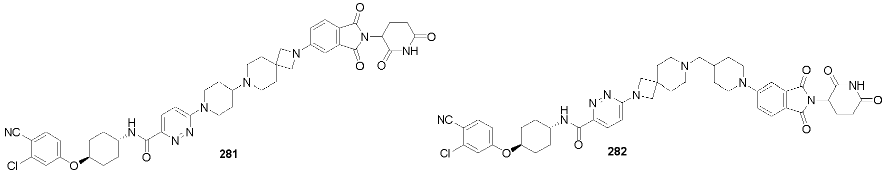

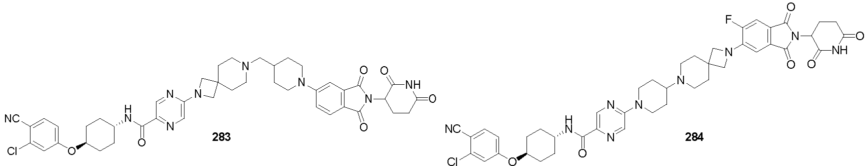

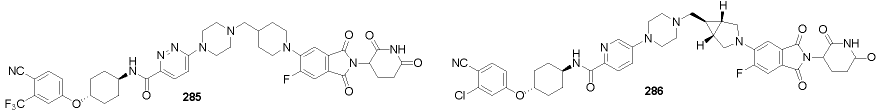

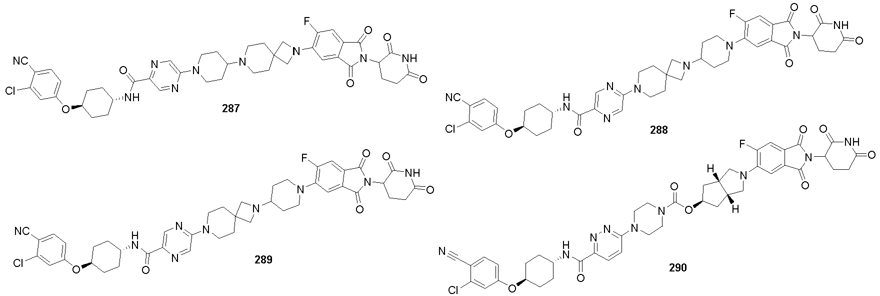

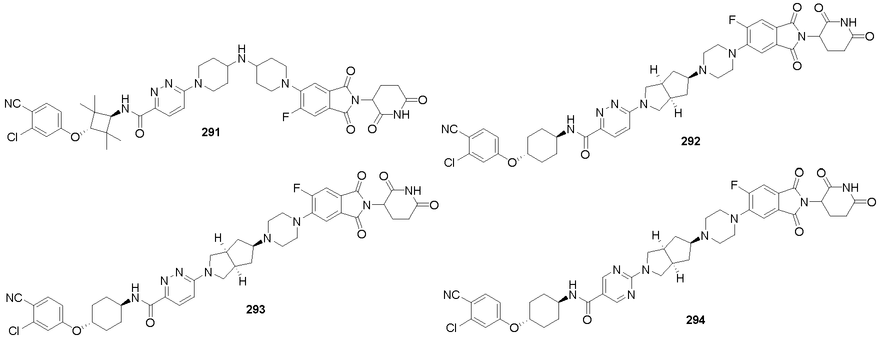

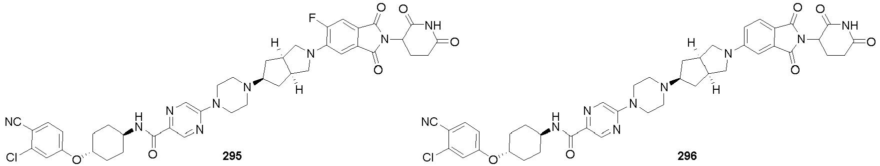

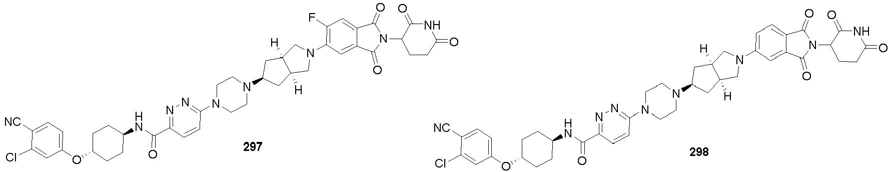

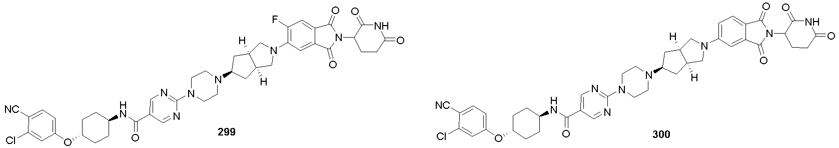

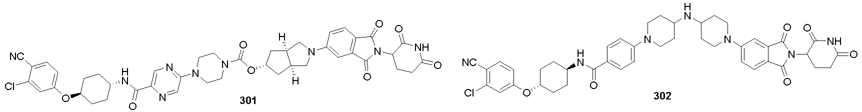

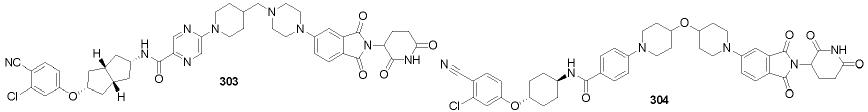

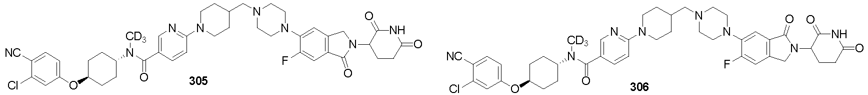

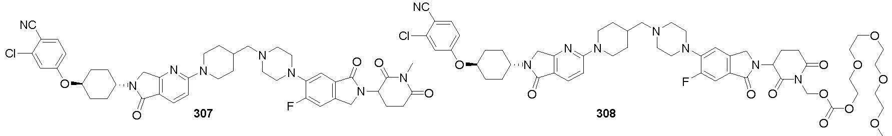

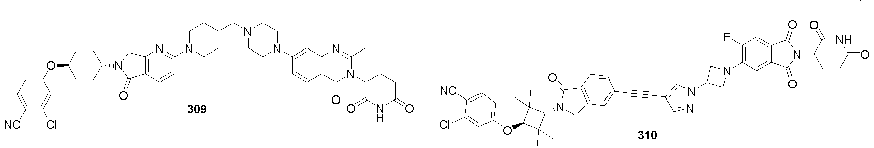

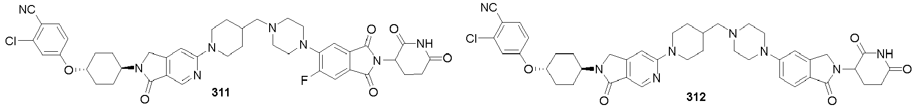

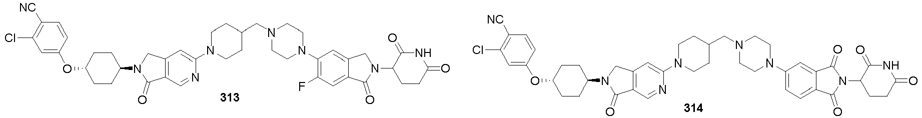

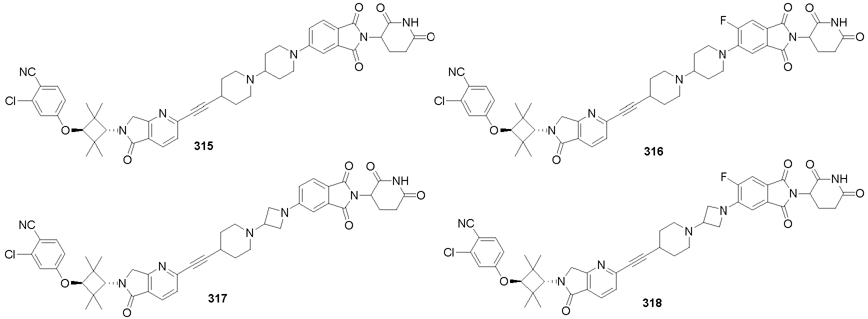

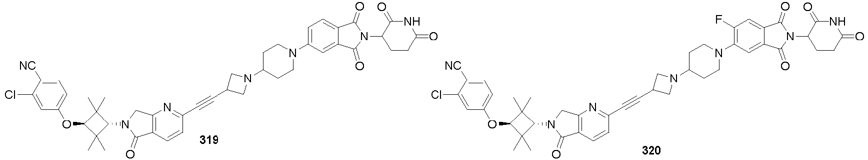

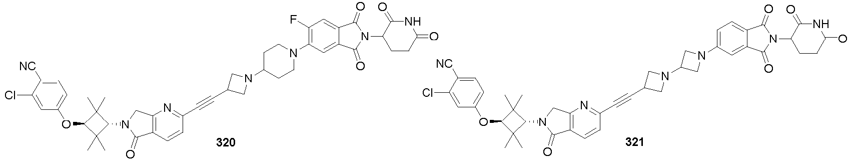

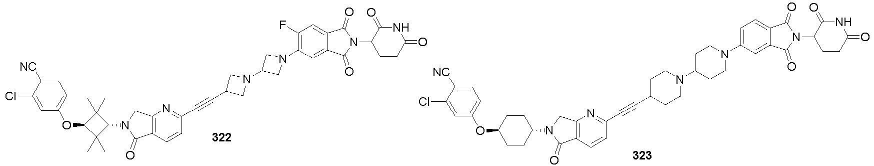

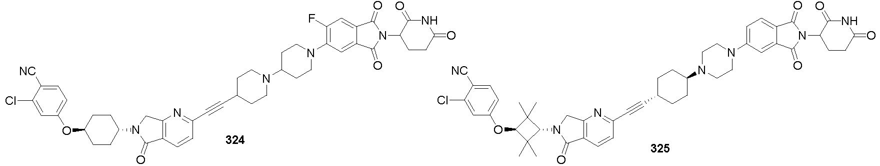

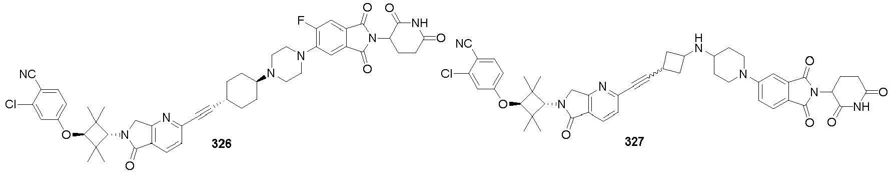

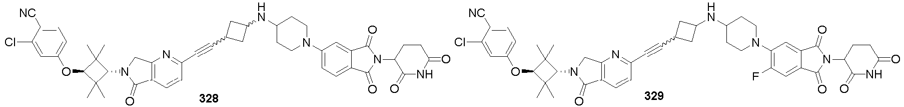

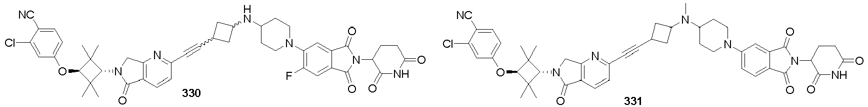

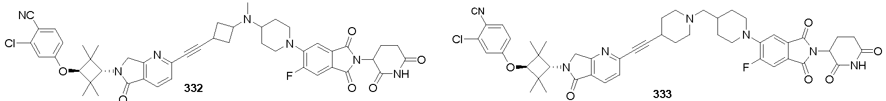

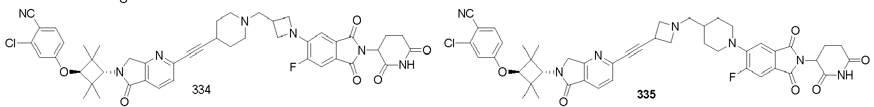

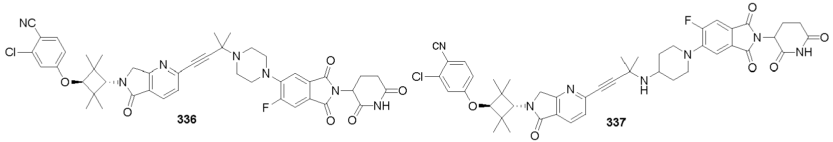

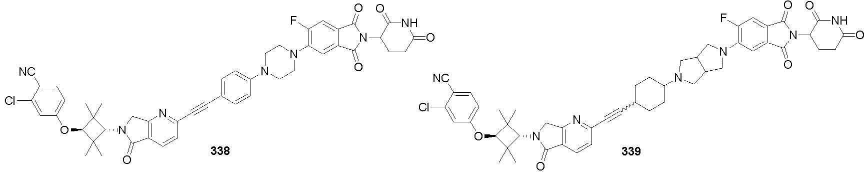

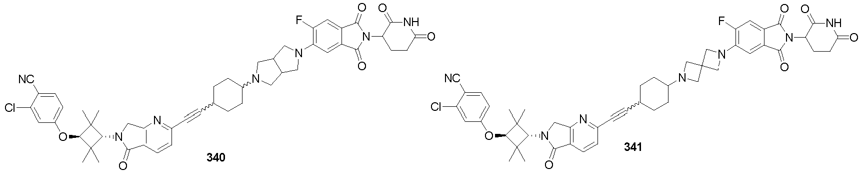

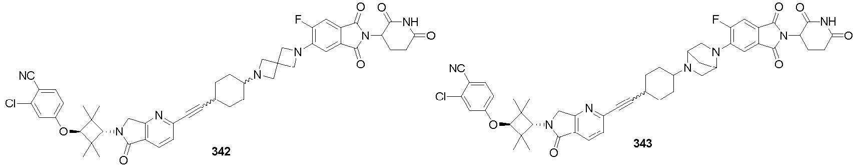

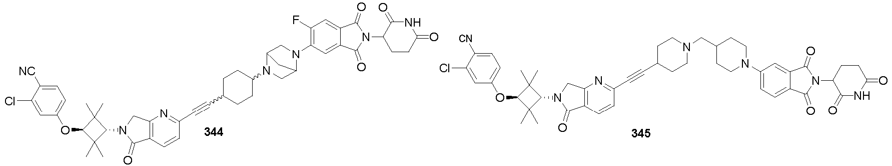

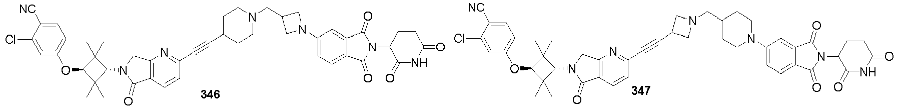

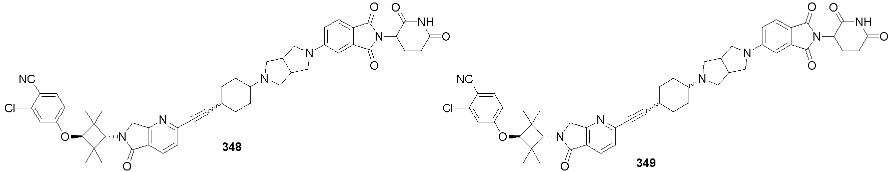

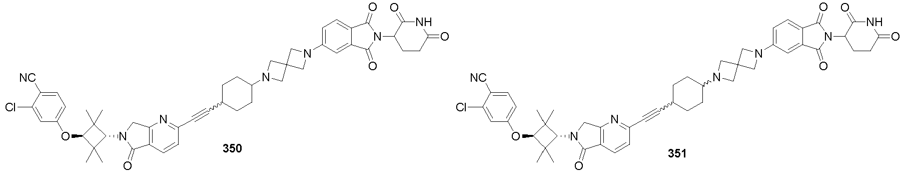

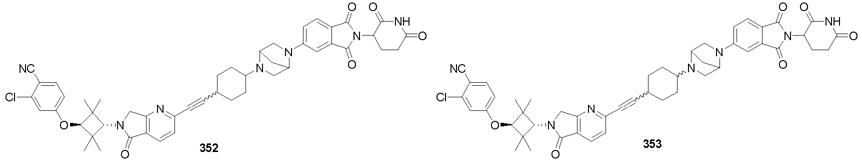

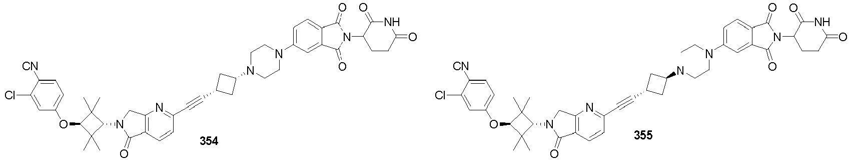

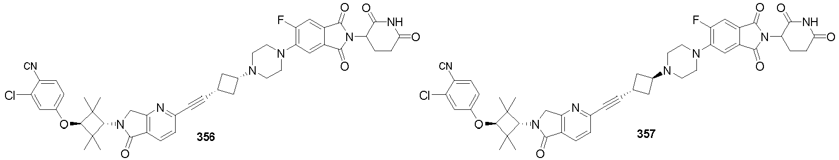

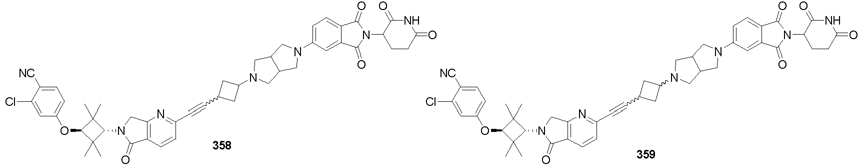

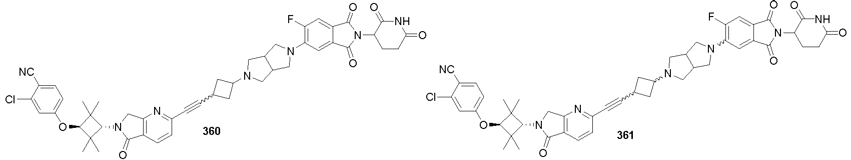

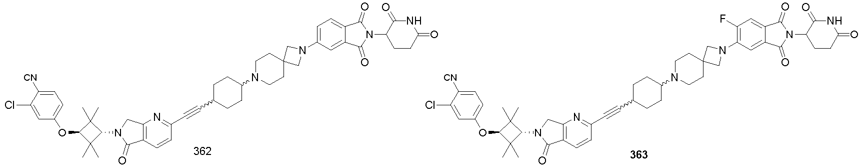

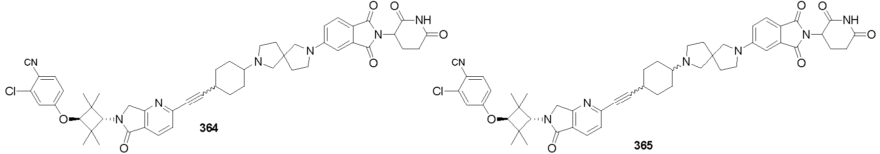

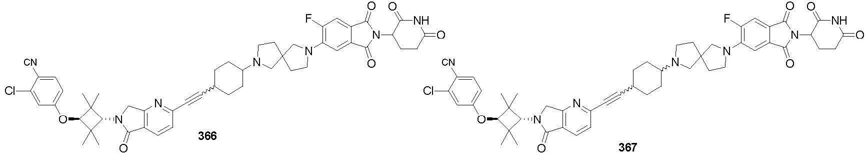

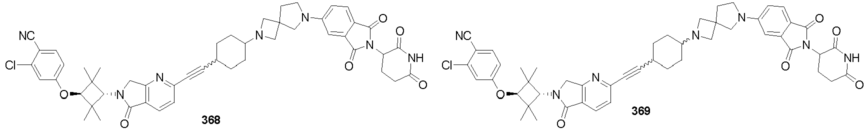

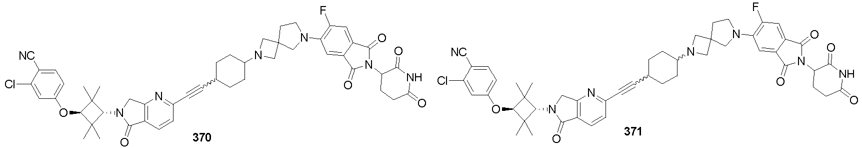

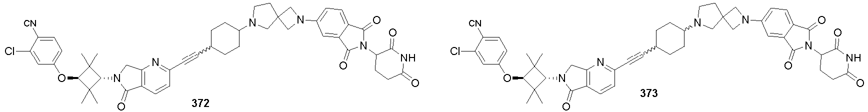

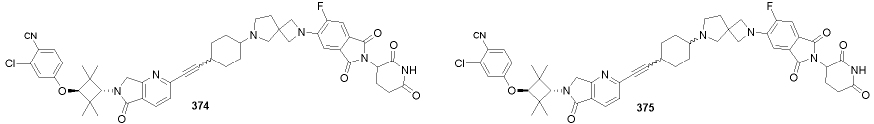

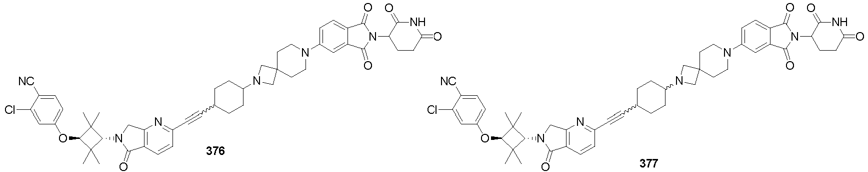

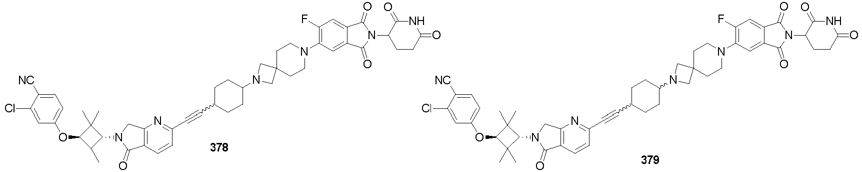

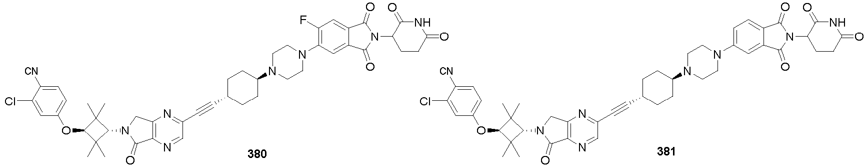

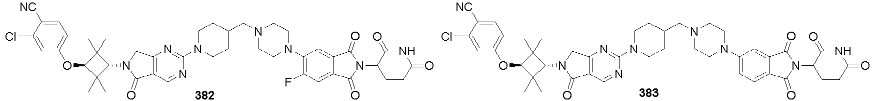

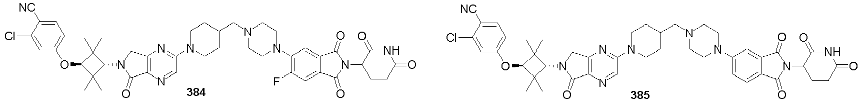

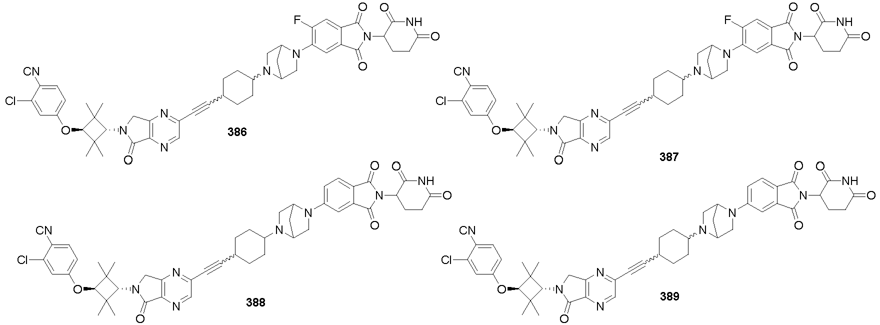

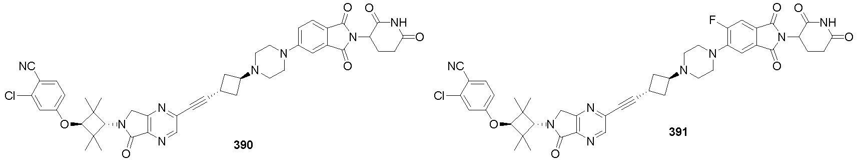

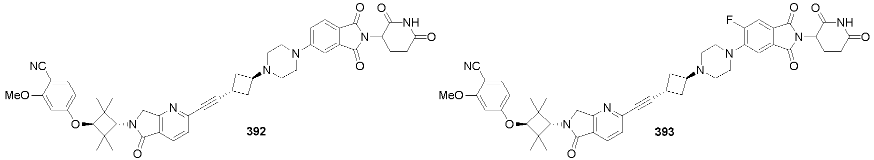

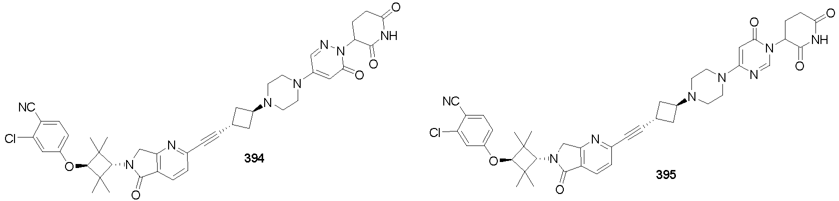

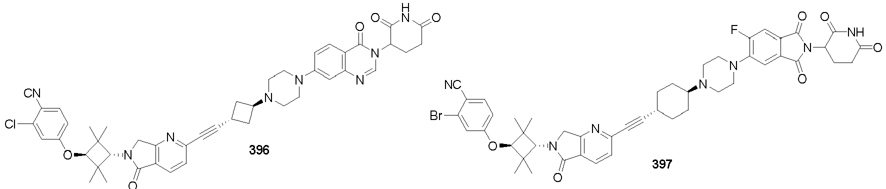

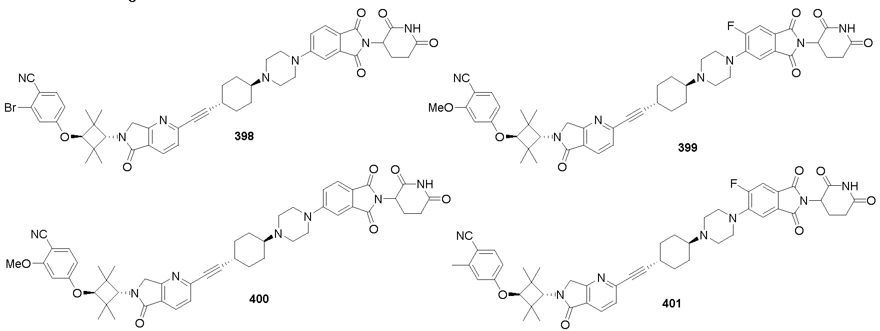

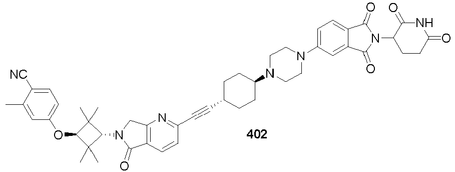

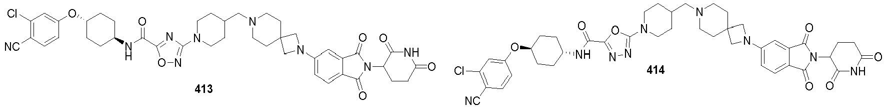

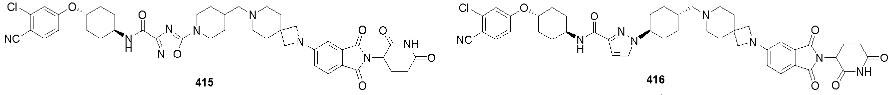

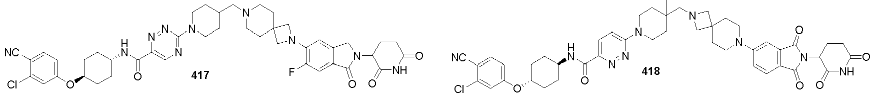

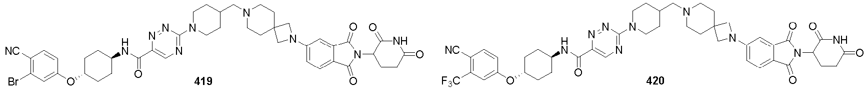

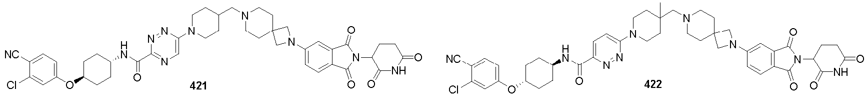

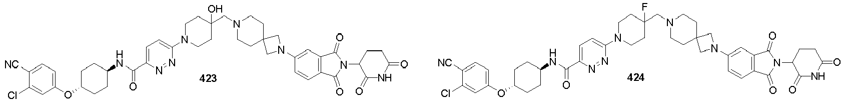

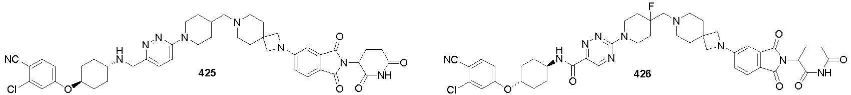

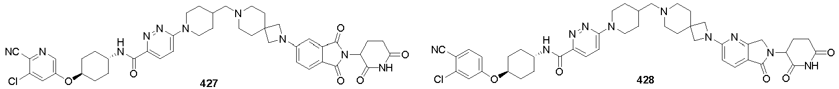

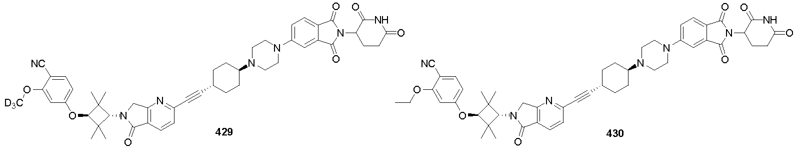

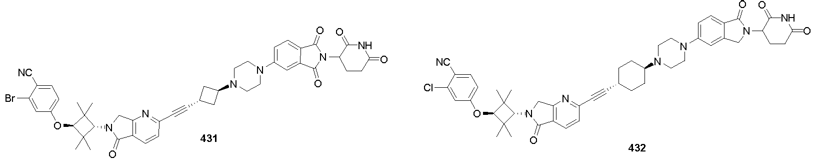

8. The use according to any one of claims 1-7, **characterized in that** the triple negative breast cancer is androgen receptor (AR)-positive triple negative breast cancer (TNBC).
